(19) Europäisches Patentamt
European Patent Office
Office européen des brevets

(11) EP 3 647 397 A1

(12) **EUROPEAN PATENT APPLICATION**

(43) Date of publication:
**06.05.2020 Bulletin 2020/19**

(51) Int Cl.:
***C11D 3/386*** *(2006.01)* ***C12N 9/24*** *(2006.01)*

(21) Application number: **18203731.7**

(22) Date of filing: **31.10.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Henkel AG & Co. KGaA**
**40589 Düsseldorf (DE)**

(72) Inventors:
- **WEIDE, Mirko**
**40223 Düsseldorf (DE)**
- **WIELAND, Susanne**
**41541 Dormagen/ Zons (DE)**
- **DENGUTH, Ulrike**
**45739 Oer-Erkenschwick (DE)**
- **VEJBORG, Rebecca**
**3450 Alleroed (DK)**
- **SEGURA, Dorotea Raventos**
**2880 Bagsvaerd (DK)**
- **SALOMON, Jesper**
**2840 Holte (DK)**
- **JENSEN, Johanne M.**
**2880 Bagsvaerd (DK)**
- **MONRAD, Rune Nygaard**
**3400 Hilleroed (DK)**
- **DUE, Anne Vindum**
**2880 Bagsvaerd (DK)**
- **GUDMAND, Martin**
**2880 Bagsvaerd (DK)**

(74) Representative: **Viering, Jentschura & Partner mbB**
**Patent- und Rechtsanwälte**
**Kennedydamm 55 / Roßstrasse**
**40476 Düsseldorf (DE)**

(54) **CLEANING COMPOSITIONS CONTAINING DISPERSINS IV**

(57) The present invention relates to specific cleaning compositions comprising enzymes. The invention further relates to the use of such compositions in cleaning processes.

Figure 1

**Phylogenetic tree**

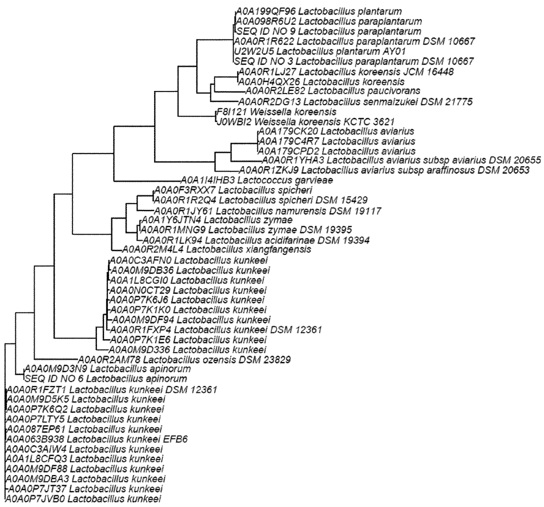


EP 3 647 397 A1

SEQUENCE LISTING

<110> Henkel AG & Co. KGaA

<120> Cleaning compositions containing dispersins IV

<130> 2018P35467EP

<160> 18

<170> PatentIn version 3.5

<210> 1
<211> 1086
<212> DNA
<213> Lactobacillus paraplantarum

<220>
<221> CDS
<222> (1)..(1083)

<220>
<221> sig_peptide
<222> (1)..(90)

<220>
<221> mat_peptide
<222> (91)..(1083)

<400> 1
```
atg aag tac cga cat tac ttt aca caa cta tta ata ttt att agc ccg        48
Met Lys Tyr Arg His Tyr Phe Thr Gln Leu Leu Ile Phe Ile Ser Pro
-30                 -25                 -20                 -15

ctg att ctt ctt tgc ttc agc cag ccc cgt acg gca act gcc aat tca        96
Leu Ile Leu Leu Cys Phe Ser Gln Pro Arg Thr Ala Thr Ala Asn Ser
                -10                 -5                  -1  1

tca aca ttg aat act agt caa ggg gtc atg tta gat tta ggc cgc cat       144
Ser Thr Leu Asn Thr Ser Gln Gly Val Met Leu Asp Leu Gly Arg His
        5                   10                  15

ccg tta gat gaa act gca att aaa gcc gtc att agt gct gct gcc gaa       192
Pro Leu Asp Glu Thr Ala Ile Lys Ala Val Ile Ser Ala Ala Ala Glu
    20                  25                  30

caa cac atg caa tac gtc gaa cta cac tta tca gat aac gaa cat cta       240
Gln His Met Gln Tyr Val Glu Leu His Leu Ser Asp Asn Glu His Leu
35                  40                  45                  50

tgc ttt caa tcg gct tat tta ggt aat gcc gca tcg gca acc gta tta       288
Cys Phe Gln Ser Ala Tyr Leu Gly Asn Ala Ala Ser Ala Thr Val Leu
                55                  60                  65

tcg gcg acg act tta gaa cag cta gtt gct tat gcc aat cag ttg aac       336
Ser Ala Thr Thr Leu Glu Gln Leu Val Ala Tyr Ala Asn Gln Leu Asn
            70                  75                  80

att gaa cta gtt cct gat gtt gac ctt ccc tcg cac gcg gga gcc att       384
Ile Glu Leu Val Pro Asp Val Asp Leu Pro Ser His Ala Gly Ala Ile
        85                  90                  95
```

```
tta cgc caa ttg caa caa act cat ccc gat att tac aat acc gtt aag        432
Leu Arg Gln Leu Gln Gln Thr His Pro Asp Ile Tyr Asn Thr Val Lys
    100                 105                 110

ttg gat gac gaa acc atc gac tat act aaa ccg gca gca atc agt ctc        480
Leu Asp Asp Glu Thr Ile Asp Tyr Thr Lys Pro Ala Ala Ile Ser Leu
115                 120                 125                 130

gct acc aca ctt tat ggc gag ctc gat gct agt ttt aac aat caa agc        528
Ala Thr Thr Leu Tyr Gly Glu Leu Asp Ala Ser Phe Asn Asn Gln Ser
                135                 140                 145

cag cac gat ttg atg ctc ggc gct gat gag gtt cct ggc agc gct agc        576
Gln His Asp Leu Met Leu Gly Ala Asp Glu Val Pro Gly Ser Ala Ser
            150                 155                 160

gcc tat atc gaa ctg acc acc ttt atc aat cag gtc agt cga ttt caa        624
Ala Tyr Ile Glu Leu Thr Thr Phe Ile Asn Gln Val Ser Arg Phe Gln
        165                 170                 175

aat caa cac ggc ttc aac act agt att tgg aat gat tcg cta tta aaa        672
Asn Gln His Gly Phe Asn Thr Ser Ile Trp Asn Asp Ser Leu Leu Lys
    180                 185                 190

aat gaa ctc act cgt ctg gat tca aac att aca atc aat tac tgg tca        720
Asn Glu Leu Thr Arg Leu Asp Ser Asn Ile Thr Ile Asn Tyr Trp Ser
195                 200                 205                 210

caa tct ggt aac aat acc gat gtg gct atc att gcc gac cgc tat gcc        768
Gln Ser Gly Asn Asn Thr Asp Val Ala Ile Ile Ala Asp Arg Tyr Ala
                215                 220                 225

aac cgt gta tcc gtt ccc gac att tta gcc tct ggg cat ccg atc gtg        816
Asn Arg Val Ser Val Pro Asp Ile Leu Ala Ser Gly His Pro Ile Val
            230                 235                 240

aac tgt aat agt tat gcg acc tat tat caa atc aaa aat att ggc aat        864
Asn Cys Asn Ser Tyr Ala Thr Tyr Tyr Gln Ile Lys Asn Ile Gly Asn
        245                 250                 255

gtc aat gat gac gat tac ttt att aat tat ctt aat cac acc ttt cgc        912
Val Asn Asp Asp Asp Tyr Phe Ile Asn Tyr Leu Asn His Thr Phe Arg
    260                 265                 270

ccg aat atc ttt aac gaa att gac acc aat ggg cat aat cag gat tgg        960
Pro Asn Ile Phe Asn Glu Ile Asp Thr Asn Gly His Asn Gln Asp Trp
275                 280                 285                 290

acc att gaa gat ggc gtc aca act aac ggt atc tta gtt agc ttg tgg       1008
Thr Ile Glu Asp Gly Val Thr Thr Asn Gly Ile Leu Val Ser Leu Trp
                295                 300                 305

ggg gcc gat tca gag cat gtt aca cca act gcc atc gtc aat ttc att       1056
Gly Ala Asp Ser Glu His Val Thr Pro Thr Ala Ile Val Asn Phe Ile
            310                 315                 320

aaa cgt atg acg att cca cgg tca ttt taa                               1086
Lys Arg Met Thr Ile Pro Arg Ser Phe
        325                 330


<210>  2
<211>  361
```

<212> PRT
<213> Lactobacillus paraplantarum

<400> 2

```
Met Lys Tyr Arg His Tyr Phe Thr Gln Leu Leu Ile Phe Ile Ser Pro
-30                 -25                 -20                 -15

Leu Ile Leu Leu Cys Phe Ser Gln Pro Arg Thr Ala Thr Ala Asn Ser
                -10                 -5              -1  1

Ser Thr Leu Asn Thr Ser Gln Gly Val Met Leu Asp Leu Gly Arg His
        5                   10                  15

Pro Leu Asp Glu Thr Ala Ile Lys Ala Val Ile Ser Ala Ala Ala Glu
    20                  25                  30

Gln His Met Gln Tyr Val Glu Leu His Leu Ser Asp Asn Glu His Leu
35                  40                  45                  50

Cys Phe Gln Ser Ala Tyr Leu Gly Asn Ala Ala Ser Ala Thr Val Leu
                55                  60                  65

Ser Ala Thr Thr Leu Glu Gln Leu Val Ala Tyr Ala Asn Gln Leu Asn
            70                  75                  80

Ile Glu Leu Val Pro Asp Val Asp Leu Pro Ser His Ala Gly Ala Ile
        85                  90                  95

Leu Arg Gln Leu Gln Gln Thr His Pro Asp Ile Tyr Asn Thr Val Lys
    100                 105                 110

Leu Asp Asp Glu Thr Ile Asp Tyr Thr Lys Pro Ala Ala Ile Ser Leu
115                 120                 125                 130

Ala Thr Thr Leu Tyr Gly Glu Leu Asp Ala Ser Phe Asn Asn Gln Ser
                135                 140                 145

Gln His Asp Leu Met Leu Gly Ala Asp Glu Val Pro Gly Ser Ala Ser
            150                 155                 160

Ala Tyr Ile Glu Leu Thr Thr Phe Ile Asn Gln Val Ser Arg Phe Gln
        165                 170                 175

Asn Gln His Gly Phe Asn Thr Ser Ile Trp Asn Asp Ser Leu Leu Lys
    180                 185                 190

Asn Glu Leu Thr Arg Leu Asp Ser Asn Ile Thr Ile Asn Tyr Trp Ser
195                 200                 205                 210
```

```
Gln Ser Gly Asn Asn Thr Asp Val Ala Ile Ile Ala Asp Arg Tyr Ala
                215                 220                 225

Asn Arg Val Ser Val Pro Asp Ile Leu Ala Ser Gly His Pro Ile Val
            230                 235                 240

Asn Cys Asn Ser Tyr Ala Thr Tyr Tyr Gln Ile Lys Asn Ile Gly Asn
        245                 250                 255

Val Asn Asp Asp Asp Tyr Phe Ile Asn Tyr Leu Asn His Thr Phe Arg
    260                 265                 270

Pro Asn Ile Phe Asn Glu Ile Asp Thr Asn Gly His Asn Gln Asp Trp
275                 280                 285                 290

Thr Ile Glu Asp Gly Val Thr Thr Asn Gly Ile Leu Val Ser Leu Trp
                295                 300                 305

Gly Ala Asp Ser Glu His Val Thr Pro Thr Ala Ile Val Asn Phe Ile
            310                 315                 320

Lys Arg Met Thr Ile Pro Arg Ser Phe
        325                 330


<210>  3
<211>  331
<212>  PRT
<213>  Lactobacillus paraplantarum

<400>  3

Asn Ser Ser Thr Leu Asn Thr Ser Gln Gly Val Met Leu Asp Leu Gly
1               5                   10                  15

Arg His Pro Leu Asp Glu Thr Ala Ile Lys Ala Val Ile Ser Ala Ala
            20                  25                  30

Ala Glu Gln His Met Gln Tyr Val Glu Leu His Leu Ser Asp Asn Glu
        35                  40                  45

His Leu Cys Phe Gln Ser Ala Tyr Leu Gly Asn Ala Ala Ser Ala Thr
    50                  55                  60

Val Leu Ser Ala Thr Thr Leu Glu Gln Leu Val Ala Tyr Ala Asn Gln
65                  70                  75                  80

Leu Asn Ile Glu Leu Val Pro Asp Val Asp Leu Pro Ser His Ala Gly
                85                  90                  95
```

```
Ala Ile Leu Arg Gln Leu Gln Gln Thr His Pro Asp Ile Tyr Asn Thr
            100                 105                 110

Val Lys Leu Asp Asp Glu Thr Ile Asp Tyr Thr Lys Pro Ala Ala Ile
        115                 120                 125

Ser Leu Ala Thr Thr Leu Tyr Gly Glu Leu Asp Ala Ser Phe Asn Asn
    130                 135                 140

Gln Ser Gln His Asp Leu Met Leu Gly Ala Asp Glu Val Pro Gly Ser
145                 150                 155                 160

Ala Ser Ala Tyr Ile Glu Leu Thr Thr Phe Ile Asn Gln Val Ser Arg
                165                 170                 175

Phe Gln Asn Gln His Gly Phe Asn Thr Ser Ile Trp Asn Asp Ser Leu
            180                 185                 190

Leu Lys Asn Glu Leu Thr Arg Leu Asp Ser Asn Ile Thr Ile Asn Tyr
        195                 200                 205

Trp Ser Gln Ser Gly Asn Asn Thr Asp Val Ala Ile Ile Ala Asp Arg
    210                 215                 220

Tyr Ala Asn Arg Val Ser Val Pro Asp Ile Leu Ala Ser Gly His Pro
225                 230                 235                 240

Ile Val Asn Cys Asn Ser Tyr Ala Thr Tyr Tyr Gln Ile Lys Asn Ile
                245                 250                 255

Gly Asn Val Asn Asp Asp Asp Tyr Phe Ile Asn Tyr Leu Asn His Thr
            260                 265                 270

Phe Arg Pro Asn Ile Phe Asn Glu Ile Asp Thr Asn Gly His Asn Gln
        275                 280                 285

Asp Trp Thr Ile Glu Asp Gly Val Thr Thr Asn Gly Ile Leu Val Ser
    290                 295                 300

Leu Trp Gly Ala Asp Ser Glu His Val Thr Pro Thr Ala Ile Val Asn
305                 310                 315                 320

Phe Ile Lys Arg Met Thr Ile Pro Arg Ser Phe
                325                 330
```

<210> 4

```
<211>  1146
<212>  DNA
<213>  Lactobacillus apinorum


<220>
<221>  CDS
<222>  (1)..(1143)

<220>
<221>  sig_peptide
<222>  (1)..(84)

<220>
<221>  mat_peptide
<222>  (85)..(1143)

<400>  4
atg aga aat aaa cga ttt ata atc gtt gga atg ata ttg ttt tta gtc         48
Met Arg Asn Lys Arg Phe Ile Ile Val Gly Met Ile Leu Phe Leu Val
            -25                 -20                 -15

tta atg ttt ata caa tta ggc agt tta gcg aaa aag aca ctt gcc gat         96
Leu Met Phe Ile Gln Leu Gly Ser Leu Ala Lys Lys Thr Leu Ala Asp
        -10                 -5                  -1  1

aca agt aac gat acc aaa aga att ggt cta tca tta gat tgt tcc aga        144
Thr Ser Asn Asp Thr Lys Arg Ile Gly Leu Ser Leu Asp Cys Ser Arg
5                   10                  15                  20

aca tat tat tct cct tct aca atc aaa aag tat ata gat tta tta aag        192
Thr Tyr Tyr Ser Pro Ser Thr Ile Lys Lys Tyr Ile Asp Leu Leu Lys
                25                  30                  35

aaa gat cat ggt aca tat ctt caa tta cac tta aat gac aat gaa aga        240
Lys Asp His Gly Thr Tyr Leu Gln Leu His Leu Asn Asp Asn Glu Arg
            40                  45                  50

tat ggt gtt gaa agt tca acg tta gga caa aca acg caa aac gct aca        288
Tyr Gly Val Glu Ser Ser Thr Leu Gly Gln Thr Thr Gln Asn Ala Thr
        55                  60                  65

ctt aaa gat ggt gtt tat tac aat aat aaa aca cac tta gca ttt tta        336
Leu Lys Asp Gly Val Tyr Tyr Asn Asn Lys Thr His Leu Ala Phe Leu
    70                  75                  80

agt aaa aat caa tta tta gat gta att caa tac ggt tac act cat gga        384
Ser Lys Asn Gln Leu Leu Asp Val Ile Gln Tyr Gly Tyr Thr His Gly
85                  90                  95                  100

att gaa gta att cca gaa ata gac tta cct gga cat gct caa tct ata        432
Ile Glu Val Ile Pro Glu Ile Asp Leu Pro Gly His Ala Gln Ser Ile
                105                 110                 115

ttt aag ctt ctt tca tat act tca gaa gga aag aaa cta gtt aaa gag        480
Phe Lys Leu Leu Ser Tyr Thr Ser Glu Gly Lys Lys Leu Val Lys Glu
            120                 125                 130

tta gaa aat aaa gat ggt tac aat gaa atg tac tac aac aaa caa gct        528
Leu Glu Asn Lys Asp Gly Tyr Asn Glu Met Tyr Tyr Asn Lys Gln Ala
        135                 140                 145

acg att gat ttt tca aaa aag ctt tta agt gaa tat gtt ggc atg ctt        576
```

```
Thr Ile Asp Phe Ser Lys Lys Leu Leu Ser Glu Tyr Val Gly Met Leu
    150                 155                 160

ccc agt gga tac cac att att gta ggt gcc gat gaa ata act att agt        624
Pro Ser Gly Tyr His Ile Ile Val Gly Ala Asp Glu Ile Thr Ile Ser
165                 170                 175                 180

gat aaa agt gat caa gaa gcc gtt gtt aag tat att aat gcc att gat        672
Asp Lys Ser Asp Gln Glu Ala Val Val Lys Tyr Ile Asn Ala Ile Asp
                185                 190                 195

gat tat gtt aat gct aat cat tta aaa ctt gaa atg tgg aat gat agt        720
Asp Tyr Val Asn Ala Asn His Leu Lys Leu Glu Met Trp Asn Asp Ser
            200                 205                 210

ttt cat aag gcg gtt tta agt aaa tat cat aaa gat att tta att aat        768
Phe His Lys Ala Val Leu Ser Lys Tyr His Lys Asp Ile Leu Ile Asn
        215                 220                 225

tac tgg agt tta aca ggt gaa gtt agc tca agt aag gat aga aaa gac        816
Tyr Trp Ser Leu Thr Gly Glu Val Ser Ser Ser Lys Asp Arg Lys Asp
    230                 235                 240

aac atc agg atg aga gca aca ctt cct gaa tta aat aag gct ggt ttt        864
Asn Ile Arg Met Arg Ala Thr Leu Pro Glu Leu Asn Lys Ala Gly Phe
245                 250                 255                 260

aag aca att aac tac aat agt tat tat cta tat atg att aca gat cca        912
Lys Thr Ile Asn Tyr Asn Ser Tyr Tyr Leu Tyr Met Ile Thr Asp Pro
                265                 270                 275

aca tca ttt acc aat gaa tct aag aaa att tgg act tcc gag ttt aaa        960
Thr Ser Phe Thr Asn Glu Ser Lys Lys Ile Trp Thr Ser Glu Phe Lys
            280                 285                 290

aaa tgg aaa atg aat atg tgg aat gat gaa tct aca aaa gat atc aca       1008
Lys Trp Lys Met Asn Met Trp Asn Asp Glu Ser Thr Lys Asp Ile Thr
        295                 300                 305

aag agc gcc aat aat att ggt gct gcc ata tca ata tgg ggt gaa tat       1056
Lys Ser Ala Asn Asn Ile Gly Ala Ala Ile Ser Ile Trp Gly Glu Tyr
    310                 315                 320

cca aat caa tat act ggt gat caa aca tat aat aag aca tat tat tac       1104
Pro Asn Gln Tyr Thr Gly Asp Gln Thr Tyr Asn Lys Thr Tyr Tyr Tyr
325                 330                 335                 340

gtt gat acg ttt tta aag gcc cag gat aaa ttt act aag taa               1146
Val Asp Thr Phe Leu Lys Ala Gln Asp Lys Phe Thr Lys
                345                 350


<210>  5
<211>  381
<212>  PRT
<213>  Lactobacillus apinorum

<400>  5

Met Arg Asn Lys Arg Phe Ile Ile Val Gly Met Ile Leu Phe Leu Val
            -25                 -20                 -15
```

```
Leu Met Phe Ile Gln Leu Gly Ser Leu Ala Lys Lys Thr Leu Ala Asp
        -10                 -5                  -1  1

Thr Ser Asn Asp Thr Lys Arg Ile Gly Leu Ser Leu Asp Cys Ser Arg
5                   10                  15                  20

Thr Tyr Tyr Ser Pro Ser Thr Ile Lys Lys Tyr Ile Asp Leu Leu Lys
                25                  30                  35

Lys Asp His Gly Thr Tyr Leu Gln Leu His Leu Asn Asp Asn Glu Arg
            40                  45                  50

Tyr Gly Val Glu Ser Ser Thr Leu Gly Gln Thr Thr Gln Asn Ala Thr
        55                  60                  65

Leu Lys Asp Gly Val Tyr Tyr Asn Asn Lys Thr His Leu Ala Phe Leu
    70                  75                  80

Ser Lys Asn Gln Leu Leu Asp Val Ile Gln Tyr Gly Tyr Thr His Gly
85                  90                  95                  100

Ile Glu Val Ile Pro Glu Ile Asp Leu Pro Gly His Ala Gln Ser Ile
                105                 110                 115

Phe Lys Leu Leu Ser Tyr Thr Ser Glu Gly Lys Lys Leu Val Lys Glu
            120                 125                 130

Leu Glu Asn Lys Asp Gly Tyr Asn Glu Met Tyr Tyr Asn Lys Gln Ala
        135                 140                 145

Thr Ile Asp Phe Ser Lys Lys Leu Leu Ser Glu Tyr Val Gly Met Leu
    150                 155                 160

Pro Ser Gly Tyr His Ile Ile Val Gly Ala Asp Glu Ile Thr Ile Ser
165                 170                 175                 180

Asp Lys Ser Asp Gln Glu Ala Val Val Lys Tyr Ile Asn Ala Ile Asp
                185                 190                 195

Asp Tyr Val Asn Ala Asn His Leu Lys Leu Glu Met Trp Asn Asp Ser
            200                 205                 210

Phe His Lys Ala Val Leu Ser Lys Tyr His Lys Asp Ile Leu Ile Asn
        215                 220                 225

Tyr Trp Ser Leu Thr Gly Glu Val Ser Ser Ser Lys Asp Arg Lys Asp
    230                 235                 240
```

Asn Ile Arg Met Arg Ala Thr Leu Pro Glu Leu Asn Lys Ala Gly Phe
245 250 255 260

Lys Thr Ile Asn Tyr Asn Ser Tyr Tyr Leu Tyr Met Ile Thr Asp Pro
265 270 275

Thr Ser Phe Thr Asn Glu Ser Lys Lys Ile Trp Thr Ser Glu Phe Lys
280 285 290

Lys Trp Lys Met Asn Met Trp Asn Asp Glu Ser Thr Lys Asp Ile Thr
295 300 305

Lys Ser Ala Asn Asn Ile Gly Ala Ala Ile Ser Ile Trp Gly Glu Tyr
310 315 320

Pro Asn Gln Tyr Thr Gly Asp Gln Thr Tyr Asn Lys Thr Tyr Tyr Tyr
325 330 335 340

Val Asp Thr Phe Leu Lys Ala Gln Asp Lys Phe Thr Lys
345 350

<210> 6
<211> 353
<212> PRT
<213> Lactobacillus apinorum

<400> 6

Thr Leu Ala Asp Thr Ser Asn Asp Thr Lys Arg Ile Gly Leu Ser Leu
1 5 10 15

Asp Cys Ser Arg Thr Tyr Tyr Ser Pro Ser Thr Ile Lys Lys Tyr Ile
20 25 30

Asp Leu Leu Lys Lys Asp His Gly Thr Tyr Leu Gln Leu His Leu Asn
35 40 45

Asp Asn Glu Arg Tyr Gly Val Glu Ser Ser Thr Leu Gly Gln Thr Thr
50 55 60

Gln Asn Ala Thr Leu Lys Asp Gly Val Tyr Tyr Asn Asn Lys Thr His
65 70 75 80

Leu Ala Phe Leu Ser Lys Asn Gln Leu Leu Asp Val Ile Gln Tyr Gly
85 90 95

Tyr Thr His Gly Ile Glu Val Ile Pro Glu Ile Asp Leu Pro Gly His
100 105 110

```
Ala Gln Ser Ile Phe Lys Leu Leu Ser Tyr Thr Ser Glu Gly Lys Lys
        115                 120                 125

Leu Val Lys Glu Leu Glu Asn Lys Asp Gly Tyr Asn Glu Met Tyr Tyr
    130                 135                 140

Asn Lys Gln Ala Thr Ile Asp Phe Ser Lys Lys Leu Leu Ser Glu Tyr
145                 150                 155                 160

Val Gly Met Leu Pro Ser Gly Tyr His Ile Ile Val Gly Ala Asp Glu
                165                 170                 175

Ile Thr Ile Ser Asp Lys Ser Asp Gln Glu Ala Val Val Lys Tyr Ile
            180                 185                 190

Asn Ala Ile Asp Asp Tyr Val Asn Ala Asn His Leu Lys Leu Glu Met
        195                 200                 205

Trp Asn Asp Ser Phe His Lys Ala Val Leu Ser Lys Tyr His Lys Asp
    210                 215                 220

Ile Leu Ile Asn Tyr Trp Ser Leu Thr Gly Glu Val Ser Ser Ser Lys
225                 230                 235                 240

Asp Arg Lys Asp Asn Ile Arg Met Arg Ala Thr Leu Pro Glu Leu Asn
                245                 250                 255

Lys Ala Gly Phe Lys Thr Ile Asn Tyr Asn Ser Tyr Tyr Leu Tyr Met
            260                 265                 270

Ile Thr Asp Pro Thr Ser Phe Thr Asn Glu Ser Lys Lys Ile Trp Thr
        275                 280                 285

Ser Glu Phe Lys Lys Trp Lys Met Asn Met Trp Asn Asp Glu Ser Thr
    290                 295                 300

Lys Asp Ile Thr Lys Ser Ala Asn Asn Ile Gly Ala Ala Ile Ser Ile
305                 310                 315                 320

Trp Gly Glu Tyr Pro Asn Gln Tyr Thr Gly Asp Gln Thr Tyr Asn Lys
                325                 330                 335

Thr Tyr Tyr Tyr Val Asp Thr Phe Leu Lys Ala Gln Asp Lys Phe Thr
            340                 345                 350

Lys
```

```
<210>  7
<211>  1086
<212>  DNA
<213>  Lactobacillus paraplantarum


<220>
<221>  CDS
<222>  (1)..(1083)

<220>
<221>  sig_peptide
<222>  (1)..(90)

<220>
<221>  mat_peptide
<222>  (91)..(1083)

<400>  7
atg aag tgc cga cat tac ttt aca caa cta tta ata ttt att agc ccg        48
Met Lys Cys Arg His Tyr Phe Thr Gln Leu Leu Ile Phe Ile Ser Pro
-30                 -25                 -20                 -15

ctg att ctt ctt tgc ttc agc cag ccc cgt acg gca act gcc aat tca        96
Leu Ile Leu Leu Cys Phe Ser Gln Pro Arg Thr Ala Thr Ala Asn Ser
                -10                 -5                  -1  1

tca aca ttg aat act agt caa ggg gtc atg tta gat tta ggt cgc cat       144
Ser Thr Leu Asn Thr Ser Gln Gly Val Met Leu Asp Leu Gly Arg His
        5                   10                  15

ccg tta gat gaa act gca att aaa gcc gtc att agt gct gct gcc gaa       192
Pro Leu Asp Glu Thr Ala Ile Lys Ala Val Ile Ser Ala Ala Ala Glu
    20                  25                  30

caa cac atg caa tac gtc gaa cta cac tta tca gat aac gaa cat cta       240
Gln His Met Gln Tyr Val Glu Leu His Leu Ser Asp Asn Glu His Leu
35                  40                  45                  50

tgc ttt caa tcg gct tat tta ggt aat gcc gca tcg gca acc gta tta       288
Cys Phe Gln Ser Ala Tyr Leu Gly Asn Ala Ala Ser Ala Thr Val Leu
                55                  60                  65

tcg gca acg act tta gaa cag cta gtt gct tat gcc aat cag ttg aac       336
Ser Ala Thr Thr Leu Glu Gln Leu Val Ala Tyr Ala Asn Gln Leu Asn
            70                  75                  80

att gaa cta gtt cct gat gtt gac ctt ccc tcg cac gcg gga gcc att       384
Ile Glu Leu Val Pro Asp Val Asp Leu Pro Ser His Ala Gly Ala Ile
        85                  90                  95

tta cgc caa ttg caa caa act cat ccc gat att tac aat acc gtt aag       432
Leu Arg Gln Leu Gln Gln Thr His Pro Asp Ile Tyr Asn Thr Val Lys
    100                 105                 110

ttg gat gac gaa acc atc gac tat act aaa ccg gca gca gtc agt ctc       480
Leu Asp Asp Glu Thr Ile Asp Tyr Thr Lys Pro Ala Ala Val Ser Leu
115                 120                 125                 130

gct acc aca ctt tat ggc gag ctc gat gct agt ttt aac aat caa agc       528
Ala Thr Thr Leu Tyr Gly Glu Leu Asp Ala Ser Phe Asn Asn Gln Ser
```

135 140 145

cag cac gat ttg atg ctc ggc gct gat gag gtt tct ggc agc gct agc 576
Gln His Asp Leu Met Leu Gly Ala Asp Glu Val Ser Gly Ser Ala Ser
150 155 160

gcc tat atc gaa ctg acc acc ttt atc aat cag gtc agt cga ttt caa 624
Ala Tyr Ile Glu Leu Thr Thr Phe Ile Asn Gln Val Ser Arg Phe Gln
165 170 175

aat caa aac ggc ttc aac act agt att tgg aat gat tcg cta tta aaa 672
Asn Gln Asn Gly Phe Asn Thr Ser Ile Trp Asn Asp Ser Leu Leu Lys
180 185 190

aat gaa ctc aat cgt ctg gat tca aac att aca atc aat tac tgg tca 720
Asn Glu Leu Asn Arg Leu Asp Ser Asn Ile Thr Ile Asn Tyr Trp Ser
195 200 205 210

caa tct ggt aac aat acc gat gcg gct atc att gcc gac cgc tat gcc 768
Gln Ser Gly Asn Asn Thr Asp Ala Ala Ile Ile Ala Asp Arg Tyr Ala
215 220 225

aac cgt gca tcc gtt ccc gac att tta gcc tct ggg cat ccg atc gtg 816
Asn Arg Ala Ser Val Pro Asp Ile Leu Ala Ser Gly His Pro Ile Val
230 235 240

aac tgt aat agt tat gcg acc tat tat caa ttc aaa aat att ggc aat 864
Asn Cys Asn Ser Tyr Ala Thr Tyr Tyr Gln Phe Lys Asn Ile Gly Asn
245 250 255

gtc aat gat gac aat tac ttt att aat tat ctt aat cac acc ttt cgc 912
Val Asn Asp Asp Asn Tyr Phe Ile Asn Tyr Leu Asn His Thr Phe Arg
260 265 270

ccg aat atc ttt aac gaa att gac acc aac ggg cat aat cag gat tgg 960
Pro Asn Ile Phe Asn Glu Ile Asp Thr Asn Gly His Asn Gln Asp Trp
275 280 285 290

acc att gaa gat ggc gtc aca act aac ggt atc tta gtt agc ttg tgg 1008
Thr Ile Glu Asp Gly Val Thr Thr Asn Gly Ile Leu Val Ser Leu Trp
295 300 305

ggg gcc gat tca gag cat gtt aca cca act gct att gtc aat ttc att 1056
Gly Ala Asp Ser Glu His Val Thr Pro Thr Ala Ile Val Asn Phe Ile
310 315 320

aaa cgt atg gcg att ccc cgg tca ttt taa 1086
Lys Arg Met Ala Ile Pro Arg Ser Phe
325 330

<210> 8
<211> 361
<212> PRT
<213> Lactobacillus paraplantarum

<400> 8

Met Lys Cys Arg His Tyr Phe Thr Gln Leu Leu Ile Phe Ile Ser Pro
-30 -25 -20 -15

Leu Ile Leu Leu Cys Phe Ser Gln Pro Arg Thr Ala Thr Ala Asn Ser

```
                -10                 -5              -1   1

Ser Thr Leu Asn Thr Ser Gln Gly Val Met Leu Asp Leu Gly Arg His
        5                   10                  15

Pro Leu Asp Glu Thr Ala Ile Lys Ala Val Ile Ser Ala Ala Ala Glu
    20                  25                  30

Gln His Met Gln Tyr Val Glu Leu His Leu Ser Asp Asn Glu His Leu
35                  40                  45                  50

Cys Phe Gln Ser Ala Tyr Leu Gly Asn Ala Ala Ser Ala Thr Val Leu
                55                  60                  65

Ser Ala Thr Thr Leu Glu Gln Leu Val Ala Tyr Ala Asn Gln Leu Asn
            70                  75                  80

Ile Glu Leu Val Pro Asp Val Asp Leu Pro Ser His Ala Gly Ala Ile
        85                  90                  95

Leu Arg Gln Leu Gln Gln Thr His Pro Asp Ile Tyr Asn Thr Val Lys
    100                 105                 110

Leu Asp Asp Glu Thr Ile Asp Tyr Thr Lys Pro Ala Ala Val Ser Leu
115                 120                 125                 130

Ala Thr Thr Leu Tyr Gly Glu Leu Asp Ala Ser Phe Asn Asn Gln Ser
                135                 140                 145

Gln His Asp Leu Met Leu Gly Ala Asp Glu Val Ser Gly Ser Ala Ser
            150                 155                 160

Ala Tyr Ile Glu Leu Thr Thr Phe Ile Asn Gln Val Ser Arg Phe Gln
        165                 170                 175

Asn Gln Asn Gly Phe Asn Thr Ser Ile Trp Asn Asp Ser Leu Leu Lys
    180                 185                 190

Asn Glu Leu Asn Arg Leu Asp Ser Asn Ile Thr Ile Asn Tyr Trp Ser
195                 200                 205                 210

Gln Ser Gly Asn Asn Thr Asp Ala Ala Ile Ile Ala Asp Arg Tyr Ala
                215                 220                 225

Asn Arg Ala Ser Val Pro Asp Ile Leu Ala Ser Gly His Pro Ile Val
            230                 235                 240
```

Asn Cys Asn Ser Tyr Ala Thr Tyr Tyr Gln Phe Lys Asn Ile Gly Asn
245 250 255

Val Asn Asp Asp Asn Tyr Phe Ile Asn Tyr Leu Asn His Thr Phe Arg
260 265 270

Pro Asn Ile Phe Asn Glu Ile Asp Thr Asn Gly His Asn Gln Asp Trp
275 280 285 290

Thr Ile Glu Asp Gly Val Thr Thr Asn Gly Ile Leu Val Ser Leu Trp
295 300 305

Gly Ala Asp Ser Glu His Val Thr Pro Thr Ala Ile Val Asn Phe Ile
310 315 320

Lys Arg Met Ala Ile Pro Arg Ser Phe
325 330

<210> 9
<211> 331
<212> PRT
<213> Lactobacillus paraplantarum

<400> 9

Asn Ser Ser Thr Leu Asn Thr Ser Gln Gly Val Met Leu Asp Leu Gly
1 5 10 15

Arg His Pro Leu Asp Glu Thr Ala Ile Lys Ala Val Ile Ser Ala Ala
20 25 30

Ala Glu Gln His Met Gln Tyr Val Glu Leu His Leu Ser Asp Asn Glu
35 40 45

His Leu Cys Phe Gln Ser Ala Tyr Leu Gly Asn Ala Ala Ser Ala Thr
50 55 60

Val Leu Ser Ala Thr Thr Leu Glu Gln Leu Val Ala Tyr Ala Asn Gln
65 70 75 80

Leu Asn Ile Glu Leu Val Pro Asp Val Asp Leu Pro Ser His Ala Gly
85 90 95

Ala Ile Leu Arg Gln Leu Gln Gln Thr His Pro Asp Ile Tyr Asn Thr
100 105 110

Val Lys Leu Asp Asp Glu Thr Ile Asp Tyr Thr Lys Pro Ala Ala Val
115 120 125

```
Ser Leu Ala Thr Thr Leu Tyr Gly Glu Leu Asp Ala Ser Phe Asn Asn
    130                 135                 140

Gln Ser Gln His Asp Leu Met Leu Gly Ala Asp Glu Val Ser Gly Ser
145                 150                 155                 160

Ala Ser Ala Tyr Ile Glu Leu Thr Thr Phe Ile Asn Gln Val Ser Arg
                165                 170                 175

Phe Gln Asn Gln Asn Gly Phe Asn Thr Ser Ile Trp Asn Asp Ser Leu
            180                 185                 190

Leu Lys Asn Glu Leu Asn Arg Leu Asp Ser Asn Ile Thr Ile Asn Tyr
        195                 200                 205

Trp Ser Gln Ser Gly Asn Asn Thr Asp Ala Ala Ile Ile Ala Asp Arg
    210                 215                 220

Tyr Ala Asn Arg Ala Ser Val Pro Asp Ile Leu Ala Ser Gly His Pro
225                 230                 235                 240

Ile Val Asn Cys Asn Ser Tyr Ala Thr Tyr Tyr Gln Phe Lys Asn Ile
                245                 250                 255

Gly Asn Val Asn Asp Asp Asn Tyr Phe Ile Asn Tyr Leu Asn His Thr
            260                 265                 270

Phe Arg Pro Asn Ile Phe Asn Glu Ile Asp Thr Asn Gly His Asn Gln
        275                 280                 285

Asp Trp Thr Ile Glu Asp Gly Val Thr Thr Asn Gly Ile Leu Val Ser
    290                 295                 300

Leu Trp Gly Ala Asp Ser Glu His Val Thr Pro Thr Ala Ile Val Asn
305                 310                 315                 320

Phe Ile Lys Arg Met Ala Ile Pro Arg Ser Phe
                325                 330

<210>  10
<211>  27
<212>  PRT
<213>  artificial

<220>
<223>  Signal peptide

<400>  10

Met Lys Lys Pro Leu Gly Lys Ile Val Ala Ser Thr Ala Leu Leu Ile
```

1               5                   10                  15

Ser Val Ala Phe Ser Ser Ser Ile Ala Ser Ala
            20                  25

<210> 11
<211> 8
<212> PRT
<213> artificial

<220>
<223> His-tag

<400> 11

His His His His His His Pro Arg
1               5

<210> 12
<211> 4
<212> PRT
<213> artificial

<220>
<223> motif

<220>
<221> misc
<222> (2)..(2)
<223> Xaa= any natural occuring amino acids

<400> 12

Gly Xaa Asp Glu
1

<210> 13
<211> 7
<212> PRT
<213> artificial

<220>
<223> motif

<220>
<221> misc
<222> (1)..(1)
<223> Xaa = E (Glu) or Q (Gln)

<220>
<221> misc
<222> (2)..(2)
<223> Xaa = N (Asn) or R (Arg) or S (Ser) or H (His) or A (Ala)

<220>
<221> misc
<222> (3)..(3)

<223> Xaa = Y (Tyr) or V (Val) or F (Phe) or L (Leu)

<220>
<221> misc
<222> (4)..(4)
<223> Xaa = A (Ala) or G (Gly) or S (Ser) or T (Thr) or C (Cys)

<220>
<221> misc
<222> (5)..(5)
<223> Xaa =I (Ile) or V (Val) or L (Leu) or F (Phe)

<220>
<221> misc
<222> (6)..(6)
<223> Xaa =E (Glu) or A (Ala) or Q (Gln) or Y (Tyr) or N (Asn)

<220>
<221> misc
<222> (7)..(7)
<223> Xaa =S(Ser) or N (Asn)

<400> 13

Xaa Xaa Xaa Xaa Xaa Xaa Xaa
1               5

<210> 14
<211> 8
<212> PRT
<213> artificial

<220>
<223> motif

<220>
<221> misc
<222> (1)..(1)
<223> Xaa = V(Val) or L (Leu) or I (Ile) or M (Met)

<220>
<221> misc
<222> (2)..(2)
<223> Xaa = L (Leu) or I (Ile) or V(Val)

<220>
<221> misc
<222> (4)..(4)
<223> Xaa = G (Gly) or A (Ala) or V (Val)

<220>
<221> misc
<222> (7)..(7)
<223> Xaa = V (Val) or I (Ile)

<220>
<221> misc
<222> (8)..(8)
<223> Xaa = P (Pro) or S (Ser) or A (Ala)

<400> 14

```
Xaa Xaa Gly Xaa Asp Glu Xaa Xaa
1               5


<210>  15
<211>  7
<212>  PRT
<213>  artificial

<220>
<223>  SEQ ID NO 15


<220>
<221>  misc
<222>  (1)..(1)
<223>  Xaa = G(Gly) or K (Lys)

<220>
<221>  misc
<222>  (3)..(3)
<223>  Xaa = I (Ile) or L (Leu)

<220>
<221>  misc
<222>  (4)..(4)
<223>  Xaa = I (Ile) or L (Leu)

<220>
<221>  misc
<222>  (5)..(5)
<223>  Xaa = K (Lys) or S (Ser) or R (Arg)

<220>
<221>  misc
<222>  (6)..(6)
<223>  Xaa = L(Leu) or Q(Gln)

<400>  15

Xaa Ala Xaa Xaa Xaa Xaa Leu
1               5


<210>  16
<211>  1524
<212>  DNA
<213>  Streptococcus merionis


<220>
<221>  CDS
<222>  (1)..(1521)

<220>
<221>  sig_peptide
<222>  (1)..(75)

<220>
<221>  mat_peptide
<222>  (76)..(1521)
```

```
<400>  16
atg cat aag gtt aag gta ctt tta ggg acg gtt ctg ctc ttt tta aca           48
Met His Lys Val Lys Val Leu Leu Gly Thr Val Leu Leu Phe Leu Thr
-25                 -20                 -15                 -10

cta ttg ttg aca gga cag cca gag gca caa gaa cct atc gtt aaa ctc           96
Leu Leu Leu Thr Gly Gln Pro Glu Ala Gln Glu Pro Ile Val Lys Leu
                -5              -1  1               5

tca ggc gga gtg atg gtg gat gtg gct agg aga tat tac tct cta aac          144
Ser Gly Gly Val Met Val Asp Val Ala Arg Arg Tyr Tyr Ser Leu Asn
        10                  15                  20

tct ctg aaa tct atc att gat act gtt tcg gag aat aaa gga gac ttt          192
Ser Leu Lys Ser Ile Ile Asp Thr Val Ser Glu Asn Lys Gly Asp Phe
    25                  30                  35

gtt cat ctc cac ttg aca gat gat caa aat tat ggg ttg gag agt cag          240
Val His Leu His Leu Thr Asp Asp Gln Asn Tyr Gly Leu Glu Ser Gln
40                  45                  50                  55

ttt ctc aat caa aca gct tca aat gca atc tat aat caa gat gat caa          288
Phe Leu Asn Gln Thr Ala Ser Asn Ala Ile Tyr Asn Gln Asp Asp Gln
                60                  65                  70

agc tat act aat cct aat acg aat cga aaa ttt ctc agt tat gga cag          336
Ser Tyr Thr Asn Pro Asn Thr Asn Arg Lys Phe Leu Ser Tyr Gly Gln
            75                  80                  85

ttg gct gag ctt aaa agt tat gcg ggt tca aaa ggc atc cga ttg ata          384
Leu Ala Glu Leu Lys Ser Tyr Ala Gly Ser Lys Gly Ile Arg Leu Ile
        90                  95                  100

cca gaa att gac acc cca gct cat aca gga ggg cta aag gcc ttg ctt          432
Pro Glu Ile Asp Thr Pro Ala His Thr Gly Gly Leu Lys Ala Leu Leu
    105                 110                 115

ccc tac gct gag cca gca gtt act agt caa ttt aag tgg gta tct tgg          480
Pro Tyr Ala Glu Pro Ala Val Thr Ser Gln Phe Lys Trp Val Ser Trp
120                 125                 130                 135

gac gaa gat agg caa cta gat ctt gat gca gct act act cag gaa gct          528
Asp Glu Asp Arg Gln Leu Asp Leu Asp Ala Ala Thr Thr Gln Glu Ala
                140                 145                 150

gtt aga cag tta tat atg gaa cta gtc cga gaa ttg cct ggc tta gag          576
Val Arg Gln Leu Tyr Met Glu Leu Val Arg Glu Leu Pro Gly Leu Glu
            155                 160                 165

tat atc cat att gga ggc gat gaa att tct gga gga ctt ata caa ggc          624
Tyr Ile His Ile Gly Gly Asp Glu Ile Ser Gly Gly Leu Ile Gln Gly
        170                 175                 180

cag tct ttc att agt cat gtg aat cag tta tgc gac tat tta gca ggt          672
Gln Ser Phe Ile Ser His Val Asn Gln Leu Cys Asp Tyr Leu Ala Gly
    185                 190                 195

caa ggt atc aaa act caa ata tgg aat gat agc tta tcc cgt caa ctg          720
Gln Gly Ile Lys Thr Gln Ile Trp Asn Asp Ser Leu Ser Arg Gln Leu
200                 205                 210                 215

cta cct tct tta aat cgt aac gtt gag att gct tat tgg ggg tat ctt          768
Leu Pro Ser Leu Asn Arg Asn Val Glu Ile Ala Tyr Trp Gly Tyr Leu
```

```
                220                 225                 230

cct cac aga aat cca gat tta gca aca gct tct gac ctt agt gat cag      816
Pro His Arg Asn Pro Asp Leu Ala Thr Ala Ser Asp Leu Ser Asp Gln
            235                 240                 245

gat ttt aag ctc ctt aat tat aat ggg tat tat cta gct ttt gtt cct      864
Asp Phe Lys Leu Leu Asn Tyr Asn Gly Tyr Tyr Leu Ala Phe Val Pro
        250                 255                 260

aag ccc tca gaa aaa ctt caa tcg gat gcc cta ttt gca gca aat gat      912
Lys Pro Ser Glu Lys Leu Gln Ser Asp Ala Leu Phe Ala Ala Asn Asp
    265                 270                 275

att cta aag act tgg aat ctt tcc cag ttt cat atg gat acg ggt gat      960
Ile Leu Lys Thr Trp Asn Leu Ser Gln Phe His Met Asp Thr Gly Asp
280                 285                 290                 295

tcc att aat agt tta aag aat gtc att gga gcg gct ttt tcc ata tgg     1008
Ser Ile Asn Ser Leu Lys Asn Val Ile Gly Ala Ala Phe Ser Ile Trp
                300                 305                 310

agt gaa gag tca gct ggt ctg act gac gag gag ata ttt tct gcc atg     1056
Ser Glu Glu Ser Ala Gly Leu Thr Asp Glu Glu Ile Phe Ser Ala Met
            315                 320                 325

ggt agt ccg att cga gct ctt ttg aca gtt atc aat caa gaa aat ata     1104
Gly Ser Pro Ile Arg Ala Leu Leu Thr Val Ile Asn Gln Glu Asn Ile
        330                 335                 340

aag aga aac gag aat acg aca act acc acc acc gag tca atg act gag     1152
Lys Arg Asn Glu Asn Thr Thr Thr Thr Thr Thr Glu Ser Met Thr Glu
    345                 350                 355

gct acg aca act att aca act gag ccg aca acc caa tca acc aca gaa     1200
Ala Thr Thr Thr Ile Thr Thr Glu Pro Thr Thr Gln Ser Thr Thr Glu
360                 365                 370                 375

agt acg aca act act aca acc gag tca acg aca gag act acg aca act     1248
Ser Thr Thr Thr Thr Thr Thr Glu Ser Thr Thr Glu Thr Thr Thr Thr
                380                 385                 390

gtc aca act gag tca aca act aag tcg acc aca gaa ggc acg aca gaa     1296
Val Thr Thr Glu Ser Thr Thr Lys Ser Thr Thr Glu Gly Thr Thr Glu
            395                 400                 405

aca aca acc cct atc cca cca atg cct cag cct aca acg tct cct gag     1344
Thr Thr Thr Pro Ile Pro Pro Met Pro Gln Pro Thr Thr Ser Pro Glu
        410                 415                 420

aca agt aca gct aca cat gca aca acg act aac cca agt aca tca aaa     1392
Thr Ser Thr Ala Thr His Ala Thr Thr Thr Asn Pro Ser Thr Ser Lys
    425                 430                 435

gat ggc aat aaa ctg tct aaa tca aaa cgg ata ttg cca agt aca ggt     1440
Asp Gly Asn Lys Leu Ser Lys Ser Lys Arg Ile Leu Pro Ser Thr Gly
440                 445                 450                 455

gaa acg att ggt gtc ctt tca gta gca gga ttg gcg ctc ttc ttg ttt     1488
Glu Thr Ile Gly Val Leu Ser Val Ala Gly Leu Ala Leu Phe Leu Phe
                460                 465                 470

gtt ggg ctt aca tat tac cgt cac aag aag aat taa                     1524
```

Val Gly Leu Thr Tyr Tyr Arg His Lys Lys Asn
475 480

<210> 17
<211> 507
<212> PRT
<213> Streptococcus merionis

<400> 17

Met His Lys Val Lys Val Leu Leu Gly Thr Val Leu Leu Phe Leu Thr
-25 -20 -15 -10

Leu Leu Leu Thr Gly Gln Pro Glu Ala Gln Glu Pro Ile Val Lys Leu
-5 -1 1 5

Ser Gly Gly Val Met Val Asp Val Ala Arg Arg Tyr Tyr Ser Leu Asn
10 15 20

Ser Leu Lys Ser Ile Ile Asp Thr Val Ser Glu Asn Lys Gly Asp Phe
25 30 35

Val His Leu His Leu Thr Asp Asp Gln Asn Tyr Gly Leu Glu Ser Gln
40 45 50 55

Phe Leu Asn Gln Thr Ala Ser Asn Ala Ile Tyr Asn Gln Asp Asp Gln
60 65 70

Ser Tyr Thr Asn Pro Asn Thr Asn Arg Lys Phe Leu Ser Tyr Gly Gln
75 80 85

Leu Ala Glu Leu Lys Ser Tyr Ala Gly Ser Lys Gly Ile Arg Leu Ile
90 95 100

Pro Glu Ile Asp Thr Pro Ala His Thr Gly Gly Leu Lys Ala Leu Leu
105 110 115

Pro Tyr Ala Glu Pro Ala Val Thr Ser Gln Phe Lys Trp Val Ser Trp
120 125 130 135

Asp Glu Asp Arg Gln Leu Asp Leu Asp Ala Ala Thr Thr Gln Glu Ala
140 145 150

Val Arg Gln Leu Tyr Met Glu Leu Val Arg Glu Leu Pro Gly Leu Glu
155 160 165

Tyr Ile His Ile Gly Gly Asp Glu Ile Ser Gly Gly Leu Ile Gln Gly
170 175 180

Gln Ser Phe Ile Ser His Val Asn Gln Leu Cys Asp Tyr Leu Ala Gly
185 190 195

Gln Gly Ile Lys Thr Gln Ile Trp Asn Asp Ser Leu Ser Arg Gln Leu
200 205 210 215

Leu Pro Ser Leu Asn Arg Asn Val Glu Ile Ala Tyr Trp Gly Tyr Leu
220 225 230

Pro His Arg Asn Pro Asp Leu Ala Thr Ala Ser Asp Leu Ser Asp Gln
235 240 245

Asp Phe Lys Leu Leu Asn Tyr Asn Gly Tyr Tyr Leu Ala Phe Val Pro
250 255 260

Lys Pro Ser Glu Lys Leu Gln Ser Asp Ala Leu Phe Ala Ala Asn Asp
265 270 275

Ile Leu Lys Thr Trp Asn Leu Ser Gln Phe His Met Asp Thr Gly Asp
280 285 290 295

Ser Ile Asn Ser Leu Lys Asn Val Ile Gly Ala Ala Phe Ser Ile Trp
300 305 310

Ser Glu Glu Ser Ala Gly Leu Thr Asp Glu Glu Ile Phe Ser Ala Met
315 320 325

Gly Ser Pro Ile Arg Ala Leu Leu Thr Val Ile Asn Gln Glu Asn Ile
330 335 340

Lys Arg Asn Glu Asn Thr Thr Thr Thr Thr Thr Glu Ser Met Thr Glu
345 350 355

Ala Thr Thr Thr Ile Thr Thr Glu Pro Thr Thr Gln Ser Thr Thr Glu
360 365 370 375

Ser Thr Thr Thr Thr Thr Thr Glu Ser Thr Thr Glu Thr Thr Thr Thr
380 385 390

Val Thr Thr Glu Ser Thr Thr Lys Ser Thr Thr Glu Gly Thr Thr Glu
395 400 405

Thr Thr Thr Pro Ile Pro Pro Met Pro Gln Pro Thr Thr Ser Pro Glu
410 415 420

Thr Ser Thr Ala Thr His Ala Thr Thr Thr Asn Pro Ser Thr Ser Lys
425 430 435

Asp Gly Asn Lys Leu Ser Lys Ser Lys Arg Ile Leu Pro Ser Thr Gly
440 445 450 455

Glu Thr Ile Gly Val Leu Ser Val Ala Gly Leu Ala Leu Phe Leu Phe
460 465 470

Val Gly Leu Thr Tyr Tyr Arg His Lys Lys Asn
475 480

<210> 18
<211> 482
<212> PRT
<213> Streptococcus merionis

<400> 18

Gln Glu Pro Ile Val Lys Leu Ser Gly Gly Val Met Val Asp Val Ala
1 5 10 15

Arg Arg Tyr Tyr Ser Leu Asn Ser Leu Lys Ser Ile Ile Asp Thr Val
20 25 30

Ser Glu Asn Lys Gly Asp Phe Val His Leu His Leu Thr Asp Asp Gln
35 40 45

Asn Tyr Gly Leu Glu Ser Gln Phe Leu Asn Gln Thr Ala Ser Asn Ala
50 55 60

Ile Tyr Asn Gln Asp Asp Gln Ser Tyr Thr Asn Pro Asn Thr Asn Arg
65 70 75 80

Lys Phe Leu Ser Tyr Gly Gln Leu Ala Glu Leu Lys Ser Tyr Ala Gly
85 90 95

Ser Lys Gly Ile Arg Leu Ile Pro Glu Ile Asp Thr Pro Ala His Thr
100 105 110

Gly Gly Leu Lys Ala Leu Leu Pro Tyr Ala Glu Pro Ala Val Thr Ser
115 120 125

Gln Phe Lys Trp Val Ser Trp Asp Glu Asp Arg Gln Leu Asp Leu Asp
130 135 140

Ala Ala Thr Thr Gln Glu Ala Val Arg Gln Leu Tyr Met Glu Leu Val
145 150 155 160

Arg Glu Leu Pro Gly Leu Glu Tyr Ile His Ile Gly Gly Asp Glu Ile
165 170 175

```
Ser Gly Gly Leu Ile Gln Gly Gln Ser Phe Ile Ser His Val Asn Gln
            180                 185                 190

Leu Cys Asp Tyr Leu Ala Gly Gln Gly Ile Lys Thr Gln Ile Trp Asn
        195                 200                 205

Asp Ser Leu Ser Arg Gln Leu Leu Pro Ser Leu Asn Arg Asn Val Glu
    210                 215                 220

Ile Ala Tyr Trp Gly Tyr Leu Pro His Arg Asn Pro Asp Leu Ala Thr
225                 230                 235                 240

Ala Ser Asp Leu Ser Asp Gln Asp Phe Lys Leu Leu Asn Tyr Asn Gly
                245                 250                 255

Tyr Tyr Leu Ala Phe Val Pro Lys Pro Ser Glu Lys Leu Gln Ser Asp
            260                 265                 270

Ala Leu Phe Ala Ala Asn Asp Ile Leu Lys Thr Trp Asn Leu Ser Gln
        275                 280                 285

Phe His Met Asp Thr Gly Asp Ser Ile Asn Ser Leu Lys Asn Val Ile
    290                 295                 300

Gly Ala Ala Phe Ser Ile Trp Ser Glu Glu Ser Ala Gly Leu Thr Asp
305                 310                 315                 320

Glu Glu Ile Phe Ser Ala Met Gly Ser Pro Ile Arg Ala Leu Leu Thr
                325                 330                 335

Val Ile Asn Gln Glu Asn Ile Lys Arg Asn Glu Asn Thr Thr Thr Thr
            340                 345                 350

Thr Thr Glu Ser Met Thr Glu Ala Thr Thr Thr Ile Thr Thr Glu Pro
        355                 360                 365

Thr Thr Gln Ser Thr Thr Glu Ser Thr Thr Thr Thr Thr Thr Glu Ser
    370                 375                 380

Thr Thr Glu Thr Thr Thr Thr Val Thr Thr Glu Ser Thr Thr Lys Ser
385                 390                 395                 400

Thr Thr Glu Gly Thr Thr Glu Thr Thr Thr Pro Ile Pro Pro Met Pro
                405                 410                 415

Gln Pro Thr Thr Ser Pro Glu Thr Ser Thr Ala Thr His Ala Thr Thr
            420                 425                 430
```

```
Thr Asn Pro Ser Thr Ser Lys Asp Gly Asn Lys Leu Ser Lys Ser Lys
        435                 440                 445

Arg Ile Leu Pro Ser Thr Gly Glu Thr Ile Gly Val Leu Ser Val Ala
    450                 455                 460

Gly Leu Ala Leu Phe Leu Phe Val Gly Leu Thr Tyr Tyr Arg His Lys
465                 470                 475                 480

Lys Asn
```

## Description

### Reference to a Sequence Listing

This application contains a Sequence Listing in computer readable form, which is incorporated herein by reference.

### Background of the Invention

The present invention relates to specific cleaning compositions, as defined herein, comprising enzymes having hexosaminidase activity such as dispersins obtained from *Lactobacillus* or *Streptococcus.* The invention further relates to methods and use of said compositions comprising such enzymes in cleaning processes e.g. for stain removal.

### Description of the Related Art

Enzymes have been used in detergents for decades. Usually a cocktail of various enzymes is added to detergent compositions. The enzyme cocktail often comprises various enzymes, wherein each enzyme targets a specific substrate e.g. amylases are active towards starch stains, proteases on protein stains and so forth. Textiles surface and hard surfaces, such as dishes or the inner space of a laundry machine enduring a number of wash cycles, become soiled with many different types of soiling which may compose of proteins, grease, starch etc. One type of stain may compose of organic matter, such as cell debris, biofilm, EPS, etc. Polypeptides having hexosaminidase activity include Dispersins such as Dispersin B (DspB), which are described as β-N-acetyigiucosaminidases belonging to the Glycoside Hydrolase 20 family. WO04061117 A2 (Kane Biotech INC) describe use of compositions comprising DspB for reducing and preventing biofilm caused by poly-N-acetylglucosamine-producing bacteria and Kane et al. describes the use of compositions comprising dispersins for reducing biofilm on medical devises and for wound care. The application WO9850512 (Procter and Gamble) disclose laundry or cleaning products comprising one or more hexosaminidase enzymes. The present invention provides suitable enzymes for use in detergents and for deep cleaning of items during laundry and cleaning process.

### Summary of the Invention

A first aspect of the invention relates to a cleaning composition comprising a *Lactobacillus* or *Streptococcus* hexosaminidase,
wherein the cleaning composition

(a) is a solid, preferably granular, laundry detergent composition and further comprises

(a1) at least one zeolite builder, preferably in an amount of 10 to 50 wt.-%, more preferably 20-30 wt.-%;
(a2) at least one phosphonate builder, preferably in an amount of 0.1 to 5 wt.-%, more preferably 0.4 to 1.5 wt.-%;
(a3) at least one further enzyme, preferably a cellulase, preferably in an amount of active enzyme of 100 to 5000 ppb, more preferably 1000 to 2000 ppb; and
(a4) at least one polymer, preferably a polyvinylpyrrolidon polymer, preferably in an amount of 0.01 to 1 wt.-%, more preferably 0.1 to 0.3 wt.-%; or

(b) is a solid laundry detergent composition and further comprises

(b1) at least one silicate builder, preferably in an amount of 2 to 20 wt.-%, more preferably 5-10 wt.-%; (b2) optionally carboxymethylcellulose, preferably in an amount of 0.1 to 10 wt.-%, more preferably 0.1 to 4 wt.-%;
(b3) at least one further enzyme, preferably a cellulase, preferably in an amount of active enzyme of 0.1 to 100 ppm, more preferably 0.1 to 10 ppm;
(b4) optionally at least one soil release polymer, preferably a polyvinylpyrrolidon polymer, in an amount of 0.1 to 3 wt.-%, more preferably 0.1 to 1.0 wt.-%; and
(b5) at least one bleaching system, comprising a bleaching agent, a bleach activator and a bleach catalyst, preferably in an amount of 0.1 to 50 wt.-%, more preferably 0.1 to 30 wt.-%; or

(c) is liquid laundry detergent composition and further comprises

(c1) at least one surfactant, preferably nonionic surfactant, preferably in an amount of 1 to 20 wt.-%, preferably

3 to 15 wt.-%;
(c2) optionally at least one phosphonate builder, preferably in an amount of 0.1 to 3 wt.-%, more preferably 0.25 to 1.5 wt.-%
(c3) optionally at least at least one further enzyme, preferably a cellulase, preferably in an amount of enzyme composition of 0.001 to 1 wt.-%, more preferably 0.001 to 0.6 wt.-%; and
(c4) optionally at least one organic solvent, preferably glycerol, preferably in an amount of 0.1 to 10 wt.-%, more preferably 0.1 to 5 wt.-%; or

(d) is a liquid laundry detergent in unit dose form, preferably a pouch comprising a water-soluble film, and further comprises

(d1) water in an amount of up to 20 wt.-%, preferably 5 to 15 wt.-%;
(d2) optionally at least one bittering agent, preferably Benzyldiethyl(2,6-xylylcarbamoyl)-methylammoniumbenzoate, preferably in an amount of 0.00001 to 0.04 wt.-%;
(d3) optionally at least one optical brightener, preferably in an amount of 0.01 to 2 wt.-%, more preferably 0.01 to 1 wt.-%; and
(d4) optionally at least one polymer, preferably in an amount of 0.01 to 7 wt.-%, more preferably 0.1 to 5 wt.-%; or

(e) is a fabric finisher and further comprises

(e1) at least one softening silicone, preferably an amino-functionalized silicone, preferably in an amount of 0.1 to 10 wt.-%, more preferably 0.1 to 2 wt.-%;
(e2) at least one perfume, preferably at least partially encapsulated in microcapsules, more preferably at least partially encapsulated in melamine-formaldehyde microcapsules, preferably in an amount of 0.01 to 3 wt.-%, more preferably 0.1 to 1 wt.-%;
(e3) optionally polyquaternium 10 in an amount of 0.1 to 20 wt.-%, preferably 0.1 to 13 wt.-%;
(e4) optionally polyquaternium 37 in an amount of 0.1 to 20 wt.-%, preferably 0.1 to 13 wt.-%;
(e5) optionally a plant-based esterquat, preferably a canola- or palm-based esterquat, in an amount of 0.1 to 20 wt.-%, preferably 0.1 to 13 wt.-%; and
(e6) optionally adipic acid, in an amount of 0.1 to 20 wt.-%, preferably 0.1 to 13 wt.-%; or

(f) is an acidic cleaning agent, preferably having a pH less than 6, and further comprises

(f1) plant-based or bio-based surfactants, preferably each in an amount of 0.1 to 5, more preferably each in an amount of 0.1 to 2 wt.-%;
(f2) at least one acidic biocide, preferably selected from acids, more preferably HCl and formic acid; and
(f3) at least one soil release, water repellant or water spreading polymer, preferably in an amount of 0.01 to 3 wt.-%, more preferably 0.01 to 0.5 wt.-%; or

(g) is a neutral cleaning agent, preferably having a pH between 6.0 and 7.5, and further comprises

(g1) plant-based or bio-based surfactants, preferably each in an amount of 0.1 to 5, more preferably each in an amount of 0.1 to 2 wt.-%;
(g2) at least one biocide, preferably selected from quaternary ammonium compounds and alcohols; and
(g3) at least one soil release, water repellant or water spreading polymer, preferably in an amount of 0.01 to 3 wt.-%, more preferably 0.01 to 0.5 wt.-%; or

(h) is an alkaline cleaning agent, preferably having a pH of more than 7.5, and further comprises

(h1) plant-based or bio-based surfactants, preferably each in an amount of 0.1 to 5, more preferably each in an amount of 0.1 to 2 wt.-%; or

(i) is a hand dishwashing agent, preferably liquid hand dishwashing agent, and further comprises

(i1) at least one anionic surfactant, preferably in an amount of 0.1 to 40 wt.-%, more preferably 5 to 30 wt.-%;
(i2) at least one amphoteric surfactant, preferably betain, preferably in an amount of 0.1 to 25 wt.-%, more preferably 1 to 15 wt.-%;
(i3) at least one nonionic surfactant, preferably in an amount of 0.1 to 25 wt.-%, more preferably 2 to 10 wt.-%;

(i4) at least one further enzyme, preferably selected from proteases, amylases and combinations thereof, preferably in an amount of enzyme composition of up to 1 wt.-%, more preferably up to 0.6 wt.-%; or

(j) is an automatic dishwashing composition and further comprises

(j1) at least one builder selected from citrate, aminocarboxylates and combinations thereof, preferably in an amount of 5 to 30 wt.-%, more preferably 10 to 20 wt.-%;
(j2) at least one phosphonate builder, preferably in an amount of 0.1 to 5 wt.-%, more preferably 0.4 to 1.5 wt.-%;
(j3) at least one nonionic surfactant, preferably in an amount of 0.1 to 10 wt.-%, more preferably 1 to 5 wt.-%;
(j4) at least one bleaching system, comprising a bleaching agent, a bleach activator and a bleach catalyst, preferably in an amount of 0.1 to 50 wt.-%, more preferably 0.1 to 30 wt.-%; and
(j5) at least one polymer selected from sulfopolymers, cationic polymers and polyacrylates, preferably in an amount of 0.01 to 15 wt.-%, more preferably 2 to 10 wt.-%; or

(k) further comprises

(k1) at least one sulfopolymer, preferably in an amount of 1 to 15, more preferably 2 to 10 wt.-% and is preferably a dishwashing, more preferably an automatic dishwashing composition; or

(l) further comprises at least one adjunct ingredient selected from probiotics, preferably microbes, spores or combinations thereof; or
(m) is in unit dose form and comprises at least 2, preferably 2, 3, 4 or 5 separate compartments; or
(n) is a phosphate-free composition;

wherein the composition optionally further comprises;

(a)

i. one or more polyol(s), preferably selected from glycerol, (mono, di, or tri) propylene glycol, ethylene glycol, polyethylene glycol, sugar alcohols, sorbitol, mannitol, erythritol, dulcitol, inositol, xylitol and adonitol,
ii. optionally one or more enzyme, preferably selected from proteases, amylases or lipases,
iii. optionally one or more surfactant, preferably selected from anionic and nonionic surfactants,
iv. optionally one or more polymer;

or
(b) a granule comprising

i. a core comprising a *Lactobacillus hexosaminidase* and optionally,
ii. a coating consisting of one or more layer(s) surrounding the core.

When in the following reference is made to "compositions of the invention" or "compositions as described herein", the above-specified compositions (a)-(n) are meant. Furthermore, if not indicated otherwise, all references to percentages in relation to the disclosed compositions relate to wt % relative to the total weight of the respective composition. It is understood that when reference is made to compositions that contain a hexosaminidase as defined herein, the respective composition contains at least one of said hexoaminidases but can also comprise two or more of them.
The hexosaminidase preferably has N-acetylglucosaminidase activity, preferably β-1,6 N-acetylglucosaminidase activity
The present invention further relates to a cleaning composition, as defined herein, comprising at least 0.01 mg *Lactobacillus* or *Streptococcus* hexosaminidase and optionally a further cleaning component, wherein the cleaning component is

(a) at least one surfactant;
(b) at least one builder; or
(c) at least one polymer.

The invention further relates to the use of a composition according to the invention for cleaning of an item, wherein the item is a textile or a surface.
The invention further relates to the use of a cleaning composition such as a detergent composition comprising a *Lacto-*

*bacillus* or *Streptococcus* hexosaminidase as defined herein,

a) for preventing, reducing or removing stickiness of the item;
b) for pretreating stains on the item;
c) for preventing, reducing or removing redeposition of soil during a wash cycle;
d) for preventing, reducing or removing adherence of soil to the item;
e) for maintaining or improving whiteness of the item;
f) for preventing, reducing or removing malodor from the item, wherein the item is a textile.

The invention further relates to a method of formulating a cleaning composition as defined herein comprising adding a *Lactobacillus* or *Streptococcus* hexosaminidase and at least one cleaning component.
The invention further relates to a method of treating a fabric comprising;

(a) contacting the fabric with a composition as defined herein or an aqueous solution thereof;
(b) and optionally rinsing and drying the textile.

The invention also relates to a method for cleaning or laundering an item comprising the steps of:

(a) exposing an item to a cleaning composition of the invention or a wash liquor comprising a cleaning composition of the invention;
(b) completing at least one wash cycle; and
(c) optionally rinsing the item, wherein the item is a fabric.

**Figures**

(Figure 1). In one aspect the polypeptides comprised in the compositions of the invention e.g. all belong to the *Lactobacillus* clade, which is illustrated as a phylogenetic tree in Figure 1. The *Lactobacillus* clade or clade of *Lactobacillus* is a group of enzymes all related to the same ancestor and share common properties of taxonomic order *Lactobacillales.* Polypeptides forming a group within the clade (a subclade) of the phylogenetic tree can also share common properties and are more closely related than other polypeptides in the clade.
Figure 2 An alignment of the *Lactobacillus* polypeptides disclosed in accordance with the invention

**Overview of sequences of the Lactobacillus clade**

SEQ ID NO 1 is the DNA encoding the full-length polypeptide from *Lactobacillus paraplantarum* DSM 10667
SEQ ID NO 2 is the polypeptide derived from SEQ ID NO 1
SEQ ID NO 3 is the mature polypeptide of SEQ ID NO 2
SEQ ID NO 4 is the DNA encoding the full-length polypeptide from *Lactobacillus apinorum*
SEQ ID NO 5 is the polypeptide derived from SEQ ID NO 4
SEQ ID NO 6 is the mature polypeptide of SEQ ID NO 5
SEQ ID NO 7 is the DNA encoding the full-length polypeptide from *Lactobacillus paraplantarum*
SEQ ID NO 8 is the polypeptide derived from SEQ ID NO 7
SEQ ID NO 9 is the mature polypeptide of SEQ ID NO 8
SEQ ID NO 10 is the Bacillus clausii secretion signal
SEQ ID NO 11 is a His-tag sequence
SEQ ID NO 12 is the polypeptide motif GXDE
SEQ ID NO 13 is the polypeptide motif [EQ][NRSHA][YVFL][AGSTC][IVLF][EAQYN][SN]
SEQ ID NO 14 is the polypeptide motif [VLIM][LIV]G[GAV]DE[VI][PSA]
SEQ ID NO 15 is the polypeptide motif [GK]A[IL][IL][KSR][LQ]L
SEQ ID NO 16 is the DNA encoding the full-length polypeptide from *Streptococcus merionis*
SEQ ID NO 17 is the polypeptide derived from SEQ ID NO 16
SEQ ID NO 18 is the mature polypeptide of SEQ ID NO 17.

## Detailed Description of the Invention

Various enzymes are applied in cleaning processes each targeting specific types of soiling such as protein, starch and grease soiling. Enzymes are standard ingredients in detergents for laundry and dish wash. The effectiveness of these commercial enzymes provides detergents which removes much of the soiling. However, organic stains such as EPS (extracellular polymeric substance) comprised in much biofilm constitute a challenging type of soiling due to the complex nature of such organic matters. EPS is mostly composed of polysaccharides (exopolysaccharides) e.g. PNAG (poly-N-acetylglucosamine) and proteins, but include other macro-molecules such as eDNA, lipids and other organic substances. Organic stains, like biofilm or components hereof, such as PNAG may be sticky or glueing, which when present on textile, may give rise to redeposition or backstaining of soil resulting in a greying of the textile. Further, when dirty laundry items are washed together with less dirty laundry items the dirt present in the wash liquor tend to stick to organic stains e.g. biofilm or biofilm components as a result, hereof the laundry item is more "soiled" after wash than before wash. This effect may also be termed re-deposition. Another drawback of organic stains is the malodor as various malodor related molecules are often associated with organic stains such as biofilm.

The present invention relates to specific cleaning compositions comprising polypeptides, the use and methods of use thereof, said compositions comprising hexosaminidases preferably obtained from the taxonomic order of *Lactobacillales,* preferably from the genus *Lactobacillus.* The terms "*Lactobacillus* hexosaminidase" and "hexosaminidase obtained from *Lactobacillus* may be used interchangeably throughout. The present invention further relates to specific cleaning compositions comprising polypeptides, the use and methods of use thereof, said compositions comprising hexosaminidases obtained from the taxonomic order of *Streptococcus.* The terms "*Streptococcus* hexosaminidase" and "hexosaminidase obtained from *Streptococcus*" may be used interchangeably throughout.

The hexosaminidases are preferably dispersins and comprises N-acetylglucosaminidase and/or β-1,6-N-acetylglucosamininidase activity.

## Polypeptides having hexosaminidase activity

Dispersin: The term "dispersin" and the abbreviations "Dsp" or "Disp" means a polypeptide having hexosaminidase activity, EC 3.2.1.- that catalyzes the hydrolysis of β-1,6-glycosidic linkages of N-acetyl-glucosamine polymers (poly-N-acetylglucosamine, PNAG) found e.g. in biofilm. Thus, dispersins is an enzyme having beta-1,6 N-acetylglucosaminidase activity.

Hexosaminidase: The term "hexosaminidases" means a polypeptide having hexosaminidase activity (hexosaminidases), and includes EC 3.2.1. e.g. that catalyzes the hydrolysis of N-acetyl-D-hexosamine or N-acetyl-glucosamine polymers found e.g. in biofilm. The term includes dispersins and includes polypeptides having N-acetylglucosaminidase activity and β-1,6 N-acetylglucosaminidase activity. The term "polypeptide having hexosaminidase activity" may be used interchangeably with the term hexosaminidases and similar the term "polypeptide having beta-1,6-N-acetylglucosaminidase activity" may be used interchangeably with the term beta-1,6-N-acetylglucosamininidases. For the purposes of the present invention, hexosaminidase activity is determined according to the procedure described in Assay 1.

In a preferred aspect, the polypeptide used in the compositions of the invention is comprised in a specific clade of hexosaminidases. This clade is in the present context termed *Lactobacillus* as the hexosaminidases from the clade are obtained from bacteria within the taxonomic order of *Lactobacillales,* preferably from the *Lactobacillus* genus. The term *Lactobacillus* hexosaminidase means hexosaminidases obtained from the taxonomic order of *Lactobacillales,* preferably from the *Lactobacillus* genus. For purposes of the present invention, the term "obtained from" as used herein in connection with a given source shall mean that the polypeptide encoded by a polynucleotide is produced by the source or by a strain in which the polynucleotide from the source has been inserted. In one aspect, the polypeptide obtained from a given source is secreted extracellularly. The phylogenetic tree of the *Lactobacillus* clade is shown in Figure 1. The polypeptides of the invention are preferably obtained from *Lactobacillus paraplantarum* or *Lactobacillus apinorum.*

In another aspect, one polypeptide used in the compositions of the invention is obtained from *Streptococcus,* preferably *Streptococcus merionis.* The term *Streptococcus* hexosaminidase means hexosaminidases obtained from *Streptococcus* genus.

The polypeptides e.g. those comprised in the *Lactobacillus* clade, finds use in cleaning processes and compositions of the invention are listed in the table below. The hexosaminidases of Table 1 have beta-1,6-N-acetylglucosaminidase activity and are thus dispersins. The dispersins of this group have been found to be particularly useful in cleaning of organic stains e.g. PNAG from textiles. In particular, dispersins of Table 1 may be formulated in cleaning compositions as disclosed herein, comprising a dispersin obtained from *Lactobacillus* and a detergent adjunct. The compositions of the invention are useful in cleaning processes such as laundry and/or are useful for reduction, removal or preventing biofilm and/or for removing PNAG stains e.g. from textiles and hard surfaces.

Table 1 Hexosaminidase polypeptides having beta-1,6 N-acetylglucosaminidase activity comprised in the *Lactobacillus* clade

| | |
|---|---|
| SEQ ID NO 3 | *Lactobacillus paraplantarum DSM 10667* |
| SEQ ID NO 6 | *Lactobacillus apinorum* |
| SEQ ID NO 9 | *Lactobacillus paraplantarum* |
| UniProtKB/TrEMBL | Additional dispersins |
| J0WBI2 | *Weissella koreensis KCTC 3621* |
| A0A0P7K1K0 | *Lactobacillus kunkeei* |
| A0A0P7K6J6 | *Lactobacillus kunkeei* |
| U2W2U5 | *Lactobacillus plantarum AY01* |
| A0A1L8CGI0 | *Lactobacillus kunkeei* |
| A0A0P7K6Q2 | *Lactobacillus kunkeei* |
| A0A098R6U2 | *Lactobacillus paraplantarum* |
| A0A0P7LTY5 | *Lactobacillus kunkeei* |
| A0A0M9D336 | *Lactobacillus kunkeei* |
| A0A0R1JY61 | *Lactobacillus namurensis DSM 19117* |
| A0A0C3AIW4 | *Lactobacillus kunkeei* |
| A0A0M9DBA3 | *Lactobacillus kunkeei* |
| A0A0N0CT29 | *Lactobacillus kunkeei* |
| A0A0M9D5K5 | *Lactobacillus kunkeei* |
| A0A0R2DG13 | *Lactobacillus senmaizukei DSM 21775* |
| A0A0R1R2Q4 | *Lactobacillus spicheri DSM 15429* |
| A0A0C3AFN0 | *Lactobacillus kunkeei* |
| A0A0P7K1E6 | *Lactobacillus kunkeei* |
| A0A0M9D3N9 | *Lactobacillus apinorum* |
| A0A0R1LK94 | *Lactobacillus acidifarinae DSM 19394* |
| A0A0H4QX26 | *Lactobacillus koreensis* |
| A0A0R1FXP4 | *Lactobacillus kunkeei DSM 12361* |
| A0A179CPD2 | *Lactobacillus aviarius* |
| A0A063B938 | *Lactobacillus kunkeei EFB6* |
| F8I121 | *Weissella koreensis* |
| A0A0R1LJ27 | *Lactobacillus koreensis JCM 16448* |
| A0A1I4IHB3 | *Lactococcus garvieae* |
| A0A0P7JVB0 | *Lactobacillus kunkeei* |
| A0A1L8CFQ3 | *Lactobacillus kunkeei* |
| A0A0R1FZT1 | *Lactobacillus kunkeei DSM 12361* |
| A0A179CK20 | *Lactobacillus aviarius* |
| A0A0F3RXX7 | *Lactobacillus spicheri* |
| A0A0R2M4L4 | *Lactobacillus xiangfangensis* |
| A0A0R1 MNG9 | *Lactobacillus zymae DSM 19395* |

(continued)

| | |
|---|---|
| A0A0M9DB36 | *Lactobacillus kunkeei* |
| A0A0R1ZKJ9 | *Lactobacillus aviarius subsp. araffinosus DSM 20653* |
| A0A0P7JT37 | *Lactobacillus kunkeei* |
| A0A0R2LE82 | *Lactobacillus paucivorans* |
| A0A087EP61 | *Lactobacillus kunkeei* |
| A0A0M9DF88 | *Lactobacillus kunkeei* |
| A0A0R2AM78 | *Lactobacillus ozensis DSM 23829* |
| A0A199QF96 | *Lactobacillus plantarum* |
| A0A0M9DF94 | *Lactobacillus kunkeei* |
| A0A179C4R7 | *Lactobacillus aviarius* |
| A0A0R1R622 | *Lactobacillus paraplantarum DSM 10667* |
| A0A1Y6JTN4 | *Lactobacillus zymae* |
| A0A0R1YHA3 | *Lactobacillus aviarius subsp. aviarius DSM 20655* |

The hexosaminidases used in the compositions of the invention may be divided into clades or domain groups characterized by having various motifs. One aspect of the invention relates to hexosaminidases e.g. dispersin comprised in the FQS clade. This clade has not been described previously. The clade is termed FQS and polypeptides of this domain comprises Glyco_hydro_20 domain polypeptides of bacterial origin and are in addition to having PNAG activity, characterized by comprising certain motifs. The polypeptides of the clade comprise the motif:
[EQ][NRSHA][YVFL][AGSTC][IVLF][EAQYN][SN] (SEQ ID NO 13), corresponding to EHLCFQS at position 48 to 54 of SEQ ID NO 3.
One aspect of the invention relates to compositions as defined herein comprising hexosaminidases e.g. dispersins comprised in the GADE clade. This clade has not been described previously. The polypeptides of this clade comprise Glyco_hydro_20 domain polypeptides of bacterial origin and are in addition to having PNAG activity, characterized by comprising certain motifs. The polypeptides of the clade comprise the motif example [VLIM][LIV]G[GAV]DE[VI][PSA] (SEQ ID NO 14), corresponding to pos 151 to 158 of SEQ ID NO 3, where G and DE (corresponding to positions 153 and 155-156 of SEQ ID NO 3) are fully conserved in GADE clade and part of the active site. Residues D and E are the key catalytic residues of Glyco_hydro_20 enzymes (position 155 to 156 in SEQ ID NO 3).

One aspect of the invention relates to compositions as defined herein comprising hexosaminidases e.g. dispersins comprised in the GAIL clade This clade has not been described previously. The polypeptides of this clade comprise Glyco_hydro_20 domain polypeptides of bacterial origin and are in addition to having PNAG activity, characterized by comprising certain motifs. The polypeptides of the clade comprise the motif [GK]A[IL][IL][KSR][LQ]L (SEQ ID NO 15), corresponding to pos 96 to 102 of SEQ ID NO 3, where A and L (corresponding to positions 97 and 102 of SEQ ID NO 3) are fully conserved in GAIL clade and part of the active site.

In one aspect of the invention the compositions as defined herein comprise hexosaminidase e.g. dispersin that comprises one or more of the following motifs GXDE (SEQ ID NO 12), [EQ][NRSHA][YVFL][AGSTC][IVLF][EAQYN][SN] (SEQ ID NO 13), [VLIM][LIV]G[GAV]DE[VI][PSA] (SEQ ID NO 14), or [GK]A[IL][IL][KSR][LQ]L (SEQ ID NO 15). In one aspect, the hexosaminidases comprises the motif GXDE. In one aspect, the hexosaminidases comprises the motif [EQ][NRSHA][YVFL][AGSTC][IVLF][EAQYN][SN]. In one aspect, the hexosaminidases comprises the motif [VLIM][LIV]G[GAV]DE[VI][PSA]. In one aspect, the hexosaminidases comprises the motif [GK]A[IL][IL][KSR][LQ]L. In one aspect, the hexosaminidase comprises all four motifs GXDE (SEQ ID NO 12), [EQ][NRSHA][YVFL][AGSTC][IVLF][EAQYN][SN] (SEQ ID NO 13), [VLIM][LIV]G[GAV]DE[VI][PSA] (SEQ ID NO 14), or [GK]A[IL][IL][KSR][LQ]L (SEQ ID NO 15). In one aspect, the hexosaminidase comprises to two motifs GXDE (SEQ ID NO 12) and [EQ][NRSHA][YVFL][AGSTC][IVLF][EAQYN][SN] (SEQ ID NO 13). In one aspect, the hexosaminidase comprises to three motifs GXDE (SEQ ID NO 12), [EQ][NRSHA][YVFL][AGSTC][IVLF][EAQYN][SN] (SEQ ID NO 13) and [VLIM][LIV]G[GAV]DE[VI][PSA] (SEQ ID NO 14). In one aspect, the hexosaminidase comprises the three motifs GXDE (SEQ ID NO 12), [EQ][NRSHA][YVFL][AGSTC][IVLF][EAQYN][SN] (SEQ ID NO 13) and [GK]A[IL][IL][KSR][LQ]L (SEQ ID NO 15).

An alignment of the polypeptides useful in the compositions of the invention is shown in Figure 2. A phylogenetic tree of the polypeptides useful in the compositions of the invention is shown in Figure 1.

A polypeptide useful in the compositions of the present invention preferably has a sequence identity to the

mature polypeptide sequence shown in SEQ ID NO: 3 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, wherein the polypeptide has hexosaminidase, preferably beta-1,6 N-acetylglucosaminidase activity. In one aspect, the polypeptide differs by up to 10 amino acids, e.g., 1, 2, 3, 4, 5, 6, 7, 8, 9, or 10, from the polypeptide shown in SEQ ID NO: 3.A polypeptide useful in the compositions of the present invention preferably has a sequence identity to the mature polypeptide sequence shown in SEQ ID NO: 6 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, wherein the polypeptide has hexosaminidase, preferably beta-1,6 N-acetylglucosaminidase activity. In one aspect, the polypeptide differs by up to 10 amino acids, e.g., 1, 2, 3, 4, 5, 6, 7, 8, 9, or 10, from the polypeptide shown in SEQ ID NO: 6.

A polypeptide useful in the compositions of the present invention preferably has a sequence identity to the mature polypeptide sequence shown in SEQ ID NO: 9 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, wherein the polypeptide has hexosaminidase, preferably beta-1,6 N-acetylglucosaminidase activity. In one aspect, the polypeptide differs by up to 10 amino acids, e.g., 1, 2, 3, 4, 5, 6, 7, 8, 9, or 10, from the polypeptide shown in SEQ ID NO: 9.

A polypeptide useful in the compositions of the present invention preferably has a sequence identity to the mature polypeptide sequence shown in SEQ ID NO: 18 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, wherein the polypeptide has hexosaminidase, preferably beta-1,6 N-acetylglucosaminidase activity. In one aspect, the polypeptide differs by up to 10 amino acids, e.g., 1, 2, 3, 4, 5, 6, 7, 8, 9, or 10, from the polypeptide shown in SEQ ID NO: 18.

In an embodiment, the present invention relates to a cleaning composition as defined herein comprising a polypeptide having a sequence identity to the mature polypeptide of SEQ ID NO: 9 of at least 98%, at least 99%, or 100%, wherein the polypeptide has hexosaminidase, preferably beta-1,6 N-acetylglucosaminidase activity. In one aspect, the polypeptides differ by up to 10 amino acids, e.g., 1, 2, 3, 4, 5, 6, 7, 8, 9, or 10, from the mature polypeptide of SEQ ID NO: 9. In an embodiment, the polypeptide has been isolated. A polypeptide useful in the compositions of the present invention preferably comprises or consists of the amino acid sequence of SEQ ID NO: 9 or an allelic variant thereof; or is a fragment thereof having hexosaminidase, preferably beta-1,6-N-acetylglucosaminidase activity. In another aspect, the polypeptide comprises or consists of the mature polypeptide of SEQ ID NO: 8. In another aspect, the polypeptide comprises or consists of amino acids 1 to 331 of SEQ ID NO: 8. In another embodiment, the present invention relates to a cleaning composition as defined herein comprising a polypeptide having beta-1,6 N-acetylglucosaminidase activity encoded by a polynucleotide having a sequence identity to the mature polypeptide coding sequence of SEQ ID NO: 7 of at least 98%, at least 99%, or 100%. In a further embodiment, the polypeptide has been isolated.

In an embodiment, the present invention relates to a cleaning composition as defined herein comprising a polypeptide having a sequence identity to the mature polypeptide of SEQ ID NO: 18 of at least 60%, at least 70%, at least 75%, at least 80%, at least 85%, at least 90%, at least 95%, at least 96%, at least 97% at least 98%, at least 99%, at least 98%, at least 99%, or 100%, wherein the polypeptide has hexosaminidase, preferably beta-1,6 N-acetylglucosaminidase activity. In one aspect, the polypeptides differ by up to 10 amino acids, e.g., 1, 2, 3, 4, 5, 6, 7, 8, 9, or 10, from the mature polypeptide of SEQ ID NO: 18. In an embodiment, the polypeptide has been isolated.

A polypeptide useful in the compositions of the present invention preferably comprises or consists of the amino acid sequence of SEQ ID NO: 18 or an allelic variant thereof; or is a fragment thereof having hexosaminidase, preferably beta-1,6-N-acetylglucosaminidase activity. In another aspect, the polypeptide comprises or consists of the mature polypeptide of SEQ ID NO: 18. In another aspect, the polypeptide comprises or consists of amino acids 1 to 482 of SEQ ID NO: 18. In another embodiment, the present invention relates to a polypeptide having beta-1,6 N-acetylglucosaminidase activity encoded by a polynucleotide having a sequence identity to the mature polypeptide coding sequence of SEQ ID NO: 16 of at least 60%, at least 70%, at least 75%, at least 80%, at least 85%, at least 90%, at least 95%, at least 96%, at least 97% at least 98%, at least 99%, at least 98%, at least 99%, or 100%. In a further embodiment, the polypeptide has been isolated.

The relatedness between two amino acid sequences or between two nucleotide sequences is described by the parameter "sequence identity".

For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

(Identical Residues x 100)/(Length of Alignment – Total Number of Gaps in Alignment).

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant molecules are tested for hexosaminidase activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide

**Compositions :**

The invention relates to compositions, as defined herein, comprising *Lactobacillus* or *Streptococcus* hexosaminidases, preferably dispersins, as well as uses and methods of use thereof.

**Liquid formulations**

In one aspect the cleaning composition is a liquid composition, as defined herein. The hexosaminidase may be formulated as a liquid enzyme formulation, which is generally a pourable composition, though it may also have a high viscosity. The physical appearance and properties of a liquid enzyme formulation may vary a lot - for example, they may have different viscosities (gel to water-like), be colored, not colored, clear, hazy, and even with solid particles like in slurries and suspensions. The minimum ingredients are the enzyme(s) and a solvent system to make it a liquid.

The solvent system may comprise water, polyols (such as glycerol, (mono, di, or tri) propylene glycol, sugar alcohol (e.g. sorbitol), polypropylene glycol, and/or polyethylene glycol), ethanol, sugars, and salts. Usually the solvent system also includes a preservation agent and/or other stabilizers.

A liquid enzyme formulation may be prepared by mixing a solvent system and an enzyme concentrate with a desired degree of purity (or enzyme particles to obtain a slurry/suspension).

In an embodiment, the liquid enzyme composition comprises

(a) at least 0.01% w/w active enzyme protein,
(b) at least 0.5% w/w polyol,
(c) water, and
(d) optionally a preservation agent.

The hexosaminidases e.g. dispersins in the liquid composition of the invention may be stabilized using conventional stabilizing agents, e.g. a polyol such as propylene glycol or glycerol, ethylene glycol, polyethylene glycol, sugar alcohols, sorbitol, mannitol, erythritol, dulcitol, inositol, xylitol and adonitol.

One embodiment of the invention relates to a composition comprising a *Lactobacillus* or *Streptococcus* hexosaminidase, as defined herein, wherein the composition further comprises;

(a)

i. one or more polyol(s), preferably selected from glycerol, (mono, di, or tri) propylene glycol, ethylene glycol, polyethylene glycol, sugar alcohols, sorbitol, mannitol, erythritol, dulcitol, inositol, xylitol and adonitol,
ii. optionally one or more enzyme, preferably selected from proteases, amylases or lipases,
iii. optionally one or more surfactant, preferably selected from anionic and nonionic surfactants, or
iv. optionally one or more polymer.

Another preferred embodiment relates to a composition comprising a *Lactobacillus* or *Streptococcus* hexosaminidase, as defined herein, wherein the composition further comprises;

(a)

i. one or more polyol(s), preferably selected from glycerol, (mono, di, or tri) propylene glycol, ethylene glycol, polyethylene glycol, sugar alcohols, sorbitol, mannitol, erythritol, dulcitol, inositol, xylitol and adonitol,
ii. optionally one or more enzyme, preferably selected from proteases, amylases or lipases,
iii. optionally one or more surfactant, preferably selected from anionic and nonionic surfactants, or
iv. optionally one or more polymer; wherein the hexosaminidase has N-acetylglucosaminidase activity, preferably β-1,6 N-acetylglucosaminidase activity.

One preferred aspect relates to a composition, as defined herein, comprising a dispersin, selected from the group consisting of polypeptides shown in SEQ ID NO 3, SEQ ID NO 6, SEQ ID NO:9, SEQ ID NO 18 or polypeptides having at least 60%, at least 70%, at least 75%, at least 80%, at least 85%, at least 90%, at least 95%, at least 98% or such as at least 99% sequence identity hereto and wherein the composition optionally further comprises;

i. one or more polyol(s), preferably selected from glycerol, (mono, di, or tri) propylene glycol, ethylene glycol, polyethylene glycol, sugar alcohols, sorbitol, mannitol, erythritol, dulcitol, inositol, xylitol and adonitol,
ii. optionally one or more enzyme, preferably selected from proteases, amylases or lipases,
iii. optionally one or more surfactant, preferably selected from anionic and nonionic surfactants, or
iv. optionally one or more polymer.

One preferred aspect relates to a composition, as defined herein, comprising a polypeptide shown in SEQ ID NO 3 or a polypeptide having at least 60%, at least 70%, at least 75%, at least 80%, at least 85%, at least 90%, at least 95%, at least 98% or such as at least 99% sequence identity hereto, wherein the composition further comprises;

i. one or more polyol(s), preferably selected from glycerol, (mono, di, or tri) propylene glycol, ethylene glycol, polyethylene glycol, sugar alcohols, sorbitol, mannitol, erythritol, dulcitol, inositol, xylitol and adonitol,
ii. optionally one or more enzyme, preferably selected from proteases, amylases or lipases,
iii. optionally one or more surfactant, preferably selected from anionic and nonionic surfactants, or
iv. optionally one or more polymer; and

wherein the hexosaminidase has N-acetylglucosaminidase activity, preferably β-1,6 N-acetylglucosaminidase activity.

One preferred aspect relates to a composition, as defined herein, comprising a polypeptide shown in SEQ ID NO 6 or a polypeptide having at least 60%, at least 70%, at least 75%, at least 80%, at least 85%, at least 90%, at least 95%, at least 98% or such as at least 99% sequence identity hereto, wherein the composition further comprises;

i. one or more polyol(s), preferably selected from glycerol, (mono, di, or tri) propylene glycol, ethylene glycol, polyethylene glycol, sugar alcohols, sorbitol, mannitol, erythritol, dulcitol, inositol, xylitol and adonitol,
ii. optionally one or more enzyme, preferably selected from proteases, amylases or lipases,
iii. optionally one or more surfactant, preferably selected from anionic and nonionic surfactants, or
iv. optionally one or more polymer; and

wherein the hexosaminidase has N-acetylglucosaminidase activity, preferably β-1,6 N-acetylglucosaminidase activity.

One preferred aspect relates to a composition, as defined herein, comprising a polypeptide shown in SEQ ID NO 9 or a polypeptide having at least 60%, at least 70%, at least 75%, at least 80%, at least 85%, at least 90%, at least 95%, at least 98% or such as at least 99% sequence identity hereto, wherein the composition further comprises;

i. one or more polyol(s), preferably selected from glycerol, (mono, di, or tri) propylene glycol, ethylene glycol, polyethylene glycol, sugar alcohols, sorbitol, mannitol, erythritol, dulcitol, inositol, xylitol and adonitol,
ii. optionally one or more enzyme, preferably selected from proteases, amylases or lipases,
iii. optionally one or more surfactant, preferably selected from anionic and nonionic surfactants, or
iv. optionally one or more polymer; and

wherein the hexosaminidase has N-acetylglucosaminidase activity, preferably β-1,6 N-acetylglucosaminidase activity.

One preferred aspect relates to a composition, as defined herein, comprising a polypeptide shown in SEQ ID NO 18 or a polypeptide having at least 60%, at least 70%, at least 75%, at least 80%, at least 85%, at least 90%, at least 95%, at least 98% or such as at least 99% sequence identity hereto, wherein the composition further comprises;

i. one or more polyol(s), preferably selected from glycerol, (mono, di, or tri) propylene glycol, ethylene glycol, polyethylene glycol, sugar alcohols, sorbitol, mannitol, erythritol, dulcitol, inositol, xylitol and adonitol,
ii. optionally one or more enzyme, preferably selected from proteases, amylases or lipases,

iii. optionally one or more surfactant, preferably selected from anionic and nonionic surfactants, or
iv. optionally one or more polymer; and

wherein the hexosaminidase has N-acetylglucosaminidase activity, preferably β-1,6 N-acetylglucosaminidase activity.

One preferred aspect relates to a composition, as defined herein, comprising a *Lactobacillus* hexosaminidase selected from the group shown in Table 1, wherein the composition optionally further comprises;

i. one or more polyol(s), preferably selected from glycerol, (mono, di, or tri) propylene glycol, ethylene glycol, polyethylene glycol, sugar alcohols, sorbitol, mannitol, erythritol, dulcitol, inositol, xylitol and adonitol,
ii. optionally one or more enzyme, preferably selected from proteases, amylases or lipases,
iii. optionally one or more surfactant, preferably selected from anionic and nonionic surfactants, or
iv. optionally one or more polymer; and

wherein the hexosaminidase has N-acetylglucosaminidase activity, preferably β-1,6 N-acetylglucosaminidase activity.

## Granular formulations

Non-dusting granulates may be produced, e.g. as disclosed in US 4,106,991 and 4,661,452 and may optionally be coated by methods known in the art. Examples of waxy coating materials are poly(ethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591

The hexosaminidase e.g. *Lactobacillus* or *Streptococcus* hexosaminidase for use in the compositions of the invention may be formulated as a granule for example as a co-granule that combines one or more enzymes or benefit agents such as MnTACN. Each enzyme will then be present in more granules securing a more uniform distribution of enzymes in the detergent. This also reduces the physical segregation of different enzymes due to different particle sizes. Methods for producing multi-enzyme co-granulate for the detergent industry is disclosed in the IP.com disclosure IPCOM000200739D.

Another example of formulation of enzymes by the use of co-granulates are disclosed in WO 2013/188331, which relates to a detergent composition comprising (a) a multi-enzyme co- granule; (b) less than 10 wt zeolite (anhydrous basis); and (c) less than 10 wt phosphate salt (anhydrous basis), wherein said enzyme co-granule comprises from 10 to 98 wt% moisture sink components and the composition additionally comprises from 20 to 80 wt% detergent moisture sink components. WO 2013/188331 also relates to a method of treating and/or cleaning a surface, preferably a fabric surface comprising the steps of (i) contacting said surface with the detergent composition as claimed and described herein in aqueous wash liquor, (ii) rinsing and/or drying the surface.

An embodiment of the invention relates to compositions, as defined herein, comprising an enzyme granule/particle comprising a hexosaminidase as described herein. The granule is composed of a core, and optionally one or more coatings (outer layers) surrounding the core. Typically, the granule/particle size, measured as equivalent spherical diameter (volume based average particle size), of the granule is 20-2000 μm, particularly 50-1500 μm, 100-1500 μm or 250-1200 μm. The core may include additional materials such as fillers, fibre materials (cellulose or synthetic fibres), stabilizing agents, solubilising agents, suspension agents, viscosity regulating agents, light spheres, plasticizers, salts, lubricants and fragrances. The core may include binders, such as synthetic polymer, wax, fat, or carbohydrate. The core may comprise a salt of a multivalent cation, a reducing agent, an antioxidant, a peroxide decomposing catalyst and/or an acidic buffer component, typically as a homogenous blend. The core may consist of an inert particle with the enzyme absorbed into it, or applied onto the surface, e.g., by fluid bed coating. The core may have a diameter of 20-2000 μm, particularly 50-1500 μm, 100-1500 μm or 250-1200 μm. The core can be prepared by granulating a blend of the ingredients, e.g., by a method comprising granulation techniques such as crystallization, precipitation, pan-coating, fluid bed coating, fluid bed agglomeration, rotary atomization, extrusion, prilling, spheronization, size reduction methods, drum granulation, and/or high shear granulation. Methods for preparing the core can be found in Handbook of Powder Technology; Particle size enlargement by C. E. Capes; Volume 1; 1980; Elsevier. The core of the enzyme granule/particle may be surrounded by at least one coating, e.g., to improve the storage stability, to reduce dust formation during handling, or for coloring the granule. The optional coating(s) may include a salt coating, or other suitable coating materials, such as polyethylene glycol (PEG), methyl hydroxy-propyl cellulose (MHPC) and polyvinyl alcohol (PVA). Examples of enzyme granules with multiple coatings are shown in WO 93/07263 and WO 97/23606.

The coating may be applied in an amount of at least 0.1% by weight of the core, e.g., at least 0.5%, 1% or 5%. The amount may be at most 100%, 70%, 50%, 40% or 30%.

The coating is preferably at least 0.1 μm thick, particularly at least 0.5 μm, at least 1 μm or at least 5 μm. In

a one embodiment, the thickness of the coating is below 100 μm. In a more particular embodiment the thickness of the coating is below 60 μm. In an even more particular embodiment the total thickness of the coating is below 40 μm. The coating should encapsulate the core unit by forming a substantially continuous layer. A substantially continuous layer is to be understood as a coating having few or no holes, so that the core unit it is encapsulating/enclosing has few or none uncoated areas. The layer or coating should in preferably be homogeneous in thickness. The coating can further contain other materials as known in the art, e.g., fillers, antisticking agents, pigments, dyes, plasticizers and/or binders, such as titanium dioxide, kaolin, calcium carbonate or talc. A salt coating may comprise at least 60% by weight w/w of a salt, e.g., at least 65%, at least 70%, at least 75%, at least 80%, at least 85%, at least 90%, at least 95% or at least 99% by weight w/w. The salt may be added from a salt solution where the salt is completely dissolved or from a salt suspension wherein the fine particles is less than 50 μm, such as less than 10 μm or less than 5 μm. The salt coating may comprise a single salt or a mixture of two or more salts. The salt may be water soluble, preferably having a solubility at least 0.1 grams in 100 g of water at 20°C, preferably at least 0.5 g per 100 g water, e.g., at least 1 g per 100 g water, e.g., at least 5 g per 100 g water.

The salt may be an inorganic salt, e.g., salts of sulfate, sulfite, phosphate, phosphonate, nitrate, chloride or carbonate or salts of simple organic acids (less than 10 carbon atoms, e.g., 6 or less carbon atoms) such as citrate, malonate or acetate. Examples of cations in these salts are alkali or earth alkali metal ions, the ammonium ion or metal ions of the first transition series, such as sodium, potassium, magnesium, calcium, zinc or aluminium. Examples of anions include chloride, bromide, iodide, sulfate, sulfite, bisulfite, thiosulfate, phosphate, monobasic phosphate, dibasic phosphate, hypophosphite, dihydrogen pyrophosphate, tetraborate, borate, carbonate, bicarbonate, metasilicate, citrate, malate, maleate, malonate, succinate, lactate, formate, acetate, butyrate, propionate, benzoate, tartrate, ascorbate or gluconate. Preferably, alkali- or earth alkali metal salts of sulfate, sulfite, phosphate, phosphonate, nitrate, chloride or carbonate or salts of simple organic acids such as citrate, malonate or acetate may be used.

The salt in the coating may have a constant humidity at 20°C above 60%, particularly above 70%, above 80% or above 85%, or it may be another hydrate form of such a salt (e.g., anhydrate). The salt coating may be as described in WO 00/01793 or WO 2006/034710.

Specific examples of suitable salts are NaCl (CH20°C=76%), Na2CO3 (CH20°C=92%), NaNO3 (CH20°C=73%), Na2HPO4 (CH20°C=95%), Na3PO4 (CH25°C=92%), NH4Cl (CH20°C = 79.5%), (NH4)2HPO4 (CH20°C = 93,0%), NH4H2PO4 (CH20°C = 93.1%), (NH4)2SO4 (CH20°C=81.1%), KCl (CH20°C=85%), K2HPO4 (CH20°C=92%), KH2PO4 (CH20°C=96.5%), KNO3 (CH20°C=93.5%), Na2SO4 (CH20°C=93%), K2SO4 (CH20°C=98%), KHSO4 (CH20°C=86%), MgSO4 (CH20°C=90%), ZnSO4 (CH20°C=90%) and sodium citrate (CH25°C=86%). Other examples include NaH2PO4, (NH4)H2PO4, CuSO4, Mg(NO3)2 and magnesium acetate.

The salt may be in anhydrous form, or it may be a hydrated salt, i.e. a crystalline salt hydrate with bound water(s) of crystallization, such as described in WO 99/32595. Specific examples include anhydrous sodium sulfate (Na2SO4), anhydrous magnesium sulfate (MgSO4), magnesium sulfate heptahydrate (MgSO4.7H2O), zinc sulfate heptahydrate (ZnSO4.7H2O), sodium phosphate dibasic heptahydrate (Na2HPO4.7H2O), magnesium nitrate hexahydrate (Mg(NO3)2(6H2O)), sodium citrate dihydrate and magnesium acetate tetrahydrate. Preferably the salt is applied as a solution of the salt, e.g., using a fluid bed.

In one aspect, the present invention provides a composition, as defined herein, comprising a granule, which comprises:

(a) a core comprising a *Lactobacillus* hexosaminidase, e.g dispersin according to the invention, and

(b) optionally a coating consisting of one or more layer(s) surrounding the core.

One aspect of the invention relates to a composition, as defined herein, comprising a granule, which comprises:

(a) a core comprising a *Lactobacillus* hexosaminidase e.g. dispersin, wherein the *Lactobacillus* hexosaminidase is selected from the group consisting of polypeptides shown in SEQ ID NO 3, SEQ ID NO 6, SEQ ID NO 9 or polypeptides having at least 60%, at least 70%, at least 75%, at least 80%, at least 85%, at least 90%, at least 95%, at least 98% or such as at least 99% sequence identity hereto, and
(b) optionally a coating consisting of one or more layer(s) surrounding the core.

One aspect of the invention relates to a composition, as defined herein, comprising a granule, which comprises:

(a) a core comprising a polypeptide, selected from the group consisting of polypeptides shown in SEQ ID NO 3, SEQ ID NO 6, SEQ ID NO 9, SEQ ID NO 18 or polypeptides having at least 60%, at least 70%, at least 75%, at least 80%, at least 85%, at least 90%, at least 95%, at least 98% or such as at least 99% sequence identity hereto, and
(b) optionally a coating consisting of one or more layer(s) surrounding the core.

One aspect of the invention relates to a composition, as defined herein, comprising a granule, which comprises:

(a) a core comprising a hexosaminidase selected from the group shown in Table 1, and
(b) optionally a coating consisting of one or more layer(s) surrounding the core.

Another aspect relates to a composition, as defined herein, comprising a layered granule comprising

(a) a (non-enzymatic) core;
(b) a coating surrounding the core, wherein the coating comprises *Lactobacillus* hexosaminidase e.g. dispersin; and
(c) optionally a protective salt coating surrounding the enzyme containing coating.

Another aspect relates to a composition, as defined herein, comprising a layered granule comprising

(a) a (non-enzymatic) core;
(b) a coating surrounding the core, wherein the coating comprises *Lactobacillus* hexosaminidase e.g. dispersin, wherein the *Lactobacillus* hexosaminidase is selected from the group consisting of polypeptides shown in SEQ ID NO 3, SEQ ID NO 6, SEQ ID NO:9 or polypeptides having at least 60%, at least 70%, at least 75%, at least 80%, at least 85%, at least 90%, at least 95%, at least 98% or such as at least 99% sequence identity hereto; and
(c) optionally a protective salt coating surrounding the enzyme containing coating.

Another aspect relates to a composition, as defined herein, comprising a layered granule comprising

(a) a (non-enzymatic) core;
(b) a coating surrounding the core, wherein the coating comprises a polypeptide, selected from the group consisting of polypeptides shown in SEQ ID NO 3, SEQ ID NO 6, SEQ ID NO 9, SEQ ID NO 18 or polypeptides having at least 60%, at least 70%, at least 75%, at least 80%, at least 85%, at least 90%, at least 95%, at least 98% or such as at least 99% sequence identity hereto; and
(c) optionally a protective salt coating surrounding the enzyme containing coating.

Another aspect relates to a composition, as defined herein, comprising a layered granule comprising

(a) a (non-enzymatic) core;
(b) a coating surrounding the core, wherein the coating comprises a hexosaminidase selected from the group shown in Table 1; and
(c) optionally a protective salt coating surrounding the enzyme containing coating.

**Cleaning compositions**

The compositions of the invention are cleaning compositions comprising a *Lactobacillus hexosaminidase* in combination with one or more additional cleaning composition components, as defined herein. The choice of additional components is within the skill of the artisan and includes conventional ingredients, including the exemplary non-limiting components set forth below.

One aspect of the invention relates to a composition, as defined herein, comprising;

a) at least 0.01 mg/mL of at least one *Lactobacillus* hexosaminidase; and
b) optionally at least one additional cleaning composition component, preferably selected from surfactants, builders, bleach components, polymers, dispersing agents and additional enzymes.

One aspect of the invention relates to a composition, as defined herein, comprising;

a) at least 0.01 mg/mL of at least one *Lactobacillus* hexosaminidase, wherein the *Lactobacillus hexosaminidase* is selected from the group shown in Table 1; and
b) optionally at least one additional cleaning composition component, preferably selected from surfactants, builders, bleach components, polymers, dispersing agents and additional enzymes.

One aspect of the invention relates to a composition, as defined herein, comprising;

a) at least 0.01 mg/mL of at least one *Lactobacillus* hexosaminidase, wherein the *Lactobacillus hexosaminidase* is

selected from polypeptides having at least 60%, at least 70%, at least 75%, at least 80%, at least 85%, at least 90%, at least 95%, at least 96%, at least 97% at least 98%, at least 99%, at least 98%, at least 99%, or 100%sequence identity to the polypeptides shown in SEQ ID NO 3, 6 and 9; and
b) optionally at least one additional cleaning composition component, preferably selected from surfactants, builders, bleach components, polymers, dispersing agents and additional enzymes.

One aspect of the invention relates to a composition, as defined herein, comprising;

a) at least 0.01 mg/mL of at least one hexosaminidase, wherein the hexosaminidase has at least 60%, at least 70%, at least 75%, at least 80%, at least 85%, at least 90%, at least 95%, at least 96%, at least 97% at least 98%, at least 99%, at least 98%, at least 99%, or 100% sequence identity to the polypeptides shown in SEQ ID NO 18; and
b) optionally at least one additional cleaning composition component, preferably selected from surfactants, builders, bleach components, polymers, dispersing agents and additional enzymes.

The *Lactobacillus hexosaminidase* may be included in the cleaning e.g. detergent composition of the present invention at a level of at least 0.0001 to at least 100, at least 0.001 to at least 100, at least 0.01 to at least 100, at least 0.02 to at least 100, at least 0.01 to at least 100, at least 0.1 to at least 100, at least 0.2 to at least 100, at least 0.5 to at least 100 mg/mL, preferably, the concentration of hexosaminidase enzyme in the cleaning composition e.g. detergent is in the range 0.01 to 100 mg/mL, 0.1 to 50 mg/mL or 0.01 to 10 mg/mL. Thus, the detergent composition may comprise at least 0.00008 wt%, preferably at least 0.002 wt%, 0.003 wt%, 0.004 wt%, 0.005 wt%, 0.006 wt%, 0.008 wt%, 0.01 wt%, 0.02 wt%, 0.03 wt%, 0.05 wt%, 0.1 wt%, 0.2 wt%, 0.3 wt%, 0.4 wt%, 0.6 wt%, 0.7 wt%, 0.8 wt%, 0.9 wt% or 1.0 wt% hexosaminidase, where wt% is the weight percent, which is the mass fraction multiplied by 100. Mass fraction is the ratio of one substance with mass $m_{sub}$ to the mass of the total mixture $m_t$,($m_{frac} = m_{sub}/m_{tot}$).

The choice of cleaning components may include, for textile care, the consideration of the type of textile to be cleaned, the type and/or degree of soiling, the temperature at which cleaning is to take place, and the formulation of the detergent product.

The cleaning compositions of the invention may comprise one or more surfactants, which may be anionic and/or cationic and/or non-ionic and/or semi-polar and/or zwitterionic, or a mixture thereof. In a particular embodiment, the detergent composition includes a mixture of one or more nonionic surfactants and one or more anionic surfactants. The surfactant(s) is typically present at a level of from about 0.1% to about 60% by weight, such as about 1% to about 40%, or about 3% to about 20%, or about 3% to about 10%. The surfactant(s) is chosen based on the desired cleaning application, and may include any conventional surfactant(s) known in the art.

The compositions as defined herein, if not indicated otherwise, may comprise from 0-65 wt %, from about 2 wt % to about 60 wt %, from about 5 wt % to about 50 wt %, from about 5 wt % to about 40 wt %, from about 5 wt % to about 30 wt %, from about 5 wt % to about 20 wt %, from about 5 wt % to about 10 wt % anionic surfactants, amphoteric and/or non-ionic surfactants. "About", as used herein in relation to a numerical value means said value $\pm$10%, preferably $\pm$5%. "About 5 wt %" thus means from 4.5 to 5.5 wt %, preferably from 4.75 to 5.25 wt %.

If not indicated otherwise, the surfactant may be generally selected among nonionic, anionic and/or amphoteric surfactants. In general, bleach-stable surfactants are preferred. Preferred anionic surfactants are sulphate surfactants and in particular alkyl ether sulphates, especially C9-C15 alcohol ether sulfates, preferably ethoxylates or mixed ethoxylates/propoxylates, such as those with 1 to 30 EO, C12-C15 primary alcohol ethoxylate, , such as those with 1 to 30 EO, C8-C16 ester sulphates and C10-C14 ester sulphates, such as mono dodecyl ester sulphates. Non-limiting examples of anionic surfactants include sulfates and sulfonates, in particular, linear alkylbenzenesulfonates (LAS), in particular C12-C13 alkyl benzene sulfonates, isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ether sulfates (AES or AEOS or FES, also known as alcohol ethoxysulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or salt of fatty acids (soap), and combinations thereof. The anionic surfactants are preferably added to the detergent in the form of salts. Suitable cations in these salts are alkali metal ions, such as sodium, potassium and lithium and ammonium salts, for example (2-hydroxyethyl) ammonium, bis(2-hydroxyethyl) ammonium and tris(2-hydroxyethyl) ammonium salts. Non-limiting examples of nonionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxyalkyl

fatty acid amides, or N-acyl N-alkyl derivatives of glucosamine (glucamides, GA, or fatty acid glucamides, FAGA), as well as products available under the trade names SPAN and TWEEN, and combinations thereof. Commercially available nonionic surfactants include Plurafac ™, Lutensol™ and Pluronic™ range from BASF, Dehypon™ series from Cognis and Genapol™ series from Clariant.

In various embodiments, said surfactant preferably comprises at least one alkyl ether sulfate. Preferred alkyl ether sulfates are those of formula (I)

$$R^1\text{-O-(AO)n-}SO_3^-X^+ \qquad \text{(I).}$$

In formula (I) $R^1$ represents a linear or branched, substituted or unsubstituted alkyl group, preferably a linear, unsubstituted alkyl group, more preferably a fatty alcohol moiety. Preferred $R^1$ moieties are selected from the group consisting of decyl, undecyl, dodecyl, tridecyl, tetradecyl, pentadecyl, hexadecyl, heptadecyl, octadecyl, nonadecyl, eicosyl moieties and mixtures thereof, wherein those groups with an even number of carbon atoms are preferred. Particularly preferred $R^1$ moieties are derived from $C_{10}$-$C_{18}$ fatty alcohols, such as those derived from coconut oil alcohols, tallow fatty alcohols, lauryl, myristyl, cetyl or stearyl alcohol or from $C_{10}$-$C_{20}$ oxoalcohols.

AO represents an ethyleneoxide (EO) or propyleneoxide (PO) group, preferably an ethyleneoxide group. The index n represents an integer from 1 to 50, preferably from 1 to 20 and more preferably from 1 to 10. Particularly preferably, n is 1, 2, 3, 4, 5, 6, 7 or 8. X represents a monovalent cation or the n-th part of an n-valent cation, preferred are alkali metal cations, specifically $Na^+$ and $K^+$, most preferably $Na^+$. Further cations $X^+$ may be selected from $NH_4^+$, ½ $Zn^{2+}$,½ $Mg^{2+}$,½ $Ca^{2+}$,½ $Mn^{2+}$, and combinations thereof.

In various preferred embodiments, the detergent compositions comprise an alkyl ether sulfate selected from fatty alcohol ether sulfates of formula (II)

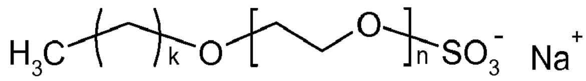

(II)

wherein k = 9 to 19, and n = 1, 2, 3, 4, 5, 6, 7 or 8. Preferred are $C_{10-16}$ fatty alcohol ether sulfates with 1-7 EO (k = 9-15, n = 1-7), such as the $C_{12-14}$ fatty alcohol ether sulfates with 1-3, particularly 2 EO (k = 11-13, n = 1-3 or 2), more particularly the sodium salts thereof. One specific embodiment thereof is lauryl ether sulfate sodium salt with 2 EO. The level of ethoxylation is an average value and can, for a specific compound, be an integer or fractional number.

In various embodiments, the surfactant comprises at least one alkyl benzene sulfonate. Said alkyl benzene sulfonate may be present alternatively to the above alkyl ether sulfate or, preferably, in addition to it.

Exemplary alkyl benzene sulfonates include, but are not limited to linear and branched alkyl benzene sulfonates, preferably linear alkyl benzene sulfonates. Exemplary compounds are those of formula (III)

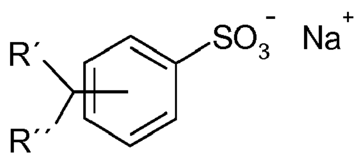

(III),

wherein R' and R" are independently H or alkyl and combined comprise 9 to 19, preferably 9 to 15 and more preferably 9 to 13 carbon atoms. Particularly preferred are dodecyl and tridecyl benzene sulfonates, in particular the sodium salts thereof.

In addition or alternatively, the compositions of the invention may further comprise one or more nonionic surfactants. Preferred nonionic surfactants are those of formula (IV)

$$R^2\text{-O-(AO)}_m\text{-H} \qquad \text{(IV),}$$

wherein $R^2$ represents a linear or branched substituted or unsubstituted alkyl moiety, AO represents an ethylene oxide (EO) or propylene oxide (PO) group and m is an integer from 1 to 50.

In formula (IV) $R^2$ preferably represents a linear or branched, substituted or unsubstited alkyl group, preferably a linear, unsubstituted alkyl group, particularly preferred a fatty alcohol group. Preferred groups are $R^2$ are selected from decyl, undecyl, dodecyl, tridecyl, tetradecyl, pentadecyl, hexadecyl, heptadecyl, octadecyl, nonadecyl, eicosyl groups and combinations thereof, wherein those groups with an even number of carbon atoms are preferred. Particularly preferred

are $R^2$ groups derived from $C_{12}$-$C_{18}$ fatty alcohols, such as coconut oil alcohol, tallow oil alcohol, lauryl, myristyl, cetyl or stearyl alcohol or from $C_{10}$-$C_{20}$ oxoalcohols.

AO represents an ethyleneoxide (EO) or propyleneoxide (PO) group, preferably an ethyleneoxide group. The index m represents an integer from 1 to 50, preferably from 1 to 20 and more preferably from 1 to 6. Particularly preferably, m is 1, 2, 3, 4 or 5, most preferably 3-5, as higher degrees of ethoxylation may negatively influence viscosity and stability.

In various preferred embodiments, the detergent compositions comprise an alkyl ether selected from fatty alcohol ethers of formula (V)

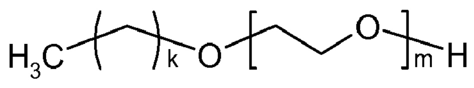

(V)

wherein k = 11 to 19, m = 1, 2, 3, 4, 5, 6, 7 or 8. Preferred are $C_{12-18}$ fatty alcohols with 1-6 EO (k = 11-17, m = 1-5 in formula (V)). More preferred are $C_{12-14}$ alcohols having 1-5 EO, most preferred are $C_{12-14}$ alkyl ethers with 3-5 EO, in particular lauryl ether with 5 EO.

The detergent compositions may further include other nonionic surfactants, such as alkyl glucosides of the general formula $RO(G)_x$, where R is a primary linear or 2-methyl-branched aliphatic radical containing 8 to 22 and preferably 12 to 18 carbon atoms and G stands for a glucose unit. The degree of oligomerization x, which indicates the distribution of monoglucosides and oligoglucosides, is a number of 1 to 10 and preferably a number of 1.2 to 1.4.

In various embodiments, the composition comprises at least two anionic surfactants, e.g. at least one alkyl ether sulfate and preferably at least one alkyl benzene sulfonate, and optionally an alkyl ether.

Suitable amphoteric surfactants comprise betains. Preferred betaines are the alkylbetaines, the alkylamidobetaines, the imidazolinium betaines, the sulfobetaines (INCI Sultaines) and the phosphobetaines. Examples of suitable betaines and sulfobetaines are the following compounds designated as INCI: almondamidopropyl betaines, apricotam idopropyl betaines, avocadamidopropyl betaines, babassuamidopropyl betaines, behenamide idopropyl betaines, behenyl betaines, betaines, canola idopropyl betaines, caprylic / capram idopropyl betaines, carnitines, cetyl betaines, Cocamidoethyl betaines, cocamidopropyl betaines, cocam idopropyl hydroxysultaines, cocobetaines, coco-hydroxysultaines, coco / oleam idopropyl betaines, coco-sultaines, decyl betaines, dihydroxyethyl oleyl glycinates, dihydroxyethyl soy glycinates, dihydroxyethyl stearyl glycinates, dihydroxyethyl tallow glycinates, dimethicones propyl PG Betaines, erucam idopropyl hydroxysultaines, hydrogenated tallow betaines, isostearam idopropyl betaines, lauram idopropyl betaines, lauryl betaines, lauryl hydroxysultaine, lauryl sultaines, milkamidopropyl betaines, minkam idopropyl betaines, myristamine idopropyl betaines, myristyl betaines, oleam idopropyl betaines, oleam idropy Hydroxysultain, Oleyl Betaine, Olivamidopropyl Betaine, Palmam Idopropyl Betaine, Palm Itam Idopropyl Betaine, Palmitoyl Carnitine, Palm Kernelamidopropyl Betaine, Polytetrafluoroethylene Acetoxypropyl Betaine, Ricinoleam Idopropyl Betaine, Sesamidopropyl Betaine, Soyamidopropyl Betaine, Stearam Idopropyl Betaine, Stearyl Betaine, Tallowam Idopropyl Betaine, Tallowamidopropyl Hydroxysultaine, Tallow Betaine, Tallow Dihydroxyethyl Betaine, Undecylenamidopropyl Betaine and Wheat Germamidopropyl Betaine. A preferred betaine is, for example, cocamidopropyl betaine (cocoamidopropylbetaine). The betaines are particularly preferred for dishwashing compositions, most preferably hand dishwashing detergent compositions.

Further suitable surfactants include the amine oxides. The amine oxides suitable in accordance with the invention include alkylamine oxides, in particular alkyldimethylamine oxides, alkylamidoamine oxides and alkoxyalkylamine oxides. Examples of suitable amine oxides are the following compounds designated as INCI: Almond amidopropylamine oxides, Babassu amidopropylamine oxides, Behenamine oxides, Cocamidopropyl Amine oxides, Cocamidopropylamine oxides, Cocamine oxides, Coco-Morpholine oxides, Decylamine oxides, Decyltetradecylamine oxides, Diaminopyrimidine oxides, Dihydroxyethyl C8-10 alkoxypropylamines oxides , Dihydroxyethyl C9-11 alkoxypropylamines oxides, dihydroxyethyl C12-15 alkoxypropylamines oxides, dihydroxyethyl cocamine oxides, dihydroxyethyl lauramine oxides, dihydroxyethyl stearamines oxides, dihydroxyethyl tallowamine oxides, hydrogenated palm kernel amine oxides, hydrogenated tallowamine oxides, hydroxyethyl hydroxypropyl C12-15 alkoxypropylamines oxides, isostearamidopropylamines Oxides, isostearamidopropyl morpholine oxides, lauram idopropylamine oxides, lauramine oxides, methyl morpholine oxides, milkamidopropyl amine oxides, mincamidopropylamine oxides, myristamine idopropylamine oxides, myristamine oxides, myristyl / cetyl amines Oxides, Oleam idopropylamine oxides, Oleamine oxides, Ol ivam idopropylam ine oxides, Palmitamidopropylamine oxides, Palmitamine oxides, PEG-3 Lauramine oxides, Potassium dihydroxyethyl Cocamine oxides phosphates, Potassium Trisphosphonomethylamine oxides, Sesamidopropylamine oxides, Soyamidopropylamine oxides, Stearam idopropylam ine oxides, stearamines Oxides, Tallowam idopropylam ine oxides, Tallowamine oxides, Undecylenamidopropylamine oxides and Wheat Germam idopropylam ine oxides. A preferred amine oxide is, for example, cocamidopropylamine oxides (cocoamidopropylamine oxide).

For automatic dishwashing applications, low-foaming nonionic surfactants are preferably used, in particular

alkoxylated, especially ethoxylated, low-foaming nonionic surfactants. With particular preference, the automatic dishwashing detergents contain nonionic surfactants from the group of the alkoxylated alcohols. Particular preference is given to nonionic surfactants which have a melting point above room temperature. Nonionic surfactants having a melting point above 20 °C, preferably above 25 °C, more preferably between 25 and 60 °C and especially between 26.6 and 43.3 °C, are particularly preferred. Preferably used surfactants are those from the groups of alkoxylated nonionic surfactants, in particular the ethoxylated primary alcohols and mixtures of these surfactants with structurally more complex surfactants such as polyoxypropylene / polyoxyethylene / polyoxypropylene ((PO / EO / PO) surfactants). Such (PO / EO / PO) nonionic surfactants are also characterized by good foam control. Particularly preferred nonionic surfactants are those containing alternating ethylene oxide and different alkylene oxide units. Among these, in turn, surfactants with EO-AO-EO-AO blocks are preferred, with one to ten EO or AO groups before one block from the other group follows. Exemplary nonionic surfactants are those having a C9-alkyl group with 1 to 4 ethylene oxide units followed by 1 to 4 propylene oxide units, followed by 1 to 4 ethylene oxide units followed by 1 to 4 propylene oxide units. Preference is given in particular to end-capped, poly (oxyalkylated) nonionic surfactants with the end-cap being a linear or branched, saturated or unsaturated, aliphatic or aromatic hydrocarbon radical R having 1 to 30 carbon atoms. The alkyl groups may also comprise hydroxyl groups. The group of these nonionic surfactants include, for example, the C4-22 fatty alcohol $(EO)_{10-50}$-2- hydroxyalkyl ethers, in particular also the C8-12 fatty alcohol $(EO)_{22}$-2-hydroxydecyl ethers and the C4-22 fatty alcohol $(EO)_{40-80}$-2-hydroxyalkyl ethers.

When included therein the detergent will usually contain from about 1% to about 40% by weight of an anionic surfactant, such as from about 5% to about 30%, including from about 5% to about 15%, or from about 15% to about 20%, or from about 20% to about 25% of an anionic surfactant. Non-limiting examples of anionic surfactants include sulfates and sulfonates, in particular, linear alkylbenzenesulfonates (LAS), isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ethersulfates (AES or AEOS or FES, also known as alcohol ethoxysulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or salt of fatty acids (soap), and combinations thereof.

When included therein the detergent will usually contain from about 1% to about 40% by weigh of a cationic surfactant, for example from about 0.5% to about 30%, in particular from about 1% to about 20%, from about 3% to about 10%, such as from about 3% to about 5%, from about 8% to about 12% or from about 10% to about 12%. Non-limiting examples of cationic surfactants include alkyldimethylethanolamine quat (ADMEAQ), cetyltrimethylammonium bromide (CTAB), dimethyldistearylammonium chloride (DSDMAC), and alkylbenzyldimethylammonium, alkyl quaternary ammonium compounds, alkoxylated quaternary ammonium (AQA) compounds, ester quats, and combinations thereof.

When included therein the detergent will usually contain from about 0.2% to about 40% by weight of a nonionic surfactant, for example from about 0.5% to about 30%, in particular from about 1% to about 20%, from about 3% to about 10%, such as from about 3% to about 5%, from about 8% to about 12%, or from about 10% to about 12%. Non-limiting examples of nonionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxyalkyl fatty acid amides, or N-acyl N-alkyl derivatives of glucosamine (glucamides, GA, or fatty acid glucamides, FAGA), as well as products available under the trade names SPAN and TWEEN, and combinations thereof.

When included therein the detergent will usually contain from about 0.01 to about 10 % by weight of a semipolar surfactant. Non-limiting examples of semipolar surfactants include amine oxides (AO) such as alkyldimethylamineoxide, N-(coco alkyl)-N,N-dimethylamine oxide and N-(tallow-alkyl)-N,N-bis(2-hydroxyethyl)amine oxide, and combinations thereof.

When included therein the detergent will usually contain from about 0.01 % to about 10 % by weight of a zwitterionic surfactant. Non-limiting examples of zwitterionic surfactants include betaines such as alkyldimethylbetaines, sulfobetaines, and combinations thereof.

The detergent compositions of the invention may contain about 0-65% by weight, such as about 5% to about 50% of a detergent builder or co-builder, or a mixture thereof. In a dish wash detergent, the level of builder is typically in the range 40-65%, particularly in the range 50-65%. The builder and/or co-builder may particularly be a chelating agent that forms water-soluble complexes with Ca and Mg. Any builder and/or co-builder known in the art for use in cleaning detergents may be utilized. Non-limiting examples of builders include zeolites, diphosphates (pyrophosphates), triphosphates such as sodium triphosphate (STP or STPP), carbonates such as sodium carbonate, soluble silicates

such as sodium metasilicate, layered silicates (e.g., SKS-6 from Hoechst), ethanolamines such as 2-aminoethan-1-ol (MEA), diethanolamine (DEA, also known as 2,2'-iminodiethan-1-ol), triethanolamine (TEA, also known as 2,2',2"-nitrilotriethan-1-ol), and (carboxymethyl)inulin (CMI), and combinations thereof. "Co-builder", as used herein, means that the respective component is used in combination with another builder. All compounds disclosed as co-builders herein may also be used as main builders and vice versa, unless indicated otherwise.

In various embodiments, the composition, as defined herein, may comprise, if not indicated otherwise, from 0-65 wt %, for example about 1 wt% to about 65 wt%, from about 5 wt% to about 50 wt%, preferably from about 40 wt% to 65 wt%, such as 50 - 65 wt %, particularly about 20 wt% to about 65 wt%, particularly from 10 wt% to 50 wt% of at least one builder.

Generally and if not indicated otherwise, the builder may be preferably selected from citrate, carbonate, silicate, aluminosilicate (zeolite) and combinations thereof. Suitable builders also include phosphonates, polyphosphonates, bicarbonates, borates, and further polycarboxylates. Citrate builders, e.g., citric acid and soluble salts thereof (particularly sodium salt), are particularly suitable water-soluble organic builders. Citrates can be used in combination with zeolite, silicates like the BRITESIL types, and/or layered silicate builders. The builder and/or co-builder may be any chelating agent that forms water-soluble complexes with Ca and Mg. Any builder and/or co-builder known in the art for use in cleaning detergents may be utilized. Non-limiting examples of builders include zeolites, in particular zeolite A or P or X, carbonates such as sodium carbonate, soluble silicates such as sodium metasilicate, layered silicates (e.g., SKS-6 from Hoechst), and (carboxymethyl)inulin (CMI), and combinations thereof. Further non-limiting examples of builders include aminocarboxylates, aminopolycarboxylates, and alkyl- or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-N,N'-disuccinic acid (EDDS), methylglycine-N,N- diacetic acid (MGDA), glutamic acid-N,N-diacetic acid (GLDA), 1-hydroxyethane-1,1-diphosphonic acid, N-(2-hydroxyethyl)iminodiacetic acid (EDG), aspartic acid-N-monoacetic acid (ASMA), aspartic acid-N,N-diacetic acid (ASDA), aspartic acid-N-monopropionic acid (ASMP), iminodisuccinic acid (IDA), N-(sulfomethyl)aspartic acid (SMAS), N-(2-sulfoethyl)-aspartic acid (SEAS), N-(sulfomethylglutamic acid (SMGL), N-(2-sulfoethyl)-glutamic acid (SEGL), N-methyliminodiacetic acid (MIDA), serine-N,N-diacetic acid (SEDA), isoserine-N,N-diacetic acid (ISDA), phenylalanine-N,N-diacetic acid (PHDA), anthranilic acid-N,N-diacetic acid (ANDA), sulfanilic acid-N,N-diacetic acid (SLDA), taurine-N,N-diacetic acid (TUDA) and N'-(2-hydroxyethyl)ethylenediamine-N,N,N'-triacetic acid (HEDTA), diethanolglycine (DEG), and combinations and salts thereof. Phosphonates suitable for use herein include 1-hydroxyethane-1,1-diphosphonic acid (HEDP), ethylenediaminetetrakis (methylenephosphonic acid) (EDTMPA), diethylenetriaminepentakis (methylenephosphonic acid) (DTMPA or DTPMPA or DTPMP), nitrilotris (methylenephosphonic acid) (ATMP or NTMP), 2-phosphonobutane-1,2,4-tricarboxylic acid (PBTC), hexamethylenediaminetetrakis (methylenephosphonic acid) (HDTMP). Particularly preferred are HEDP and DTPMP.

Suitable silicates are crystalline, layered sodium silicates of the general formula $NaMSi_xO_2+1*yH_2O$, wherein M is sodium or H, x a number of from 1.9 to 4 and ya number of from 0 to 20 and x is preferably 2, 3 or 4. Such silicates are for example disclosed in EP-A-0 164 514. Preferred are silicates in which M is sodium and is 2 or 3. Particularly preferred are β- and δ-sodium disilicate $Na_2Si_2O_5*yH_2O$.

Although not preferred, the compositions may also comprise phosphates, diphosphates (pyrophosphates) and/or triphosphates such as sodium triphosphate (STP or STPP). It is however preferred that all compositions disclosed herein are phosphate-free, i.e. do not contain deliberately added phosphate, in particular the phosphate content is below 1 wt %, more preferably less than 0.5 wt %, even more preferably less than 0.1 wt %, relative to the total weight of the composition. In alternative embodiments, the invention also relates to phosphate-free cleaning compositions in general that contain the polypeptides of the invention. In one aspect, the invention thus features a phosphate-free cleaning composition comprising any one or more of the polypeptides having hexosaminidase activity disclosed herein.

If not indicated otherwise, the composition may also contain 0-50% by weight, such as about 5% to about 30%, of a detergent co-builder. The composition may include a co-builder alone, or in combination with a builder, for example a zeolite builder. Non-limiting examples of co-builders include homopolymers of polyacrylates or copolymers thereof, such as poly (acrylic acid) (PAA) or copoly (acrylic acid/maleic acid) (PAA/PMA) or polyaspartic acid. Further exemplary builders and/or co-builders are described in, e.g., WO 09/102854, US 5977053.

Preferred as co-builders are acrylate-containing water-soluble polymers, such as alkali metal salts of polyacrylic acid or polymethacrylic acid, for example those having a molecular weight $M_w$ in the range of 600 to 750,000 g / mol, as determined by gel permeation chromatography (GPC) according to DIN 55672-1:2007-08 with THF as an eluent.

Preferred polymers are polyacrylates with a molecular weight $M_w$ of 1,000 to 15,000 g / mol, more preferred, due to their solubility, are short-chain polyacrylates with a molecular weight $M_w$ of 1,000 to 10,000 g / mol, most preferred from 1,000 to 5,000 g / mol.

Preferred acrylates for use in the present invention are alkali metal salts of polymers of acrylic acid, preferably the sodium salts, in particular those with molecular weights in the range of 1,000 to 10,000 g / mol or 1,000 to 5,000 g / mol. Suitable acrylates are commercially available, for example under the tradename Acusol® from Dow Chemical.

Suitable are also copolymers of acrylates, in particular those of acrylic acid and methacrylic acid, and acrylic acid or methacrylic acid and maleic acid.

In preferred embodiments, the compositions of the invention comprise a sulfopolymer, preferably a copolymer comprising an ethylenically unsaturated sulfonate/sulfonic acid as a co-monomer. Particularly suitable are monomers of allyl sulfonic acids, such as allyloxybenzene sulfonic acid and methallyl sulfonic acid. Particularly preferred sulfonic acid group-containing monomers are 1-acrylamido propane sulfonic acid-1, 2-acrylamido-2-propanesulfonic acid, 2-acrylamido-2-methyl-1-propanesulfonic acid, 2-methacrylamido-2-methyl-1-propanesulfonic acid, 3- methacrylamido-2-hydroxy-propanesulfonic acid, allylsulfonic acid, methallylsulfonic acid, allyloxybenzenesulfonic acid, methallyloxybenzolsulfonsaure, 2-hydroxy-3- (2-propenyloxy) propanesulfonic acid, 2-methyl-2-propenl-sulfonic acid, styrenesulfonic acid, vinylsulfonic acid, 3-sulfopropyl, 3-sulfo - propyl, sulfomethacrylamide, sulfomethylmethacrylamide and mixtures of said acids or their water-soluble salts. The sulfopolymers are preferably copolymers of the afore-described monomers with unsaturated carboxylic acids, Especially preferred unsaturated carboxylic acids are acrylic acid, methacrylic acid, ethacrylic acid, chloroacrylic acid, alpha-cyanoacrylic acid, crotonic acid, alpha-phenyl-acrylic acid, maleic acid, maleic anhydride, fumaric acid, itaconic acid, citraconic acid, methylenemalonic acid, sorbic acid, cinnamic acid or mixtures thereof. Usable are of course also the unsaturated dicarboxylic acids. Preferred are copolymers with acrylates, in particular with acrylic acid and methacrylic acid, and acrylic acid or methacrylic acid and maleic acid.

Such polymers are, for example, commercially available under the trade names Acusol ®590 or Acusol® 588 from Dow Chemical.

In one aspect of the invention, the cleaning compositions of the invention comprise a polypeptide as defined herein and at least one sulfopolymer, as defined above. Such compositions are preferably dishwashing compositions.

In one preferred embodiment, the builder is a non-phosphorus based builder such as citric acid and/or methylglycine-N,N-diacetic acid (MGDA) and/or glutamic-N,N-diacetic acid (GLDA) and / or salts thereof.

Further non-limiting examples of co-builders include citrate, chelators such as aminocarboxylates, aminopolycarboxylates and phosphonates, and alkyl- or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-N,N'-disuccinic acid (EDDS), methylglycinediacetic acid (MGDA), glutamic acid-N,N-diacetic acid (GLDA), 1-hydroxyethane-1,1-diphosphonic acid (HEDP), ethylenediaminetetra(methylenephosphonic acid) (EDTMPA), diethylenetriaminepentakis(methylenephosphonic acid) (DTMPA or DTPMPA), N-(2-hydroxyethyl)iminodiacetic acid (EDG), aspartic acid-N-monoacetic acid (ASMA), aspartic acid-N,N-diacetic acid (ASDA), aspartic acid-N-monopropionic acid (ASMP), iminodisuccinic acid (IDA), N-(2-sulfomethyl)-aspartic acid (SMAS), N-(2-sulfoethyl)-aspartic acid (SEAS), N-(2-sulfomethyl)-glutamic acid (SMGL), N-(2-sulfoethyl)-glutamic acid (SEGL), N-methyliminodiacetic acid (MIDA), α-alanine-N,N-diacetic acid (α-ALDA), serine-N,N-diacetic acid (SEDA), isoserine-N,N-diacetic acid (ISDA), phenylalanine-N,N-diacetic acid (PHDA), anthranilic acid-N,N-diacetic acid (ANDA), sulfanilic acid-N,N-diacetic acid (SLDA), taurine-N,N-diacetic acid (TUDA) and sulfomethyl-N,N-diacetic acid (SMDA), N-(2-hydroxyethyl)ethylenediamine-N,N',N"-triacetic acid (HEDTA), diethanolglycine (DEG), diethylenetriamine penta(methylenephosphonic acid) (DTPMP), aminotris(methylenephosphonic acid) (ATMP), and combinations and salts thereof. Further exemplary builders and/or co-builders are described in, e.g., WO 09/102854, US 5977053

The cleaning composition may contain 0-50% by weight, such as 1-40%, such as 1-30%, such as about 1% to about 20%, of a bleaching system. Any bleaching system comprising components known in the art for use in cleaning detergents may be utilized. Suitable bleaching system components include sources of hydrogen peroxide; sources of peracids; and bleach catalysts or boosters.

Suitable sources of hydrogen peroxide are inorganic persalts, including alkali metal salts such as sodium percarbonate and sodium perborates (usually mono- or tetrahydrate), and hydrogen peroxide-urea (1/1).

Peracids may be (a) incorporated directly as preformed peracids or (b) formed in situ in the wash liquor from hydrogen peroxide and a bleach activator (perhydrolysis) or (c) formed in situ in the wash liquor from hydrogen peroxide and a perhydrolase and a suitable substrate for the latter, e.g., an ester.

a) Suitable preformed peracids include, but are not limited to, peroxycarboxylic acids such as peroxybenzoic acid and its ring-substituted derivatives, peroxy-α-naphthoic acid, peroxyphthalic acid, peroxylauric acid, peroxystearic acid, ε-phthalimidoperoxycaproic acid [phthalimidoperoxyhexanoic acid (PAP)], and o-carboxybenzamidoperoxycaproic acid; aliphatic and aromatic diperoxydicarboxylic acids such as diperoxydodecanedioic acid, diperoxyazelaic acid, diperoxysebacic acid, diperoxybrassylic acid, 2-decyldiperoxybutanedioic acid, and diperoxyphthalic, -isophthalic and - terephthalic acids; perimidic acids; peroxymonosulfuric acid; peroxydisulfuric acid; peroxyphosphoric acid; peroxysilicic acid; and mixtures of said compounds. It is understood that the peracids mentioned may in some cases be best added as suitable salts, such as alkali metal salts (e.g., Oxone®) or alkaline earth-metal salts.

b) Suitable bleach activators include those belonging to the class of esters, amides, imides, nitriles or anhydrides and, where applicable, salts thereof. Suitable examples are tetraacetylethylenediamine (TAED), sodium 4-[(3,5,5-trimethylhexanoyl)oxy]benzene-1-sulfonate (ISONOBS), sodium 4-(dodecanoyloxy)benzene-1-sulfonate (LOBS), sodium 4-(decanoyloxy)benzene-1-sulfonate, 4-(decanoyloxy)benzoic acid (DOBA), sodium 4-(nonanoyloxy)benzene-1-sulfonate (NOBS), and/or those disclosed in WO98/17767. A particular family of bleach activators of interest was disclosed in EP624154 and particularly preferred in that family is acetyl triethyl citrate (ATC). ATC or a short chain triglyceride like triacetin has the advantage that they are environmentally friendly. Furthermore, acetyl triethyl citrate and triacetin have good hydrolytical stability in the product upon storage and are efficient bleach activators. Finally, ATC is multifunctional, as the citrate released in the perhydrolysis reaction may function as a builder.

The bleaching system may also include a bleach catalyst or booster.

Some non-limiting examples of bleach catalysts that may be used in the compositions of the present invention include manganese oxalate, manganese acetate, manganese-collagen, cobalt-amine catalysts and manganese triazacyclononane (MnTACN) catalysts; particularly preferred are complexes of manganese with 1,4,7-trimethyl-1,4,7-triazacyclononane (Me3-TACN) or 1,2,4,7-tetramethyl-1,4,7-triazacyclononane (Me4-TACN), in particular Me3-TACN, such as the dinuclear manganese complex [(Me3-TACN)Mn(O)3Mn(Me3-TACN)](PF6)2, and [2,2',2''-nitrilotris(ethane-1,2-diylazanylylidene-κN-methanylylidene)triphenolato-κ3O]manganese(III). The bleach catalysts may also be other metal compounds; such as iron or cobalt complexes.

In some embodiments, where a source of a peracid is included, an organic bleach catalyst or bleach booster may be used having one of the following formulae:

(i) 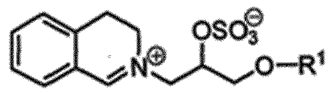

(ii) 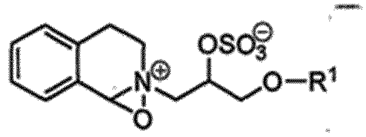

(iii) and mixtures thereof; wherein each R1 is independently a branched alkyl group containing from 9 to 24 carbons or linear alkyl group containing from 11 to 24 carbons, preferably each R1 is independently a branched alkyl group containing from 9 to 18 carbons or linear alkyl group containing from 11 to 18 carbons, more preferably each R1 is independently selected from the group consisting of 2-propylheptyl, 2-butyloctyl, 2-pentylnonyl, 2-hexyldecyl, dodecyl, tetradecyl, hexadecyl, octadecyl, isononyl, isodecyl, isotridecyl and isopentadecyl.

Other exemplary bleaching systems are described, e.g. in WO2007/087258, WO2007/087244, WO2007/087259, EP1867708 (Vitamin K) and WO2007/087242. Suitable photobleaches may for example be sulfonated zinc or aluminium phthalocyanines.

The compositions may comprise metal care agents. Metal care agents may prevent or reduce the tarnishing, corrosion or oxidation of metals, including aluminium, stainless steel and non-ferrous metals, such as silver and copper. Suitable examples include one or more of the following:

(a) benzatriazoles, including benzotriazole or bis-benzotriazole and substituted derivatives thereof. Benzotriazole derivatives are those compounds in which the available substitution sites on the aromatic ring are partially or completely substituted. Suitable substituents include linear or branch-chain Ci-C20-alkyl groups (e.g., C1-C20- alkyl groups) and hydroxyl, thio, phenyl or halogen such as fluorine, chlorine, bromine and iodine.

(b) metal salts and complexes chosen from the group consisting of zinc, manganese, titanium, zirconium, hafnium, vanadium, cobalt, gallium and cerium salts and/or complexes, the metals being in one of the oxidation states II, III, IV, V or VI. In one aspect, suitable metal salts and/or metal complexes may be chosen from the group consisting of Mn(II) sulphate, Mn(II) citrate, Mn(II) stearate, Mn(II) acetylacetonate, K^TiF6 (e.g., K2TiF6), K^ZrF6 (e.g., K2ZrF6), CoSO4, Co(NOs)2 and Ce(NOs)3, zinc salts, for example zinc sulphate, hydrozincite or zinc acetate.;

(c) silicates, including sodium or potassium silicate, sodium disilicate, sodium metasilicate, crystalline phyllosilicate and mixtures thereof.

Further suitable organic and inorganic redox-active substances that act as silver/copper corrosion inhibitors are disclosed in WO 94/26860 and WO 94/26859. Preferably the composition of the invention comprises from 0.1 to 5% by weight of the composition of a metal care agent, preferably the metal care agent is a zinc salt.

The cleaning composition may contain 0-10% by weight, for example 0-5% by weight, such as about 0.5 to about 5%, or about 3% to about 5%, of a hydrotrope. Any hydrotrope known in the art for use in detergents may be utilized. Non-limiting examples of hydrotropes include sodium benzenesulfonate, sodium p-toluene sulfonate (STS),

sodium xylene sulfonate (SXS), sodium cumene sulfonate (SCS), sodium cymene sulfonate, amine oxides, alcohols and polyglycolethers, sodium hydroxynaphthoate, sodium hydroxynaphthalene sulfonate, sodium ethylhexyl sulfate, and combinations thereof.

The cleaning composition may contain 0-10% by weight, such as 0.5-5%, 2-5%, 0.5-2% or 0.2-1% of a polymer. Any polymer known in the art for use in detergents may be utilized. The polymer may function as a co-builder as mentioned above, or may provide antiredeposition, fiber protection, soil release, dye transfer inhibition, grease cleaning and/or anti-foaming properties. Some polymers may have more than one of the above-mentioned properties and/or more than one of the below-mentioned motifs. Exemplary polymers include (carboxymethyl)cellulose (CMC), poly(vinyl alcohol) (PVA), poly(vinylpyrrolidone) (PVP), poly(ethyleneglycol) or poly(ethylene oxide) (PEG), ethoxylated poly(ethyleneimine), carboxymethyl inulin (CMI), and polycarboxylates such as PAA, PAA/PMA, poly-aspartic acid, and lauryl methacrylate/acrylic acid copolymers , hydrophobically modified CMC (HM-CMC) and silicones, copolymers of terephthalic acid and oligomeric glycols, copolymers of poly(ethylene terephthalate) and poly(oxyethene terephthalate) (PET-POET), PVP, poly(vinylimidazole) (PVI), poly(vinylpyridine-N-oxide) (PVPO or PVPNO) and polyvinylpyrrolidone-vinylimidazole (PVPVI). Suitable examples include PVP-K15, PVP-K30, ChromaBond S-400, ChromaBond S- 403E and Chromabond S-100 from Ashland Aqualon, and Sokalan® HP 165, Sokalan® HP 50 (Dispersing agent), Sokalan® HP 53 (Dispersing agent), Sokalan® HP 59 (Dispersing agent), Sokalan® HP 56 (dye transfer inhibitor), Sokalan® HP 66 K (dye transfer inhibitor) from BASF. Further exemplary polymers include sulfonated polycarboxylates, polyethylene oxide and polypropylene oxide (PEO-PPO) and diquaternium ethoxy sulfate. Other exemplary polymers are disclosed in, e.g., WO 2006/130575. Salts of the above-mentioned polymers are also contemplated. Particularly preferred polymer is ethoxylated homopolymer Sokalan® HP 20 from BASF, which helps to prevent redeposition of soil in the wash liquor.

The cleaning composition of the present invention may also include fabric hueing agents such as dyes or pigments, which when formulated in detergent compositions can deposit onto a fabric when said fabric is contacted with a wash liquor comprising said detergent compositions and thus altering the tint of said fabric through absorption/reflection of visible light. Fluorescent whitening agents emit at least some visible light. In contrast, fabric hueing agents alter the tint of a surface as they absorb at least a portion of the visible light spectrum. Suitable fabric hueing agents include dyes and dye-clay conjugates, and may also include pigments. Suitable dyes include small molecule dyes and polymeric dyes. Suitable small molecule dyes include small molecule dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof, for example as described in WO2005/03274, WO2005/03275, WO2005/03276 and EP1876226 (hereby incorporated by reference). The detergent composition preferably comprises from about 0.00003 wt% to about 0.2 wt%, from about 0.00008 wt% to about 0.05 wt%, or even from about 0.0001 wt% to about 0.04 wt% fabric hueing agent. The composition may comprise from 0.0001 wt% to 0.2 wt% fabric hueing agent, this may be especially preferred when the composition is in the form of a unit dose pouch. Suitable hueing agents are also disclosed in, e.g. WO 2007/087257 and WO2007/087243.

The cleaning composition may comprise one or more additional enzymes such as one or more lipase, cutinase, an amylase, carbohydrase, cellulase, pectinase, mannanase, arabinase, galactanase, xylanase, oxidase, e.g., a laccase, and/or peroxidase.

In general, the properties of the selected enzyme(s) should be compatible with the selected detergent, (i.e., pH-optimum, compatibility with other enzymatic and non-enzymatic ingredients, etc.), and the enzyme(s) should be present in effective amounts.

Suitable proteases for the compositions of the invention include those of bacterial, fungal, plant, viral or animal origin e.g. vegetable or microbial origin. Microbial origin is preferred. Chemically modified or protein engineered mutants are included. It may be an alkaline protease, such as a serine protease or a metalloprotease. A serine protease may for example be of the S1 family, such as trypsin, or the S8 family such as subtilisin. A metalloproteases protease may for example be a thermolysin from e.g. family M4 or other metalloprotease such as those from M5, M7 or M8 families.

Examples of subtilases are those derived from *Bacillus* such as *Bacillus lentus, Bacillus alkalophilus, Bacillus subtilis, Bacillus amyloliquefaciens, Bacillus pumilus* and *Bacillus gibsonii* described in; US7262042 and WO09/021867. *Subtilisin lentus, Subtilisin* Novo, *subtilisin* Carlsberg, *Bacillus licheniformis, subtilisin* BPN', *subtilisin* 309, *subtilisin* 147 *and subtilisin* 168 and e.g. protease PD138 described in (WO93/18140). Other useful proteases may be those described in WO01/016285 and WO02/016547. Examples of trypsin-like proteases are trypsin (e.g. of porcine or bovine origin) and the Fusarium protease described in WO94/25583 and WO05/040372, and the chymotrypsin proteases derived from Cellumonas described in WO05/052161 and WO05/052146.

A further preferred protease is the alkaline protease from Bacillus lentus DSM 5483, as described for example in WO95/23221, and variants thereof which are described in WO92/21760, WO95/23221, EP1921147 and EP1921148.

Examples of metalloproteases are the neutral metalloprotease as described in WO07/044993 (Proctor & Gamble/Genencor Int.) such as those derived from Bacillus amyloliquefaciens.

Examples of useful proteases are the variants described in: WO89/06279, WO92/19729, WO96/034946, WO98/20115, WO98/20116, WO99/011768, WO01/44452, WO03/006602, WO04/03186, WO04/041979,

WO07/006305, WO11/036263, WO11/036264, especially the variants with substitutions in one or more of the following positions: 3, 4, 9, 15, 24, 27, 42, 55, 59, 60, 66, 74, 85, 96, 97, 98, 99, 100, 101, 102, 104, 116, 118, 121, 126, 127, 128, 154, 156, 157, 158, 161, 164, 176, 179, 182, 185, 188, 189, 193, 198, 199, 200, 203, 206, 211, 212, 216, 218, 226, 229, 230, 239, 246, 255, 256, 268 and 269 wherein the positions correspond to the positions of the *Bacillus lentus* protease shown in SEQ ID NO 1 of WO 2016/001449. More preferred the protease variants may comprise one or more of the mutations selected from the group consisting of: S3T, V4I, S9R, S9E, A15T, S24G, S24R, K27R, N42R, S55P, G59E, G59D, N60D, N60E, V66A, N74D, S85R, A96S, S97G, S97D, S97A, S97SD, S99E, S99D, S99G, S99M, S99N, S99R, S99H, S101A, V102I, V102Y, V102N, S104A, G116V, G116R, H118D, H118N, A120S, S126L, P127Q, S128A, S154D, A156E, G157D, G157P, S158E, Y161A, R164S, Q176E, N179E, S182E, Q185N, A188P, G189E, V193M, N198D, V199I, Y203W, S206G, L211Q, L211D, N212D, N212S, M216S, A226V, K229L, Q230H, Q239R, N246K, N255W, N255D, N255E, L256E, L256D T268A and R269H. The protease variants are preferably variants of the *Bacillus lentus* protease (Savinase®) shown in SEQ ID NO 1 of WO 2016/001449, the *Bacillus amylolichenifaciens* protease (BPN') shown in SEQ ID NO 2 of WO2016/001449. The protease variants preferably have at least 80 % sequence identity to SEQ ID NO 1 or SEQ ID NO 2 of WO 2016/001449. A protease variant comprising a substitution at one or more positions corresponding to positions 171, 173, 175, 179, or 180 of SEQ ID NO: 1 of WO2004/067737, wherein said protease variant has a sequence identity of at least 75% but less than 100% to SEQ ID NO: 1 of WO2004/067737.

Suitable commercially available protease enzymes include those sold under the trade names Alcalase®, Duralase™, Durazym™, Relase®, Relase® Ultra, Savinase®, Savinase® Ultra, Primase®, Polarzyme®, Kannase®, Liquanase®, Liquanase® Ultra, Ovozyme®, Coronase®, Coronase® Ultra, Blaze®, Blaze Evity® 100T, Blaze Evity® 125T, Blaze Evity® 150T, Neutrase®, Everlase® and Esperase® (Novozymes A/S), those sold under the tradename Maxatase®, Maxacal®, Maxapem®, Purafect Ox®, Purafect OxP®, Puramax®, FN2®, FN3®, FN4®, Excellase®, Excellenz P1000™, Excellenz P1250™, Eraser®, Preferenz P100™, Purafect Prime®, Preferenz P110™, Effectenz P1000™, Purafect®™, Effectenz P1050™, Purafect Ox®™, Effectenz P2000™, Purafast®, Properase®, Opticlean® and Optimase® (Danisco/DuPont), Axapem™ (Gist-Brocases N.V.), BLAP (sequence shown in Figure 29 of US5352604) and variants hereof (Henkel AG) and KAP (*Bacillus alkalophilus* subtilisin) from Kao.

The protease, as described above, may be stabilized using conventional stabilizing agents, e.g., a polyol such as glycerol, (mono, di, or tri) propylene glycol, sugar alcohol, polypropylene glycol, and/or polyethylene glycol, preferably polyethylene glycol or polypropylene glycol with a molecular weight in the range of 200-1000; or compounds that act by temporarily reducing the activity of proteases (reversible inhibitors).

Thus, the composition of the invention may also include a protease inhibitor/stabilizer, which is a reversible inhibitor of protease activity, e.g., serine protease activity. Preferably, the protease inhibitor is a (reversible) subtilisin protease inhibitor. In particular, the protease inhibitor may be a peptide aldehyde, boric acid, or a boronic acid; or a derivative of any of these.

The protease inhibitor may have an inhibition constant to a serine protease, Ki (mol/L) of from 1E-12 to 1E-03; more preferred from 1E-11 to 1E-04; even more preferred from 1E-10 to 1E-05; even more preferred from 1E-10 to 1E-06; and most preferred from 1E-09 to 1E-07.

The protease inhibitor may be a boronic acid or a derivative thereof; preferably, a phenylboronic acid or a derivative thereof. In an embodiment of the invention, the phenyl boronic acid derivative is of the following formula:

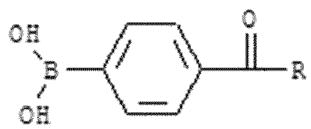

wherein R is selected from the group consisting of hydrogen, hydroxy, C1-C6 alkyl, substituted C1-C6 alkyl, C1-C6 alkenyl and substituted C1-C6 alkenyl. Preferably, R is hydrogen, CH3, CH3CH2 or CH3CH2CH2.

In a preferred embodiment, the protease inhibitor (phenyl boronic acid derivative) is 4-formyl-phenyl boronic acid (4-FPBA).

In another particular embodiment, the protease inhibitor is selected from the group consisting of thiophene-2 boronic acid, thiophene-3 boronic acid, acetamidophenyl boronic acid, benzofuran-2 boronic acid, naphtalene-1 boronic acid, naphtalene-2 boronic acid, 2-FPBA, 3-FBPA, 4-FPBA, 1-thianthrene boronic acid, 4-dibenzofuran boronic acid, 5-methylthiophene-2 boronic, acid, thionaphtrene boronic acid, furan-2 boronic acid, furan-3 boronic acid, 4,4 biphenyl-diborinic acid, 6-hydroxy-2-naphtalene, 4-(methylthio) phenyl boronic acid, 4 (trimethyl-silyl)phenyl boronic acid, 3-bromothiophene boronic acid, 4-methylthiophene boronic acid, 2-naphtyl boronic acid, 5-bromothiphene boronic acid, 5-chlorothiophene boronic acid, dimethylthiophene boronic acid, 2-bromophenyl boronic acid, 3-chlorophenyl boronic acid, 3-methoxy-2-thiophene, p-methyl-phenylethyl boronic acid, 2-thianthrene boronic acid, di-benzothiophene boronic acid, 4-carboxyphenyl boronic acid, 9-anthryl boronic acid, 3,5 dichlorophenyl boronic, acid, diphenyl boronic acidanhydride,

o-chlorophenyl boronic acid, p-chlorophenyl boronic acid, m-bromophenyl boronic acid, p-bromophenyl boronic acid, p-flourophenyl boronic acid, p-tolyl boronic acid, o-tolyl boronic acid, octyl boronic acid, 1,3,5 trimethylphenyl boronic acid, 3-chloro-4-flourophenyl boronic acid, 3-aminophenyl boronic acid, 3,5-bis-(triflouromethyl) phenyl boronic acid, 2,4 dichlorophenyl boronic acid, and 4-methoxyphenyl boronic acid.

Further boronic acid derivatives suitable as protease inhibitors in the detergent composition are described in US 4,963,655, US 5,159,060, WO 95/12655, WO 95/29223, WO 92/19707, WO 94/04653, WO 94/04654, US 5442100, US 5488157 and US 5472628.

The protease stabilizer may have the formula: P-(A)y-L-(B)x-B0-R* wherein:

R* is H (hydrogen), $CH_3$, $CX_3$, $CHX_2$, or $CH_2X$. Preferably, R*=H so that the stabilizer is a peptide aldehyde with the formula P-(A)y-L-(B)x-BO-H;
X is a halogen atom, particularly F (fluorine);
B0 is a single amino acid residue with L- or D-configuration of the formula -NH-CH(R)-C(=O)-;
x is 1,2 or 3;
Bx is independently a single amino acid residue, each connected to the next B or to B0 via its C-terminal;
L is absent or independently a linker group of the formula -C(=O)-, -C(=O)-C(=O)-, -C(=S)-, - C(=S)-C(=S)- or -C(=S)-C(=O)-;
A is absent if L is absent or is independently a single amino acid residue connected to L via the N-terminal of the amino acid;
P is selected from the group consisting of hydrogen or if L is absent an N-terminal protection group;
y is 0, 1, or 2,
R is independently selected from the group consisting of $C_{1-6}$ alkyl, $C_{6-10}$ aryl or $C_{7-10}$ arylalkyl, optionally substituted with one or more, identical or different, substituent's R';
R' is independently selected from the group consisting of halogen, -OH, -OR", -SH, -SR", $-NH_2$, -NHR", $-NR"_2$, $-CO_2H$, $-CONH_2$, -CONHR", $-CONR"_2$, $-NHC(=N)NH_2$; and
R" is a $C_{1-6}$ alkyl group.
x may be 1, 2 or 3 and therefore B may be 1, 2 or 3 amino acid residues respectively. Thus, B may represent B1, B2-B1 or B3-B2-B1, where B3, B2 and B1 each represent one amino acid residue. y may be 0, 1 or 2 and therefore A may be absent, or 1 or 2 amino acid residues respectively having the formula A1 or A2-A1 wherein A2 and A1 each represent one amino acid residue.

B0 may be a single amino acid residue with L- or D-configuration, which is connected to H via the C-terminal of the amino acid. B0 has the formula -NH-CH(R)-C(=O)-, wherein R is a $C_{1-6}$ alkyl, $C_{6-10}$ aryl or $C_{7-10}$ arylalkyl side chain, such as methyl, ethyl, propyl, isopropyl, butyl, isobutyl, phenyl or benzyl, and wherein R may be optionally substituted with one or more, identical or different, substituents R'. Particular examples of B0 are the D- or L-form of arginine (Arg), 3,4-dihydroxyphenylalanine, isoleucine (Ile), leucine (Leu), methionine (Met), norleucine (Nle), norvaline (Nva), phenylalanine (Phe), m-tyrosine, p-tyrosine (Tyr) and valine (Val). A particular embodiment is when B0 is leucine, methionine, phenylalanine, p-tyrosine and valine.

B1, which is connected to B0 via the C-terminal of the amino acid, may be an aliphatic, hydrophobic and/or neutral amino acid. Examples of B1 are alanine (Ala), cysteine (Cys), glycine (Gly), isoleucine (Ile), leucine (Leu), norleucine (Nle), norvaline (Nva), proline (Pro), serine (Ser), threonine (Thr) and valine (Val). Particular examples of B1 are alanine, glycine, isoleucine, leucine and valine. A particular embodiment is when B1 is alanine, glycine or valine.

If present, B2, which is connected to B1 via the C-terminal of the amino acid, may be an aliphatic, hydrophobic, neutral and/or polar amino acid. Examples of B2 are alanine (Ala), arginine (Arg), capreomycidine (Cpd), cysteine (Cys), glycine (Gly), isoleucine (Ile), leucine (Leu), norleucine (Nle), norvaline (Nva), phenylalanine (Phe), proline (Pro), serine (Ser), threonine (Thr), and valine (Val). Particular examples of B2 are alanine, arginine, capreomycidine, glycine, isoleucine, leucine, phenylalanine and valine. A particular embodiment is when B2 is arginine, glycine, leucine, phenylalanine or valine.

B3, which if present is connected to B2 via the C-terminal of the amino acid, may be a large, aliphatic, aromatic, hydrophobic and/or neutral amino acid. Examples of B3 are isoleucine (Ile), leucine (Leu), norleucine (Nle), norvaline (Nva), phenylalanine (Phe), phenylglycine, tyrosine (Tyr), tryptophan (Trp) and valine (Val). Particular examples of B3 are leucine, phenylalanine, tyrosine and tryptophan.

The linker group L may be absent or selected from the group consisting of -C(=O)-, -C(=O)-C(=O)-, - C(=S)-, -C(=S)-C(=S)- or -C(=S)-C(=O)-. Particular embodiments of the invention are when L is absent or L is a carbonyl group -C(=O)-.

A1, which if present is connected to L via the N-terminal of the amino acid, may be an aliphatic, aromatic, hydrophobic, neutral and/or polar amino acid. Examples of A1 are alanine (Ala), arginine (Arg), capreomycidine (Cpd), glycine (Gly), isoleucine (Ile), leucine (Leu), norleucine (Nle), norvaline (Nva), phenylalanine (Phe), threonine (Thr),

tyrosine (Tyr), tryptophan (Trp) and valine (Val). Particular examples of A1 are alanine, arginine, glycine, leucine, phenylalanine, tyrosine, tryptophan and valine. A particular embodiment is when B2 is leucine, phenylalanine, tyrosine or tryptophan.

The A2 residue, which if present is connected to A1 via the N-terminal of the amino acid, may be a large, aliphatic, aromatic, hydrophobic and/or neutral amino acid. Examples of A2 are arginine (Arg), isoleucine (Ile), leucine (Leu), norleucine (Nle), norvaline (Nva), phenylalanine (Phe), phenylglycine, Tyrosine (Tyr), tryptophan (Trp) and valine (Val). Particular examples of A2 are phenylalanine and tyrosine.

The N-terminal protection group P (if present) may be selected from formyl, acetyl (Ac), benzoyl (Bz), trifluoroacetyl, methoxysuccinyl, aromatic and aliphatic urethane protecting groups such as fluorenylmethyloxycarbonyl (Fmoc), methoxycarbonyl (Moc), (fluoromethoxy)carbonyl, benzyloxycarbonyl (Cbz), t-butyloxycarbonyl (Boc) and adamantyloxycarbonyl; p-methoxybenzyl carbonyl, benzyl (Bn), p-methoxybenzyl (PMB), p-methoxyphenyl (PMP), methoxyacetyl, methylamino carbonyl, methylsulfonyl, ethylsulfonyl, benzylsulfonyl, methylphosphoramidyl (MeOP(OH)(=O)) and benzylphosphoramidyl ($PhCH_2OP(OH)(=O)$).

In the case of a tripeptide aldehyde with a protection group (i.e. x=2, L is absent and A is absent), P is preferably acetyl, methoxycarbonyl, benzyloxycarbonyl, methylamino carbonyl, methylsulfonyl, benzylsulfonyl and benzylphosphoramidyl. In the case of a tetrapeptide aldehyde with a protection group (i.e. x=3, L is absent and A is absent), P is preferably acetyl, methoxycarbonyl, methylsulfonyl, ethylsulfonyl and methylphosphoramidyl.

Suitable peptide aldehydes are described in WO94/04651, WO95/25791, WO98/13458, WO98/13459, WO98/13460, WO98/13461, WO98/13462, WO07/141736, WO07/145963, WO09/118375, WO10/055052 and WO11/036153. More particularly, the peptide aldehyde may be Cbz-Arg-Ala-Tyr-H, Ac-Gly-Ala-Tyr-H, Cbz-Gly-Ala-Tyr-H, Cbz-Gly-Ala-Tyr-$CF_3$, Cbz-Gly-Ala-Leu-H, Cbz-Val-Ala-Leu-H, Cbz-Val-Ala-Leu-$CF_3$, Moc-Val-Ala-Leu-$CF_3$, Cbz-Gly-Ala-Phe-H, Cbz-Gly-Ala-Phe-$CF_3$, Cbz-Gly-Ala-Val-H, Cbz-Gly-Gly-Tyr-H, Cbz-Gly-Gly-Phe-H, Cbz-Arg-Val-Tyr-H, Cbz-Leu-Val-Tyr-H, Ac-Leu-Gly-Ala-Tyr-H, Ac-Phe-Gly-Ala-Tyr-H, Ac-Tyr-Gly-Ala-Tyr-H, Ac-Phe-Gly-Ala-Leu-H, Ac-Phe-Gly-Ala-Phe-H, Ac-Phe-Gly-Val-Tyr-H, Ac-Phe-Gly-Ala-Met-H, Ac-Trp-Leu-Val-Tyr-H, MeO-CO-Val-Ala-Leu-H, MeNCO-Val-Ala-Leu-H, MeO-CO-Phe-Gly-Ala-Leu-H, MeO-CO-Phe-Gly-Ala-Phe-H, $MeSO_2$-Phe-Gly-Ala-Leu-H, $MeSO_2$-Val-Ala-Leu-H, $PhCH_2O$-P(OH)(O)-Val-Ala-Leu-H, $EtSO_2$-Phe-Gly-Ala-Leu-H, $PhCH_2SO_2$-Val-Ala-Leu-H, $PhCH_2O$-P(OH)(O)-Leu-Ala-Leu-H, $PhCH_2O$-P(OH)(O)-Phe-Ala-Leu-H, or MeO-P(OH)(O)-Leu-Gly-Ala-Leu-H. A preferred stabilizer for use in the liquid composition of the invention is Cbz-Gly-Ala-Tyr-H, or a hydrosulfite adduct thereof, wherein Cbz is benzyloxycarbonyl. Further examples of such peptide aldehydes include $\alpha$-MAPI, $\beta$-MAPI, Phe-C(=O)-Arg-Val-Tyr-H, Phe-C(=O)-Gly-Gly-Tyr-H, Phe-C(=O)-Gly-Ala-Phe-H, Phe-C(=O)-Gly-Ala-Tyr-H, Phe-C(=O)-Gly-Ala-L-H, Phe-C(=O)-Gly-Ala-Nva-H, Phe-C(=O)-Gly-Ala-Nle-H, Tyr-C(=O)-Arg-Val-Tyr-H, Tyr-C(=O)-Gly-Ala-Tyr-H, Phe-C(=S)-Arg-Val-Phe-H, Phe-C(=S)-Arg-Val-Tyr-H, Phe-C(=S)-Gly-Ala-Tyr-H, Antipain, GE20372A, GE20372B, Chymostatin A, Chymostatin B, and Chymostatin C.

The protease stabilizer may be a hydrosulfite adduct of the peptide aldehyde described above, e.g. as described in WO 2013/004636. The adduct may have the formula P-(A)y-L-(B)x-N(H)-CHR-CH(OH)-$SO_3M$, wherein P, A, y, L, B, x and R are defined as above, and M is H or an alkali metal, preferably Na or K.

An aqueous solution of the hydrosulfite adduct may be prepared by reacting the corresponding peptide aldehyde with an aqueous solution of sodium bisulfite (sodium hydrogen sulfite, $NaHSO_3$); potassium bisulfite ($KHSO_3$) by known methods, e.g., as described in WO 98/47523; US 6,500,802; US 5,436,229; J. Am. Chem. Soc. (1978) 100, 1228; Org. Synth., Coll. vol. 7: 361.

Particularly preferred peptide aldehyde protease stabilizers have the formula P-B3-B2-B1-B0-H, or a hydrosulfite adduct having the formula P-B3-B2-B1-N(H)-CHR-CHOH-$SO_3M$, wherein

i) H is hydrogen;
ii) B0 is a single amino acid residue with L- or D-configuration of the formula -NH-CH(R)-C(=O)-;
iii) B1 and B2 are independently single amino acid residues;
iv) B3 is a single amino acid residue, or is absent;
v) R is independently selected from the group consisting of $C_{1-6}$ alkyl, $C_{6-10}$ aryl or $C_{7-10}$ arylalkyl optionally substituted with one or more, identical or different, substituents R';
vi) R' is independently selected from the group consisting of halogen, -OH, -OR", -SH, -SR", $-NH_2$,-NHR", $-NR"_2$, $-CO_2H$, $-CONH_2$, -CONHR", $-CONR"_2$, $-NHC(=N)NH_2$;
vii) R" is a $C_{1-6}$ alkyl group;
viii) P is an N-terminal protection group, preferably methoxycarbonyl (Moc) or benzyloxycarbonyl (Cbz); and
ix) M is H or an alkali metal, preferably Na or K.

In an even more preferred embodiment, the peptide aldehyde protease stabilizer has the formula P-B2-B1-B0-H or an adduct having the formula P-B2-B1-N(H)-CHR-CHOH-$SO_3M$, wherein

i) H is hydrogen;
ii) B0 is a single amino acid residue with L- or D-configuration of the formula -NH-CH(R)-C(=O)-;
iii) B1 and B2 are independently single amino acid residues;
iv) R is independently selected from the group consisting of $C_{1-6}$ alkyl, $C_{6-10}$ aryl or $C_{7-10}$ arylalkyl optionally substituted with one or more, identical or different, substituents R';
v) R' is independently selected from the group consisting of halogen, -OH, -OR", -SH, -SR", $-NH_2$,-NHR", $-NR"_2$, $-CO_2H$, $-CONH_2$, -CONHR", $-CONR"_2$, $-NHC(=N)NH_2$;
vi) R" is a $C_{1-6}$ alkyl group;
vii) P is an N-terminal protection group, preferably methoxycarbonyl (Moc) or benzyloxycarbonyl (Cbz); and
viii) M is H or an alkali metal, preferably Na or K.

Preferred embodiments of B0, B1, B2, B3, and P are as described above.

The molar ratio of the above-mentioned peptide aldehydes (or hydrosulfite adducts) to the protease may be at least 1:1 or 1.5:1, and it may be less than 1000:1, more preferred less than 500:1, even more preferred from 100:1 to 2:1 or from 20:1 to 2:1, or most preferred, the molar ratio is from 10:1 to 2:1.

Formate salts (e.g., sodium formate) and formic acid have also shown good effects as inhibitor of protease activity. Formate can be used synergistically with the above-mentioned protease inhibitors, as shown in WO 2013/004635. The formate salts may be present in the slurry composition in an amount of at least 0.1% w/w or 0.5% w/w, e.g., at least 1.0%, at least 1.2% or at least 1.5%. The amount is typically below 5% w/w, below 4% or below 3%.

In an embodiment, the protease is a metalloprotease and the inhibitor is a metalloprotease inhibitor, e.g., a protein hydrolysate based inhibitor (e.g., as described in WO 2008/134343).

Suitable cellulases include those of bacterial or fungal origin. Chemically modified or protein engineered mutants are included. Suitable cellulases include cellulases from the genera Bacillus, Pseudomonas, Humicola, Fusarium, Thielavia, Acremonium, e.g., the fungal cellulases produced from Humicola insolens, Myceliophthora thermophila and Fusarium oxysporum disclosed in US 4,435,307, US 5,648,263, US 5,691,178, US 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and WO99/001544.

Other cellulases are endo-beta-1,4-glucanase enzyme having a sequence of at least 97% identity to the amino acid sequence of position 1 to position 773 of SEQ ID NO:2 of WO 2002/099091 or a family 44 xyloglucanase, which a xyloglucanase enzyme having a sequence of at least 60% identity to positions 40-559 of SEQ ID NO: 2 of WO 2001/062903.

Commercially available cellulases include Celluzyme™, and Carezyme™ (Novozymes A/S) Carezyme Premium™ (Novozymes A/S), Celluclean ™ (Novozymes A/S), Celluclean Classic™ (Novozymes A/S), Cellusoft™ (Novozymes A/S), Whitezyme™ (Novozymes A/S), Clazinase™, and Puradax HA™ (Genencor International Inc.), and KAC-500(B)™ (Kao Corporation).

Suitable mannanases include those of bacterial or fungal origin. Chemically or genetically modified mutants are included. The mannanase may be an alkaline mannanase of Family 5 or 26. It may be a wild-type from Bacillus or Humicola, particularly B. agaradhaerens, B. licheniformis, B. halodurans, B. clausii, or H. insolens. Suitable mannanases are described in WO1999/064619. A commercially available mannanase is Mannaway (Novozymes A/S).

Suitable peroxidases/oxidases include those of plant, bacterial or fungal origin. Chemically modified or protein engineered mutants are included. Examples of useful peroxidases include peroxidases from Coprinus, e.g., from C. cinereus, and variants thereof as those described in WO 93/24618, WO 95/10602, and WO 98/15257. Commercially available peroxidases include Guardzyme™ (Novozymes A/S).

Suitable lipases and cutinases include those of bacterial or fungal origin. Chemically modified or protein engineered mutant enzymes are included. Examples include lipase from Thermomyces, e.g. from T. lanuginosus (previously named Humicola lanuginosa) as described in EP258068 and EP305216, cutinase from Humicola, e.g. H. insolens (WO96/13580), lipase from strains of Pseudomonas (some of these now renamed to Burkholderia), e.g. P. alcaligenes or P. pseudoalcaligenes (EP218272), P. cepacia (EP331376), P. sp. strain SD705 (WO95/06720 & WO96/27002), P. wisconsinensis (WO96/12012), GDSL-type Streptomyces lipases (WO10/065455), cutinase from Magnaporthe grisea (WO10/107560), cutinase from Pseudomonas mendocina (US5,389,536), lipase from Thermobifida fusca (WO11/084412), Geobacillus stearothermophilus lipase (WO11/084417), lipase from Bacillus subtilis (WO11/084599), and lipase from Streptomyces griseus (WO11/150157) and S. pristinaespiralis (WO12/137147).

Other examples are lipase variants such as those described in EP407225, WO92/05249, WO94/01541, WO94/25578, WO95/14783, WO95/30744, WO95/35381, WO95/22615, WO96/00292, WO97/04079, WO97/07202, WO00/34450, WO00/60063, WO01/92502, WO07/87508 and WO09/109500.

Preferred commercial lipase products include Lipolase™, Lipex™; Lipolex™ and Lipoclean™ (Novozymes

A/S), Lumafast (originally from Genencor) and Lipomax (originally from Gist-Brocades).

Still other examples are lipases sometimes referred to as acyltransferases or perhydrolases, e.g. acyltransferases with homology to Candida antarctica lipase A (WO10/111143), acyltransferase from Mycobacterium smegmatis (WO05/56782), perhydrolases from the CE 7 family (WO09/67279), and variants of the M. smegmatis perhydrolase in particular the S54V variant used in the commercial product Gentle Power Bleach from Huntsman Textile Effects Pte Ltd (WO10/100028).

Suitable amylases include alpha-amylases and/or a glucoamylases and may be of bacterial or fungal origin. Chemically modified or protein engineered mutants are included. Amylases include, for example, alpha-amylases obtained from Bacillus, e.g., a special strain of Bacillus licheniformis, described in more detail in GB 1,296,839.

Suitable amylases include amylases having SEQ ID NO: 2 in WO 95/10603 or variants having 90% sequence identity to SEQ ID NO: 3 thereof. Preferred variants are described in WO 94/02597, WO 94/18314, WO 97/43424 and SEQ ID NO: 4 of WO 99/019467, such as variants with substitutions in one or more of the following positions: 15, 23, 105, 106, 124, 128, 133, 154, 156, 178, 179, 181, 188, 190, 197, 201, 202, 207, 208, 209, 211, 243, 264, 304, 305, 391, 408, and 444.

Different suitable amylases include amylases having SEQ ID NO: 6 in WO 02/010355 or variants thereof having 90% sequence identity to SEQ ID NO: 6. Preferred variants of SEQ ID NO: 6 are those having a deletion in positions 181 and 182 and a substitution in position 193.

Other amylases which are suitable are hybrid alpha-amylase comprising residues 1-33 of the alpha-amylase derived from B. amyloliquefaciens shown in SEQ ID NO: 6 of WO 2006/066594 and residues 36-483 of the B. licheniformis alpha-amylase shown in SEQ ID NO: 4 of WO 2006/066594 or variants having 90% sequence identity thereof. Preferred variants of this hybrid alpha-amylase are those having a substitution, a deletion or an insertion in one of more of the following positions: G48, T49, G107, H156, A181, N190, M197, 1201, A209 and Q264. Most preferred variants of the hybrid alpha-amylase comprising residues 1-33 of the alpha-amylase derived from B. amyloliquefaciens shown in SEQ ID NO: 6 of WO 2006/066594 and residues 36-483 of SEQ ID NO: 4 are those having the substitutions:

M197T;
H156Y+A181T+N190F+A209V+Q264S;or
G48A+T49I+G107A+H156Y+A181T+N190F+I201F+A209V+Q264S.

Further amylases which are suitable are amylases having SEQ ID NO: 6 in WO 99/019467 or variants thereof having 90% sequence identity to SEQ ID NO: 6. Preferred variants of SEQ ID NO: 6 are those having a substitution, a deletion or an insertion in one or more of the following positions: R181, G182, H183, G184, N195, I206, E212, E216 and K269. Particularly preferred amylases are those having deletion in positions R181 and G182, or positions H183 and G184.

Additional amylases which can be used are those having SEQ ID NO: 1, SEQ ID NO: 3, SEQ ID NO: 2 or SEQ ID NO: 7 of WO 96/023873 or variants thereof having 90% sequence identity to SEQ ID NO: 1, SEQ ID NO: 2, SEQ ID NO: 3 or SEQ ID NO: 7. Preferred variants of SEQ ID NO: 1, SEQ ID NO: 2, SEQ ID NO: 3 or SEQ ID NO: 7 are those having a substitution, a deletion or an insertion in one or more of the following positions: 140, 181, 182, 183, 184, 195, 206, 212, 243, 260, 269, 304 and 476, using SEQ ID 2 of WO 96/023873 for numbering. More preferred variants are those having a deletion in two positions selected from 181, 182, 183 and 184, such as 181 and 182, 182 and 183, or positions 183 and 184. Most preferred amylase variants of SEQ ID NO: 1, SEQ ID NO: 2 or SEQ ID NO: 7 are those having a deletion in positions 183 and 184 and a substitution in one or more of positions 140, 195, 206, 243, 260, 304 and 476.

Other amylases which can be used are amylases having SEQ ID NO: 2 of WO 08/153815, SEQ ID NO: 10 in WO 01/66712 or variants thereof having 90% sequence identity to SEQ ID NO: 2 of WO 08/153815 or 90% sequence identity to SEQ ID NO: 10 in WO 01/66712. Preferred variants of SEQ ID NO: 10 in WO 01/66712 are those having a substitution, a deletion or an insertion in one of more of the following positions: 176, 177, 178, 179, 190, 201, 207, 211 and 264.

Further suitable amylases are amylases having SEQ ID NO: 2 of WO 09/061380 or variants having 90% sequence identity to SEQ ID NO: 2 thereof. Preferred variants of SEQ ID NO: 2 are those having a truncation of the C-terminus and/or a substitution, a deletion or an insertion in one of more of the following positions: Q87, Q98, S125, N128, T131, T165, K178, R180, S181, T182, G183, M201, F202, N225, S243, N272, N282, Y305, R309, D319, Q320, Q359, K444 and G475. More preferred variants of SEQ ID NO: 2 are those having the substitution in one of more of the following positions: Q87E,R, Q98R, S125A, N128C, T131I, T165I, K178L, T182G, M201L, F202Y, N225E,R, N272E,R, S243Q,A,E,D, Y305R, R309A, Q320R, Q359E, K444E and G475K and/or deletion in position R180 and/or S181 or of T182 and/or G183. Most preferred amylase variants of SEQ ID NO: 2 are those having the substitutions:

N128C+K178L + T182G+Y305R+G475K;

N128C+K178L+T182G+F202Y+Y305R+D319T+G475K;
S125A+N128C+K178L+T182G+Y305R+G475K;or
S125A+N128C+T1311+T1651+K178L+T182G+Y305R+G475K wherein the variants are C-terminally truncated and optionally further comprises a substitution at position 243 and/or a deletion at position 180 and/or position 181.

Further suitable amylases are amylases having SEQ ID NO: 1 of WO13184577 or variants having 90% sequence identity to SEQ ID NO: 1 thereof. Preferred variants of SEQ ID NO: 1 are those having a substitution, a deletion or an insertion in one of more of the following positions: K176, R178, G179, T180, G181, E187, N192, M199, I203, S241, R458, T459, D460, G476 and G477. More preferred variants of SEQ ID NO: 1 are those having the substitution in one of more of the following positions: K176L, E187P, N192FYH, M199L, I203YF, S241QADN, R458N, T459S, D460T, G476K and G477K and/or deletion in position R178 and/or S179 or of T180 and/or G181. Most preferred amylase variants of SEQ ID NO: 1 are those having the substitutions:

E187P+I203Y+G476K
E187P+I203Y+R458N+T459S+D460T+G476K

wherein the variants optionally further comprise a substitution at position 241 and/or a deletion at position 178 and/or position 179.

Further suitable amylases are amylases having SEQ ID NO: 1 of WO10104675 or variants having 90% sequence identity to SEQ ID NO: 1 thereof. Preferred variants of SEQ ID NO: 1 are those having a substitution, a deletion or an insertion in one of more of the following positions: N21, D97, V128 K177, R179, S180, 1181, G182, M200, L204, E242, G477 and G478. More preferred variants of SEQ ID NO: 1 are those having the substitution in one of more of the following positions: N21D, D97N, V128IK177L, M200L, L204YF, E242QA, G477K and G478K and/or deletion in position R179 and/or S180 or of 1181 and/or G182. Most preferred amylase variants of SEQ ID NO: 1 are those having the substitutions: N21D+D97N+V128I wherein the variants optionally further comprise a substitution at position 200 and/or a deletion at position 180 and/or position 181.

Other suitable amylases are the alpha-amylase having SEQ ID NO: 12 in WO01/66712 or a variant having at least 90% sequence identity to SEQ ID NO: 12. Preferred amylase variants are those having a substitution, a deletion or an insertion in one of more of the following positions of SEQ ID NO: 12 in WO01/66712: R28, R118, N174; R181, G182, D183, G184, G186, W189, N195, M202, Y298, N299, K302, S303, N306, R310, N314; R320, H324, E345, Y396, R400, W439, R444, N445, K446, Q449, R458, N471, N484. Particular preferred amylases include variants having a deletion of D183 and G184 and having the substitutions R118K, N195F, R320K and R458K, and a variant additionally having substitutions in one or more position selected from the group: M9, G149, G182, G186, M202, T257, Y295, N299, M323, E345 and A339, most preferred a variant that additionally has substitutions in all these positions.

Other examples are amylase variants such as those described in WO2011/098531, WO2013/001078 and WO2013/001087.

Commercially available amylases are Duramyl™, Termamyl™, Fungamyl™, Stainzyme ™, Stainzyme Plus™, Natalase™, Liquozyme X and BAN™ (from Novozymes A/S), and Rapidase™, Purastar™/Effectenz™, Powerase, Preferenz S1000, Preferenz S100 and Preferenz S110 (from Genencor International Inc./DuPont).

A peroxidase according to the invention is a peroxidase enzyme comprised by the enzyme classification EC 1.11.1.7, as set out by the Nomenclature Committee of the International Union of Biochemistry and Molecular Biology (IUBMB), or any fragment derived therefrom, exhibiting peroxidase activity.

Suitable peroxidases include those of plant, bacterial or fungal origin. Chemically modified or protein engineered mutants are included. Examples of useful peroxidases include peroxidases from Coprinopsis, e.g., from C. cinerea (EP 179,486), and variants thereof as those described in WO 93/24618, WO 95/10602, and WO 98/15257.

A suitable peroxidase includes a haloperoxidase enzyme, such as chloroperoxidase, bromoperoxidase and compounds exhibiting chloroperoxidase or bromoperoxidase activity. Haloperoxidases are classified according to their specificity for halide ions. Chloroperoxidases (E.C. 1.11.1.10) catalyze formation of hypochlorite from chloride ions. Preferably, the haloperoxidase is a vanadium haloperoxidase, i.e., a vanadate-containing haloperoxidase. Haloperoxidases have been isolated from many different fungi, in particular from the fungus group dematiaceous hyphomycetes, such as Caldariomyces, e.g., C. fumago, Alternaria, Curvularia, e.g., C. verruculosa and C. inaequalis, Drechslera, Ulocladium and Botrytis.

Haloperoxidases have also been isolated from bacteria such as Pseudomonas, e.g., P. pyrrocinia and Streptomyces, e.g., S. aureofaciens.

A suitable oxidase includes in particular, any laccase enzyme comprised by the enzyme classification EC 1.10.3.2, or any fragment derived therefrom exhibiting laccase activity, or a compound exhibiting a similar activity, such as a catechol oxidase (EC 1.10.3.1), an o-aminophenol oxidase (EC 1.10.3.4), or a bilirubin oxidase (EC 1.3.3.5). Preferred laccase enzymes are enzymes of microbial origin. The enzymes may be derived from plants, bacteria or fungi

(including filamentous fungi and yeasts). Suitable examples from fungi include a laccase derivable from a strain of Aspergillus, Neurospora, e.g., N. crassa, Podospora, Botrytis, Collybia, Fomes, Lentinus, Pleurotus, Trametes, e.g., T. villosa and T. versicolor, Rhizoctonia, e.g., R. solani, Coprinopsis, e.g., C. cinerea, C. comatus, C. friesii, and C. plicatilis, Psathyrella, e.g., P. condelleana, Panaeolus, e.g., P. papilionaceus, Myceliophthora, e.g., M. thermophila, Schytalidium, e.g., S. thermophilum, Polyporus, e.g., P. pinsitus, Phlebia, e.g., P. radiata (WO 92/01046), or Coriolus, e.g., C. hirsutus (JP 2238885). Suitable examples from bacteria include a laccase derivable from a strain of Bacillus. A laccase derived from Coprinopsis or Myceliophthora is preferred; in particular, a laccase derived from Coprinopsis cinerea, as disclosed in WO 97/08325; or from Myceliophthora thermophila, as disclosed in WO 95/33836.

The cleaning composition of the present invention can also contain dispersants. In particular, powdered detergents may comprise dispersants. Suitable water-soluble organic materials include the homo- or co-polymeric acids or their salts, in which the polycarboxylic acid comprises at least two carboxyl radicals separated from each other by not more than two carbon atoms. Suitable dispersants are for example described in Powdered Detergents, Surfactant science series volume 71, Marcel Dekker, Inc.

The cleaning composition of the present invention may also include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof. When present in a subject composition, the dye transfer inhibiting agents may be present at levels from about 0.0001 % to about 10%, from about 0.01% to about 5% or even from about 0.1% to about 3% by weight of the composition.

The cleaning composition of the present invention will preferably also contain additional components that may tint articles being cleaned, such as fluorescent whitening agent or optical brighteners. Where present the brightener is preferably at a level of about 0.01% to about 0.5%. Any fluorescent whitening agent suitable for use in a laundry detergent composition may be used in the composition of the present invention. The most commonly used fluorescent whitening agents are those belonging to the classes of diaminostilbene-sulfonic acid derivatives, diarylpyrazoline derivatives and bisphenyl-distyryl derivatives. Examples of the diaminostilbene-sulfonic acid derivative type of fluorescent whitening agents include the sodium salts of: 4,4'-bis-(2-diethanolamino-4-anilino-s-triazin-6-ylamino) stilbene-2,2'-disulfonate, 4,4'-bis-(2,4-dianilino-s-triazin-6-ylamino) stilbene-2.2'-disulfonate, 4,4'-bis-(2-anilino-4-(N-methyl-N-2-hydroxy-ethylamino)-s-triazin-6-ylamino) stilbene-2,2'-disulfonate, 4,4'-bis-(4-phenyl-1,2,3-triazol-2-yl)stilbene-2,2'-disulfonate and sodium 5-(2H-naphtho[1,2-d][1,2,3]triazol-2-yl)-2-[(E)-2-phenylvinyl]benzenesulfonate. Preferred fluorescent whitening agents are Tinopal DMS and Tinopal CBS available from Ciba-Geigy AG, Basel, Switzerland. Tinopal DMS is the disodium salt of 4,4'-bis-(2-morpholino-4-anilino-s-triazin-6-ylamino) stilbene-2,2'-disulfonate. Tinopal CBS is the disodium salt of 2,2'-bis-(phenyl-styryl)-disulfonate. Also preferred are fluorescent whitening agents is the commercially available Parawhite KX, supplied by Paramount Minerals and Chemicals, Mumbai, India. Other fluorescers suitable for use in the invention include the 1-3-diaryl pyrazolines and the 7-alkylaminocoumarins. Suitable fluorescent brightener levels include lower levels of from about 0.01, from 0.05, from about 0.1 or even from about 0.2 wt % to upper levels of 0.5 or even 0.75 wt%.

The cleaning composition of the present invention may also include one or more soil release polymers which aid the removal of soils from fabrics such as cotton and polyester based fabrics, in particular the removal of hydrophobic soils from polyester based fabrics. The soil release polymers may for example be nonionic or anionic terephthalte based polymers, polyvinyl caprolactam and related copolymers, vinyl graft copolymers, polyester polyamides see for example Chapter 7 in Powdered Detergents, Surfactant science series volume 71, Marcel Dekker, Inc. Another type of soil release polymers is amphiphilic alkoxylated grease cleaning polymers comprising a core structure and a plurality of alkoxylate groups attached to that core structure. The core structure may comprise a polyalkylenimine structure or a polyalkanolamine structure as described in detail in WO 2009/087523 (hereby incorporated by reference). Furthermore, random graft co-polymers are suitable soil release polymers. Suitable graft co-polymers are described in more detail in WO 2007/138054, WO 2006/108856 and WO 2006/113314 (hereby incorporated by reference). Suitable polyethylene glycol polymers include random graft co-polymers comprising: (i) hydrophilic backbone comprising polyethylene glycol; and (ii) side chain(s) selected from the group consisting of: C4-C25 alkyl group, polypropylene, polybutylene, vinyl ester of a saturated C1-C6 mono-carboxylic acid, C1-C6 alkyl ester of acrylic or methacrylic acid, and mixtures thereof. Suitable polyethylene glycol polymers have a polyethylene glycol backbone with random grafted polyvinyl acetate side chains. The average molecular weight of the polyethylene glycol backbone can be in the range of from 2,000 Da to 20,000 Da, or from 4,000 Da to 8,000 Da. The molecular weight ratio of the polyethylene glycol backbone to the polyvinyl acetate side chains can be in the range of from 1: 1 to 1:5, or from 1: 1.2 to 1:2. The average number of graft sites per ethylene oxide units can be less than 1, or less than 0.8, the average number of graft sites per ethylene oxide units can be in the range of from 0.5 to 0.9, or the average number of graft sites per ethylene oxide units can be in the range of from 0.1 to 0.5, or from 0.2 to 0.4. A suitable polyethylene glycol polymer is Sokalan HP22. Other soil release polymers are substituted polysaccharide structures especially substituted cellulosic structures such as modified cellulose deriviatives such as those described in EP 1867808 or WO 2003/040279 (both are hereby incorporated by reference). Suitable

cellulosic polymers include cellulose, cellulose ethers, cellulose esters, cellulose amides and mixtures thereof. Suitable cellulosic polymers include anionically modified cellulose, nonionically modified cellulose, cationically modified cellulose, zwitterionically modified cellulose, and mixtures thereof. Suitable cellulosic polymers include methyl cellulose, carboxy methyl cellulose, ethyl cellulose, hydroxyl ethyl cellulose, hydroxyl propyl methyl cellulose, ester carboxy methyl cellulose, and mixtures thereof.

The cleaning composition of the present invention may also include one or more anti-redeposition agents such as carboxymethylcellulose (CMC), polyvinyl alcohol (PVA), polyvinylpyrrolidone (PVP), polyoxyethylene and/or polyethyleneglycol (PEG), homopolymers of acrylic acid, copolymers of acrylic acid and maleic acid, and ethoxylated polyethyleneimines. The cellulose based polymers described under soil release polymers above may also function as anti-redeposition agents.

The cleaning composition of the present invention may also include one or more rheology modifiers, structurants or thickeners, as distinct from viscosity reducing agents. The rheology modifiers are selected from the group consisting of non-polymeric crystalline, hydroxy-functional materials, polymeric rheology modifiers which impart shear thinning characteristics to the aqueous liquid matrix of a liquid detergent composition. The rheology and viscosity of the detergent can be modified and adjusted by methods known in the art, for example as shown in EP 2169040.

Other suitable cleaning composition components include, but are not limited to, anti-shrink agents, anti-wrinkling agents, bactericides, binders, carriers, dyes, enzyme stabilizers, fabric softeners, fillers, foam regulators, hydrotropes, perfumes, pigments, sud suppressors, solvents, bittering agents, and structurants for liquid detergents and/or structure elasticizing agents.

In various embodiments, the invention relates to cleaning compositions which comprise the polypeptides having hexosaminidase activity, as described herein, and any one or more of an adjunct ingredient selected from bittering agents and organic solvents, such as glycerol and 1,2-propane diol.

**Formulation of detergent products**

The cleaning composition of the invention may be in any convenient form, e.g., a bar, a homogenous tablet, a tablet having two or more layers, a pouch having one or more compartments, such as 2 or more, preferably 2, 3, 4 or 5 compartments, a regular or compact powder, a granule, a paste, a gel, or a regular, compact or concentrated liquid.

Pouches can be configured as single or multicompartments. It can be of any form, shape and material which is suitable for hold the composition, e.g. without allowing the release of the composition to release of the composition from the pouch prior to water contact. The pouch is made from water soluble film which encloses an inner volume. Said inner volume can be divided into compartments of the pouch. Preferred films are polymeric materials preferably polymers which are formed into a film or sheet. Preferred polymers, copolymers or derivates thereof are selected polyacrylates, and water soluble acrylate copolymers, methyl cellulose, carboxy methyl cellulose, sodium dextrin, ethyl cellulose, hydroxyethyl cellulose, hydroxypropyl methyl cellulose, malto dextrin, poly methacrylates, most preferably polyvinyl alcohol copolymers and, hydroxypropyl methyl cellulose (HPMC). Preferably the level of polymer in the film for example PVA is at least about 60%. Preferred average molecular weight will typically be about 20,000 to about 150,000. Films can also be of blended compositions comprising hydrolytically degradable and water soluble polymer blends such as polylactide and polyvinyl alcohol (known under the Trade reference M8630 as sold by MonoSol LLC, Indiana, USA) plus plasticisers like glycerol, ethylene glycerol, propylene glycol, sorbitol and mixtures thereof. The pouches can comprise a solid laundry cleaning composition or part components and/or a liquid cleaning composition or part components separated by the water soluble film. The compartment for liquid components can be different in composition than compartments containing solids: US2009/0011970 A1.

Detergent ingredients can be separated physically from each other by compartments in water dissolvable pouches or in different layers of tablets. Thereby negative storage interaction between components can be avoided. Different dissolution profiles of each of the compartments can also give rise to delayed dissolution of selected components in the wash solution.

A liquid or gel detergent, which is not unit dosed, may be aqueous, typically containing at least 20% by weight and up to 95% water, such as up to about 70% water, up to about 65% water, up to about 55% water, up to about 45% water, up to about 35% water. Other types of liquids, including without limitation, alkanols, amines, diols, ethers and polyols may be included in an aqueous liquid or gel. An aqueous liquid or gel detergent may contain from 0-30% organic solvent. A liquid or gel detergent may be non-aqueous.

**Uses**

The present invention is also directed to methods for using a composition comprising a hexosaminidase e.g. a *Lactobacillus or Streptococcus* hexosaminidase . A *hexosaminidase* as described herein is useful in cleaning processes typically in laundry/textile/fabric (House hold laundry washing, Industrial laundry washing) or hard surface cleaning (ADW,

car wash, Industrial surface).

One aspect of the invention relates to the use of a composition, as defined herein, comprising a hexosaminidase e.g. *Lactobacillus or Streptococcus* hexosaminidase for cleaning of an item, wherein the item is a textile or a surface.

One aspect of the invention relates to the use of a composition, as defined herein, comprising a *Lactobacillus* or *Streptococcus* hexosaminidase for cleaning of an item, wherein the item is a textile or a surface, wherein the *hexosaminidase* is selected from the group consisting of polypeptides shown in SEQ ID NO 3, SEQ ID NO 6, SEQ ID NO 9, SEQ ID NO 18 and polypeptides having at least 60%, at least 70%, at least 75%, at least 80%, at least 85%, at least 90%, at least 95%, at least 98% or such as at least 99% sequence identity hereto.

One aspect of the invention relates to the use of a composition, as defined herein, comprising a *Lactobacillus* hexosaminidase of the invention,

a) for preventing, reducing or removing stickiness of the item;
b) for pretreating stains on the item;
c) for preventing, reducing or removing redeposition of soil during a wash cycle;
d) for preventing, reducing or removing adherence of soil to the item;
e) for maintaining or improving whiteness of the item;
f) for preventing, reducing or removal malodor from the item,
wherein the item is a textile.

One aspect of the invention relates to the use of a composition, as defined herein, comprising a *Streptococcus* hexosaminidase of the invention,

a) for preventing, reducing or removing stickiness of the item;
b) for pretreating stains on the item;
c) for preventing, reducing or removing redeposition of soil during a wash cycle;
d) for preventing, reducing or removing adherence of soil to the item;
e) for maintaining or improving whiteness of the item;
f) for preventing, reducing or removal malodor from the item,
wherein the item is a textile.

One aspect of the invention relates to the use of a composition, as defined herein, comprising a hexosaminidase e.g. dispersin, wherein the hexosaminidase is selected from the group consisting of polypeptides shown in SEQ ID NO 3, SEQ ID NO 6, SEQ ID NO 9, SEQ ID NO 18 ans polypeptides having at least 60%, at least 70%, at least 75%, at least 80%, at least 85%, at least 90%, at least 95%, at least 98% or such as at least 99% sequence identity hereto,

a) for preventing, reducing or removing stickiness of the item;
b) for pretreating stains on the item;
c) for preventing, reducing or removing redeposition of soil during a wash cycle;
d) for preventing, reducing or removing adherence of soil to the item;
e) for maintaining or improving whiteness of the item;
f) for preventing, reducing or removal malodor from the item,
wherein the item is a textile.

The detergent composition of the present invention may be formulated, for example, as a hand or machine laundry detergent composition including a laundry additive composition suitable for pre-treatment of stained fabrics and a rinse added fabric softener composition, or be formulated as a detergent composition for use in general household hard surface cleaning operations, or be formulated for hand or machine dishwashing operations. In a specific aspect, the present invention provides a detergent additive comprising one or more enzymes as described herein.

One aspect of the invention relates to the use of a composition, as defined herein, preferably a a detergent composition comprising a hexosaminidase e.g. obtained from *Lactobacillus* or *Streptococcus* for cleaning of an item, wherein the item is a textile or a surface.

One aspect of the invention relates to the use of a composition, as defined herein, preferably a detergent composition comprising a hexosaminidase e.g. obtained from *Lactobacillus* or *Streptococcus* for cleaning of an item, wherein the item is a textile or a surface, wherein the hexosaminidase e.g. obtained from *Lactobacillus* or *Streptococcus* is selected from the group consisting of polypeptides shown in SEQ ID NO 3, SEQ ID NO 6, SEQ ID NO 9, SEQ ID NO 18 and polypeptides having at least 60%, at least 70%, at least 75%, at least 80%, at least 85%, at least 90%, at least 95%, at least 98% or such as at least 99% sequence identity hereto.

One aspect of the invention relates to the use of a composition, as defined herein, preferably a detergent

composition comprising a hexosaminidase e.g. obtained from *Lactobacillus* or *Streptococcus* of the invention,

a) for preventing, reducing or removing stickiness of the item;
b) for pretreating stains on the item;
c) for preventing, reducing or removing redeposition of soil during a wash cycle;
d) for preventing, reducing or removing adherence of soil to the item;
e) for maintaining or improving whiteness of the item;
f) for preventing, reducing or removal malodor from the item,
wherein the item is a textile.

One aspect of the invention relates to the use of a composition, as defined herein, preferably a detergent composition comprising a hexosaminidase e.g. obtained from *Lactobacillus* or *Streptococcus,* wherein the hexosaminidase is selected from the group consisting of polypeptides shown in SEQ ID NO 3, SEQ ID NO 6, SEQ ID NO 9, SEQ ID NO 18 and polypeptides having at least 60%, at least 70%, at least 75%, at least 80%, at least 85%, at least 90%, at least 95%, at least 98% or such as at least 99% sequence identity hereto,

a) for preventing, reducing or removing stickiness of the item;
b) for pretreating stains on the item;
c) for preventing, reducing or removing redeposition of soil during a wash cycle;
d) for preventing, reducing or removing adherence of soil to the item;
e) for maintaining or improving whiteness of the item;
f) for preventing, reducing or removal malodor from the item,
wherein the item is a textile.

**Methods**

The invention further relates to a method of treating a method of treating a fabric comprising;

(a) contacting the fabric with a composition of the invention or an aqueous solution thereof;
(b) and optionally rinsing and drying the textile.

One aspect relates to a method of treating a fabric comprising;

(a) contacting the fabric with a composition of the invention or an aqueous solution thereof, wherein the hexosaminidase e.g. obtained from *Lactobacillus* or *Streptococcus* is selected from the group consisting of polypeptides shown in SEQ ID NO 3, SEQ ID NO 6, SEQ ID NO 9, SEQ ID NO 18 and polypeptides having at least 60%, at least 70%, at least 75%, at least 80%, at least 85%, at least 90%, at least 95%, at least 98% or such as at least 99% sequence identity hereto;
(b) and optionally rinsing and drying the textile.

The invention further relates to a method for cleaning or laundering an item comprising the steps of:

a. exposing an item to a composition of the invention or a wash liquor comprising a composition of the invention,;
b. completing at least one wash cycle; and
c. optionally rinsing the item,

wherein the item is a fabric.
The invention further relates to a method for cleaning or laundering an item comprising the steps of:

a. exposing an item to a composition of the invention or a wash liquor comprising a composition of the invention, said composition comprising a hexosaminidase e.g. obtained from *Lactobacillus* or *Streptococcus,* wherein the *hexosaminidase* is selected from the group consisting of polypeptides shown in SEQ ID NO 3, SEQ ID NO 6, SEQ ID NO 9, SEQ ID NO 18 and polypeptides having at least 60%, at least 70%, at least 75%, at least 80%, at least 85%, at least 90%, at least 95%, at least 98% or such as at least 99% sequence identity hereto;
b. completing at least one wash cycle; and
c. optionally rinsing the item, wherein the item is a fabric.

One embodiment relates to a method, wherein the *Lactobacillus* hexosaminidase comprises one or more of

the following motifs GXDE (SEQ ID NO 12), [EQ][NRSHA][YVFL][AGSTC][IVLF][EAQYN][SN] (SEQ ID NO 13), [VLIM][LIV]G[GAV]DE[VI][PSA] (SEQ ID NO 14), or [GK]A[IL][IL][KSR][LQ]L (SEQ ID NO 15).

One embodiment relates to a method, wherein the *Lactobacillus* hexosaminidase comprises the motifs GXDE (SEQ ID NO 12) and [EQ][NRSHA][YVFL][AGSTC][IVLF][EAQYN][SN] (SEQ ID NO 13).

One embodiment relates to a method, wherein the *Lactobacillus* hexosaminidase comprises the motifs [VLIM][LIV]G[GAV]DE[VI][PSA] (SEQ ID NO 14) and/or [GK]A[IL][IL][KSR][LQ]L (SEQ ID NO 15).

The pH of the aqueous/liquid solution or wash liquor may be in the range of 1 to 11, such as in the range 5.5 to 11, such as in the range of 7 to 9, in the range of 7 to 8 or in the range of 7 to 8.5.

The wash liquor may have a temperature in the range of 5°C to 95°C, or in the range of 10°C to 80°C, in the range of 10°C to 70°C, in the range of 10°C to 60°C, in the range of 10°C to 50°C, in the range of 15°C to 40°C or in the range of 20°C to 30°C. In one aspect, the temperature of the wash liquor is 30°C.

The concentration of the hexosaminidase in the wash liquor is typically in the range of at least 0.00001 ppm to at least 10 ppm, at least 0.00002 ppm to at least 10 ppm, at least 0.0001 ppm to at least 10 ppm, at least 0.0002 ppm to at least 10 ppm, at least 0.001 ppm to at least 10 ppm, at least 0.002 ppm to at least 10 ppm, at least 0.01 ppm to at least 10 ppm, at least 0.02 ppm to at least 10 ppm, at least 0.1 ppm to at least 10 ppm, at least 0.2 ppm to at least 10 ppm, at least 0.5 ppm to at least 5 ppm.

**Definitions**

Biofilm is produced by any group of microorganisms in which cells stick to each other or stick to a surface, such as a textile, dishware or hard surface or another kind of surface. These adherent cells are frequently embedded within a self-produced matrix of extracellular polymeric substance (EPS). Biofilm EPS is a polymeric conglomeration generally composed of extracellular DNA, proteins, and polysaccharides. Biofilms may form on living or non-living surfaces. The microbial cells growing in a biofilm are physiologically distinct from planktonic cells of the same organism, which, by contrast, are single-cells that may float or swim in a liquid medium. Bacteria living in a biofilm usually have significantly different properties from planktonic bacteria of the same species, as the dense and protected environment of the film allows them to cooperate and interact in various ways. One benefit of this environment for the microorganisms is increased resistance to detergents and antibiotics, as the dense extracellular matrix and the outer layer of cells protect the interior of the community. On laundry biofilm producing bacteria can be found among the following species: *Acinetobacter sp., Aeromicrobium sp., Brevundimonas sp., Microbacterium sp., Micrococcus luteus, Pseudomonas sp., Staphylococcus epidermidis,* and *Stenotrophomonas* sp. On hard surfaces biofilm producing bacteria can be found among the following species: *Acinetobacter sp., Aeromicrobium sp., Brevundimonas sp., Microbacterium sp., Micrococcus luteus, Pseudomonas sp., Staphylococcus epidermidis, Staphylococcus aureus* and *Stenotrophomonas sp.* In one aspect, the biofilm producing strain is *Brevundimonas* sp. In one aspect, the biofilm producing strain is *Pseudomonas, e.g. Pseudomonas alcaliphila* or *Pseudomonas fluorescens.* In one aspect, the biofilm producing strain is *Staphylococcus aureus.*

By the term "deep cleaning" is meant disruption or removal of components of organic matter, e.g. biofilm, such as polysaccharides, e.g. PNAG, proteins, DNA, soil or other components present in the organic matter.

Cleaning component: The cleaning component is different to the hexosaminidase. The precise nature of these additional components (e.g. adjuncts), and levels of incorporation thereof, will depend on the physical form of the composition and the nature of the operation for which it is to be used. Suitable cleaning component materials include, but are not limited to the components described below such as surfactants, builders, flocculating aid, chelating agents, dye transfer inhibitors, enzymes, enzyme stabilizers, enzyme inhibitors, catalytic materials, bleach activators, hydrogen peroxide, sources of hydrogen peroxide, preformed peracids, polymeric agents, clay soil removal/anti-redeposition agents, brighteners, suds suppressors, dyes, perfumes, structure elasticizing agents, fabric softeners, carriers, hydrotropes, builders and co-builders, fabric huing agents, anti-foaming agents, dispersants, processing aids, and/or pigments.

Cleaning Composition: The term "cleaning or detergent composition" refers to compositions that find use in the removal of undesired compounds from items to be cleaned, such as textiles. The cleaning composition may be used to e.g. clean textiles for both household cleaning and industrial cleaning. The terms encompass any materials/compounds selected for the particular type of cleaning composition desired and the form of the product (e.g., liquid, gel, powder, granulate, paste, or spray compositions) and includes, but is not limited to, detergent compositions (e.g., liquid and/or solid laundry detergents and fine fabric detergents; fabric fresheners; fabric softeners; and textile and laundry prespotters/pretreatment). In addition to containing the enzymes described herein, the detergent formulation may contain one or more additional enzymes (such as proteases, amylases, lipases, cutinases, cellulases, endoglucanases, xyloglucanases, pectinases, pectin lyases, xanthanases, peroxidases, haloperoxygenases, catalases and mannanases, or any mixture thereof), and/or detergent adjunct ingredients such as surfactants, builders, chelators or chelating agents, bleach system or bleach components, polymers, fabric conditioners, foam boosters, suds suppressors, dyes, perfume, tannish inhibitors, optical brighteners, bactericides, fungicides, soil suspending agents, anti-corrosion agents, enzyme inhibitors or stabilizers, enzyme activators, transferase(s), hydrolytic enzymes, oxido reductases, bluing agents and fluorescent

dyes, antioxidants, and solubilizers.

The term "hard surface cleaning" is defined herein as cleaning of hard surfaces wherein hard surfaces may include floors, tables, walls, roofs etc. as well as surfaces of hard objects such as cars (car wash) and dishes (dish wash). Dish washing includes but are not limited to cleaning of plates, cups, glasses, bowls, cutlery such as spoons, knives, forks, serving utensils, ceramics, plastics, metals, china, glass and acrylics.

The term "wash performance" is used as an enzyme's ability to remove stains or soil present on the object to be cleaned during e.g. wash or hard surface cleaning.

The term "whiteness" is defined herein as the quality or state of a textile of being white. Loss of whiteness may be due to removal of optical brighteners/hueing agents and result in a greying or yellowing of the textiles. Greying and yellowing can be due to soil redeposition, body soils, colouring from e.g. iron and copper ions or dye transfer. Whiteness might include one or several issues from the list below: colourant or dye effects; incomplete stain removal (e.g. body soils, sebum etc.); redeposition (greying, yellowing or other discolourations of the object) (removed soils reassociate with other parts of textile, soiled or unsoiled); chemical changes in textile during application; and clarification or brightening of colours.

The term "laundering" relates to both household laundering and industrial laundering and means the process of treating textiles with a solution containing a cleaning or detergent composition of the present invention. The laundering process can for example be carried out using e.g. a household or an industrial washing machine or can be carried out by hand.

By the term "malodor" is meant an odor which is not desired on clean items. The cleaned item should smell fresh and clean without malodors adhered to the item. One example of malodor is compounds with an unpleasant smell, which may be produced by microorganisms. Another example of unpleasant smell can be sweat or body odor adhered to an item which has been in contact with human or animal. Another example of malodor can be the odor from spices, which sticks to items for example curry or other exotic spices which smell strongly, tobacco, cooking smell (fried oil, fish etc.), scents of perfume such as deodorant and eau de cologne.

The term "mature polypeptide" means a polypeptide in its final form following translation and any post-translational modifications, such as N-terminus processing, C-terminus truncation, glycosylation, phosphorylation, etc.

The term "textile" means any textile material including yarns, yarn intermediates, fibers, non-woven materials, natural materials, synthetic materials, and any other textile material, fabrics made of these materials and products made from fabrics (e.g., garments and other articles). The textile or fabric may be in the form of knits, wovens, denims, non-wovens, felts, yarns, and towelling. The textile may be cellulose based such as natural cellulosics, including cotton, flax/linen, jute, ramie, sisal or coir or manmade cellulosics (e.g. originating from wood pulp) including viscose/rayon, cellulose acetate fibers (tricell), lyocell or blends thereof. The textile or fabric may also be non-cellulose based such as natural polyamides including wool, camel, cashmere, mohair, rabbit and silk or synthetic polymers such as nylon, aramid, polyester, acrylic, polypropylene and spandex/elastane, or blends thereof as well as blends of cellulose based and non-cellulose based fibers. Examples of blends are blends of cotton and/or rayon/viscose with one or more companion material such as wool, synthetic fiber (e.g. polyamide fiber, acrylic fiber, polyester fiber, polyvinyl chloride fiber, polyurethane fiber, polyurea fiber, aramid fiber), and/or cellulose-containing fiber (e.g. rayon/viscose, ramie, flax/linen, jute, cellulose acetate fiber, lyocell). Fabric may be conventional washable laundry, for example stained household laundry. When the term fabric or garment is used it is intended to include the broader term textiles as well.

The term "variant" means a polypeptide having the activity of the parent or precursor polypeptide and comprising an alteration, i.e., a substitution, insertion, and/or deletion, at one or more (e.g., several) positions compared to the precursor or parent polypeptide. A substitution means replacement of the amino acid occupying a position with a different amino acid; a deletion means removal of the amino acid occupying a position; and an insertion means adding an amino acid adjacent to and immediately following the amino acid occupying a position.

Sequence identity: The relatedness between two amino acid sequences or between two nucleotide sequences is described by the parameter "sequence identity". For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 6.6.0 or later. The parameters used are a gap open penalty of 10, a gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

(Identical Residues x 100)/(Length of Alignment - Total Number of Gaps in Alignment).

**Clade:** a group of polypeptides clustered together based on homologous features traced to a common ancestor. Polypeptide clades can be visualized as phylogenetic trees and a clade is a group of polypeptides that consists of a common ancestor and all its lineal descendants

**Nomenclature**

For purposes of the present invention, the nomenclature [IV] or [I/V] means that the amino acid at this position may be isoleucine (Ile, I) or valine (Val, V). Likewise, the nomenclature [LVI] and [L/V/I] means that the amino acid at this position may be a leucine (Leu, L), valine (Val, V) or isoleucine (Ile, I), and so forth for other combinations as described herein. Unless otherwise limited further, the amino acid X is defined such that it may be any of the 20 natural amino acids.
The invention is further described in the following nonlimiting paragraphs

1. A cleaning composition comprising at least 0.01 mg *Lactobacillus hexosaminidase* wherein the cleaning composition

(a) is a solid, preferably granular, laundry detergent composition and further comprises

(a1) at least one zeolite builder, preferably in an amount of 10 to 50 wt.-%, more preferably 20-30 wt.- %;
(a2) at least one phosphonate builder, preferably in an amount of 0.1 to 5 wt.-%, more preferably 0.4 to 1.5 wt.-%;
(a3) at least one further enzyme, preferably a cellulase, preferably in an amount of active enzyme of 100 to 5000 ppb, more preferably 1000 to 2000 ppb; and
(a4) at least one polymer, preferably a polyvinylpyrrolidon polymer, preferably in an amount of 0.01 to 1 wt.-%, more preferably 0.1 to 0.3 wt.-%; or

(b) is a solid laundry detergent composition and further comprises

(b1) at least one silicate builder, preferably in an amount of 2 to 20 wt.-%, more preferably 5-10 wt.-%; (b2) optionally carboxymethylcellulose, preferably in an amount of 0.1 to 10 wt.-%, more preferably 0.1 to 4 wt.-%;
(b3) at least one further enzyme, preferably a cellulase, preferably in an amount of active enzyme of 0.1 to 100 ppm, more preferably 0.1 to 10 ppm;
(b4) optionally at least one soil release polymer, preferably a polyvinylpyrrolidon polymer, in an amount of 0.1 to 3 wt.-%, more preferably 0.1 to 1.0 wt.-%; and
(b5) at least one bleaching system, comprising a bleaching agent, a bleach activator and a bleach catalyst, preferably in an amount of 0.1 to 50 wt.-%, more preferably 0.1 to 30 wt.-%; or

(c) is liquid laundry detergent composition and further comprises

(c1) at least one surfactant, preferably nonionic surfactant, preferably in an amount of 1 to 20 wt.-%, preferably 3 to 15 wt.-%;
(c2) optionally at least one phosphonate builder, preferably in an amount of 0.1 to 3 wt.-%, more preferably 0.25 to 1.5 wt.-%
(c3) optionally at least at least one further enzyme, preferably a cellulase, preferably in an amount of enzyme composition of 0.001 to 1 wt.-%, more preferably 0.001 to 0.6 wt.-%; and
(c4) optionally at least one organic solvent, preferably glycerol, preferably in an amount of 0.1 to 10 wt.-%, more preferably 0.1 to 5 wt.-%; or

(d) is a liquid laundry detergent in unit dose form, preferably a pouch comprising a water-soluble film, and further comprises

(d1) water in an amount of up to 20 wt.-%, preferably 5 to 15 wt.-%;
(d2) optionally at least one bittering agent, preferably Benzyldiethyl(2,6-xylylcarbamoyl)-methylammoniumbenzoate, preferably in an amount of 0.00001 to 0.04 wt.-%;
(d3) optionally at least one optical brightener, preferably in an amount of 0.01 to 2 wt.-%, more preferably 0.01 to 1 wt.-%; and
(d4) optionally at least one polymer, preferably in an amount of 0.01 to 7 wt.-%, more preferably 0.1 to 5 wt.-%; or

(e) is a fabric finisher and further comprises

(e1) at least one softening silicone, preferably an amino-functionalized silicone, preferably in an amount of 0.1 to 10 wt.-%, more preferably 0.1 to 2 wt.-%;

(e2) at least one perfume, preferably at least partially encapsulated in microcapsules, more preferably at least partially encapsulated in melamine-formaldehyde microcapsules, preferably in an amount of 0.01 to 3 wt.-%, more preferably 0.1 to 1 wt.-%;
(e3) optionally polyquaternium 10 in an amount of 0.1 to 20 wt.-%, preferably 0.1 to 13 wt.-%;
(e4) optionally polyquaternium 37 in an amount of 0.1 to 20 wt.-%, preferably 0.1 to 13 wt.-%;
(e5) optionally a plant-based esterquat, preferably a canola- or palm-based esterquat, in an amount of 0.1 to 20 wt.-%, preferably 0.1 to 13 wt.-%; and
(e6) optionally adipic acid, in an amount of 0.1 to 20 wt.-%, preferably 0.1 to 13 wt.-%; or

(f) is an acidic cleaning agent, preferably having a pH less than 6, and further comprises

(f1) plant-based or bio-based surfactants, preferably each in an amount of 0.1 to 5, more preferably each in an amount of 0.1 to 2 wt.-%;
(f2) at least one acidic biocide, preferably selected from acids, more preferably HCl and formic acid; and
(f3) at least one soil release, water repellant or water spreading polymer, preferably in an amount of 0.01 to 3 wt.-%, more preferably 0.01 to 0.5 wt.-%; or

(g) is a neutral cleaning agent, preferably having a pH between 6.0 and 7.5, and further comprises

(g1) plant-based or bio-based surfactants, preferably each in an amount of 0.1 to 5, more preferably each in an amount of 0.1 to 2 wt.-%;
(g2) at least one biocide, preferably selected from quaternary ammonium compounds and alcohols; and
(g3) at least one soil release, water repellant or water spreading polymer, preferably in an amount of 0.01 to 3 wt.-%, more preferably 0.01 to 0.5 wt.-%; or

(h) is an alkaline cleaning agent, preferably having a pH of more than 7.5, and further comprises

(h1) plant-based or bio-based surfactants, preferably each in an amount of 0.1 to 5, more preferably each in an amount of 0.1 to 2 wt.-%; or

(i) is a hand dishwashing agent, preferably liquid hand dishwashing agent, and further comprises

(i1) at least one anionic surfactant, preferably in an amount of 0.1 to 40 wt.-%, more preferably 5 to 30 wt.-%;
(i2) at least one amphoteric surfactant, preferably betain, preferably in an amount of 0.1 to 25 wt.-%, more preferably 1 to 15 wt.-%;
(i3) at least one nonionic surfactant, preferably in an amount of 0.1 to 25 wt.-%, more preferably 2 to 10 wt.-%;
(i4) at least one further enzyme, preferably selected from proteases, amylases and combinations thereof, preferably in an amount of enzyme composition of up to 1 wt.-%, more preferably up to 0.6 wt.-%; or

(j) is an automatic dishwashing composition and further comprises

(j1) at least one builder selected from citrate, aminocarboxylates and combinations thereof, preferably in an amount of 5 to 30 wt.-%, more preferably 10 to 20 wt.-%;
(j2) at least one phosphonate builder, preferably in an amount of 0.1 to 5 wt.-%, more preferably 0.4 to 1.5 wt.-%;
(j3) at least one nonionic surfactant, preferably in an amount of 0.1 to 10 wt.-%, more preferably 1 to 5 wt.-%;
(j4) at least one bleaching system, comprising a bleaching agent, a bleach activator and a bleach catalyst, preferably in an amount of 0.1 to 50 wt.-%, more preferably 0.1 to 30 wt.-%; and
(j5) at least one polymer selected from sulfopolymers, cationic polymers and polyacrylates, preferably in an amount of 0.01 to 15 wt.-%, more preferably 2 to 10 wt.-%; or

(k) further comprises

(k1) at least one sulfopolymer, preferably in an amount of 1 to 15, more preferably 2 to 10 wt.-% and is preferably a dishwashing, more preferably an automatic dishwashing composition; or

(l) further comprises at least one adjunct ingredient selected from probiotics, preferably microbes, spores or combinations thereof; or

(m) is in unit dose form and comprises at least 2, preferably 2, 3, 4 or 5 separate compartments; or
(n) is a phosphate-free composition.

wherein the composition optionally comprises an additional cleaning component, wherein the additional cleaning component is

(a) at least one surfactant;
(b) at least one builder; or
(c) at least one polymer

2. The composition according to paragraph 1, wherein the composition comprises from about 1% to about 40% by weight of an anionic surfactant, such as from about 5% to about 30%, including from about 5% to about 15%, or from about 15% to about 20%, or from about 20% to about 25% anionic surfactant, preferably selected from linear alkylbenzenesulfonates (LAS), isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ethersulfates (AES or AEOS or FES), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or salt of fatty acids (soap), and combinations thereof.

3. The composition according to paragraph 1 or 2 comprising from about 0.2% to about 40% by weight of a nonionic surfactant, for example from about 0.5% to about 30%, in particular from about 1% to about 20%, from about 3% to about 10%, such as from about 3% to about 5%, from about 8% to about 12%, or from about 10% to about 12% of at least one nonionic surfactant, preferably selected from alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxyalkyl fatty acid amides, or N-acyl N-alkyl derivatives of glucosamine (glucamides, GA, or fatty acid glucamides, FAGA) and combinations thereof.

4. The composition according to any of paragraphs 1 to 3, wherein the composition comprises from about 1 wt% to about 60 wt %, from about 5 wt% to about 50 wt %, from about 10 wt% to about 40 wt% of at least one builder, preferably selected from citric acid, methylglycine-N,N-diacetic acid (MGDA) and/or glutamic acid-N,N-diacetic acid (GLDA) and mixtures thereof.

5. The composition according to any of paragraphs 1 to 4, wherein the composition 0-50% by weight, such as 1-40%, such as 1-30%, such as about 1% to about 20 % of at least one bleach component preferably selected from a peroxide, preferably percabonate and a catalyst preferably a metal-containing bleach catalyst such as 1,4,7-trimethyl-1,4,7-triazacyclononane or manganese (II) acetate tetrahydrate (MnTACN).

6. The composition according to any of the preceding paragraphs, wherein the *Lactobacillus hexosaminidase* comprises the one or more of the motifs GXDE (SEQ ID NO 12), [EQ][NRSHA][YVFL][AGSTC][IVLF][EAQYN][SN] (SEQ ID NO 13), [VLIM][LIV]G[GAV]DE[VI][PSA] (SEQ ID NO 14), or [GK]A[IL][IL][KSR][LQ]L (SEQ ID NO 15).

7. The composition according to any of the preceding paragraphs, wherein the *Lactobacillus hexosaminidase* comprises the motifs GXDE (SEQ ID NO 12) and [EQ][NRSHA][YVFL][AGSTC][IVLF][EAQYN][SN] (SEQ ID NO 13).

8. The composition according to paragraph 7, wherein the *Lactobacillus hexosaminidase* comprises the motifs [VLIM][LIV]G[GAV]DE[VI][PSA] (SEQ ID NO 14) and/or [GK]A[IL][IL][KSR][LQ]L (SEQ ID NO 15).

9. The composition according to any of the preceding paragraphs, wherein the *Lactobacillus hexosaminidase* is selected from the group consisting of polypeptides having the amino acid sequence of SEQ ID NO 3, SEQ ID NO 6, SEQ ID NO 9, and polypeptides having at least 60%, at least 70%, at least 75%, at least 80%, at least 85%, at least 90%, at least 95%, at least 98% or such as at least 99% sequence identity hereto.

10. The composition according to any of the preceding paragraphs, wherein the polypeptide having hexosaminidase activity comprises the amino acid sequence of SEQ ID NO 3 or polypeptides having at least 60 % e.g. 80%, 85%, 90%, 95%, 98% or 99% sequence identity hereto.

11. The composition according to any of the preceding paragraphs, wherein the polypeptide having hexosaminidase activity comprises the amino acid sequence of SEQ ID NO 6 or polypeptides having at least 60 % e.g. 80%, 85%, 90%, 95%, 98% or 99% sequence identity hereto.

12. The composition according to any of the preceding paragraphs, wherein the polypeptide having hexosaminidase activity comprises the amino acid sequence of SEQ ID NO 9 or polypeptides having at least 60 % e.g. 80%, 85%, 90%, 95%, 98% or 99% sequence identity hereto.

13. The composition according to any of the preceding paragraphs, wherein the composition further comprises one or more enzymes selected from the group consisting of proteases, lipases, cutinases, amylases, carbohydrases, cellulases, pectinases, mannanases, arabinases, galactanases, xylanases and oxidases.

14. Use of a composition according to any of the previous paragraphs for cleaning of an item, wherein the item is a textile or a surface.

15. Use of a composition according to paragraph 14, preferably a detergent composition comprising a *Lactobacillus* hexosaminidase,

a) for preventing, reducing or removing stickiness of the item;
b) for pretreating stains on the item;
c) for preventing, reducing or removing redeposition of soil during a wash cycle;
d) for preventing, reducing or removing adherence of soil to the item;
e) for maintaining or improving whiteness of the item;
f) for preventing, reducing or removing malodor from the item,
wherein the item is a textile.

16. Use according to paragraph 14 or 15, wherein the *Lactobacillus hexosaminidase* comprises one or more of the following motifs GXDE (SEQ ID NO 12), [EQ][NRSHA][YVFL][AGSTC][IVLF][EAQYN][SN] (SEQ ID NO 13), [VLIM][LIV]G[GAV]DE[VI][PSA] (SEQ ID NO 14), or [GK]A[IL][IL][KSR][LQ]L (SEQ ID NO 15).

17. Use according to any of paragraphs 14 to 16, wherein the *Lactobacillus hexosaminidase* comprises the motifs GXDE (SEQ ID NO 12) and [EQ][NRSHA][YVFL][AGSTC][IVLF][EAQYN][SN] (SEQ ID NO 13).

18. Use according to paragraphs 14 to 16, wherein the *Lactobacillus hexosaminidase* comprises the motifs [VLIM][LIV]G[GAV]DE[VI][PSA] (SEQ ID NO 14) and/or [GK]A[IL][IL][KSR][LQ]L (SEQ ID NO 15).

19. Use of a composition according to any of paragraphs 14 to 18, wherein the *Lactobacillus hexosaminidase* is selected from the group consisting of polypeptides shown in SEQ ID NO 3, SEQ ID NO 6, SEQ ID NO 9, or polypeptides having at least 60%, at least 70%, at least 75%, at least 80%, at least 85%, at least 90%, at least 95%, at least 98% or such as at least 99% sequence identity hereto.

20. A method of formulating a cleaning composition according to paragraphs 1-13 comprising adding a *Lactobacillus hexosaminidase* and at least one cleaning component.

21. A method of treating a fabric comprising;

(a) contacting the fabric with a composition of any one of paragraphs 1-13 or an aqueous solution thereof;
(b) and optionally rinsing and drying the textile.

22. A method for cleaning or laundering an item comprising the steps of:

(a) exposing an item to a composition of any one of paragraphs 1-13 or a wash liquor comprising such a composition;
(b) completing at least one wash cycle; and

(c) optionally rinsing the item, wherein the item is a fabric.

23. The method according to paragraph 22 or 23, wherein the *Lactobacillus hexosaminidase* comprises one or more of the following motifs GXDE (SEQ ID NO 12), [EQ][NRSHA][YVFL][AGSTC][IVLF][EAQYN][SN] (SEQ ID NO 13), [VLIM][LIV]G[GAV]DE[VI][PSA] (SEQ ID NO 14), or [GK]A[IL][IL][KSR][LQ]L (SEQ ID NO 15).

24. The method according to any of paragraphs 22 to 24, wherein the *Lactobacillus hexosaminidase* comprises the motifs GXDE (SEQ ID NO 12) and [EQ][NRSHA][YVFL][AGSTC][IVLF][EAQYN][SN] (SEQ ID NO 13).

25. The method according to paragraph 25, wherein the *Lactobacillus hexosaminidase* comprises the motifs [VLIM][LIV]G[GAV]DE[VI][PSA] (SEQ ID NO 14) and/or [GK]A[IL][IL][KSR][LQ]L (SEQ ID NO 15).

26. The method according to paragraph 22 to 26, wherein the *Lactobacillus hexosaminidase* is selected from the group consisting of polypeptides shown in SEQ ID NO 3, SEQ ID NO 6, SEQ ID NO 9, or polypeptides having at least 60%, at least 70%, at least 75%, at least 80%, at least 85%, at least 90%, at least 95%, at least 98% or such as at least 99% sequence identity hereto.

Unless otherwise indicated, or if it is apparent from the context that something else is intended, all percentages are percentage by weight (% w/w).

## Examples

### Assays

### Wash assay

Mini Launder-O-Meter (MiniLOM) Model Wash System

MiniLOM is a mini wash system in which washes are performed in 50 ml test tubes placed in a Stuart rotator. Each tube simulates one small washing machine and during an experiment, each will contain a solution of a specific detergent/enzyme system to be tested along with the soiled and unsoiled fabrics it is tested on. Mechanical stress is achieved via rotation (typically 20rpm), and the temperature is controlled by placement of the rotator in a heating cabinet/room.

### Assay I: testing of hexosaminidase activity

The hexosaminidase activity of the polypeptides listed in the table below was determined using 4-Methylumbelliferyl N-acetyl-β-D-glucosaminide (Sigma-Aldrich) as substrate. The enzymatic reaction was performed in triplicates in a 96 well flat bottom polystyrene microtiter plate (Thermo Scientific) with the following conditions: 20 mM 3-(N-morpholino) propanesulfonic acid pH 7 buffer, 5 mM 4-Methylumbelliferyl N-acetyl-β-D-glucosaminide, 0.01 vol% Brij 35 detergent and 50 nM purified enzyme sample in a total reaction volume of 200 μl. Blank samples without polypeptide were run in parallel. The reactions were carried out at room temperature using a SpectraMax M2e Microplate Reader from Molecular Devices. Excitation wavelength was set to 368 nm and emission wavelength to 448 nm. Fluorescent signal was followed for 15 min in Kinetic Mode. Initial rate of reaction was evaluated in units of RFU/min by calculating the maximum initial increase in fluorescent signal over time as 4-Methylumbelliferyl was released from 4-Methylumbelliferyl N-acetyl-β-D-glucosaminide substrate due to enzymatic reaction.

### Composition of model detergent A (liquid)

Ingredients: 12% LAS, 11% AEO Biosoft N25-7 (NI), 5% AEOS (SLES), 6% MPG (monopropylene glycol), 3% ethanol, 3% TEA, 2.75% coco soap, 2.75% soya soap, 2% glycerol, 2% sodium hydroxide, 2% sodium citrate, 1% sodium formate, 0.2% DTMPA and 0.2% PCA (all percentages are w/w).

### Composition of Model detergent T (powder)

Ingredients: 11% LAS, 2% AS/AEOS, 2% soap, 3% AEO, 15.15% sodium carbonate, 3% sodium silicate, 18.75% zeolite, 0.15% chelant, 2% sodium citrate, 1.65% AA/MA copolymer, 2.5% CMC and 0.5% SRP (all percentages are w/w).

**Triple-20 Nonionic Model Detergent** was prepared by dissolving 3.33 g/l non-ionic detergent containing NaOH 0.87%, MPG (Monopropylenglycol) 6%, Glycerol 2%, Soap-soy 2.75%, Soap-coco 2.75%, PCA (Sokalon CP-5) 0.2%, AEO Biosoft N25-7(NI) 16%, Sodium formiate 1%, Sodium Citrate 2%, DTMPA 0.2%, Ethanol (96%) 3 %, adjustment of pH with NaOH or Citric acid ass water to 100% (all percentages are w/w (weight volume) in water with hardness 15 dH.

**Composition of Persil Universal Gel**

Ingredients: 15-30% anionic surfactants, 5-15% nonionic surfactant, <5% Phosphonate, soap, perfume, optical brightener and enzymes.

**Example 1: Strain and DNA**

The gene sequence encoding the hexosaminidase polypeptides from the strains *Lactobacillus paraplantarum* DSM 10667 and *Lactobacillus apinorum* respectively were found in the public database (Accession number SWISSPROT:A0A0R1R622 and EMBLWGS:AZEO01000001 for SEQ ID 1 and SWISSPROT:A0A0M9D3N9 and EMBLWGS:JXCT01000010 for SEQ ID 4).

The DNA encoding the hexosaminidase having the polypeptide comprised in SEQ ID 8 was isolated from a *Lactobacillus paraplantarum* bacterial strain, isolated from an environmental sample collected in Germany. Chromosomal DNA from the *Lactobacillus paraplantarum* strain was isolated by QIAamp DNA Blood Mini Kit (Qiagen, Hilden, Germany) and subjected to full genome sequencing using Illumina technology. The genome sequence was analyzed for protein sequences that have glycosyl hydrolase domains (GH20, www.cazv.org). One gene with corresponding sequence SEQ ID 7 was subsequently identified.

The codon optimized synthetic DNA encoding the mature peptide sequences of two hexosaminidases with SEQ ID 3 and 6 were ordered from the company Geneart.

Table 2:

| **SEQ ID** | **donor** | **country of origin** |
|---|---|---|
| SEQ ID 3 | *Lactobacillus paraplantarum DSM 10667* | France |
| SEQ ID 6 | *Lactobacillus apinorum* | Sweden |
| SEQ ID 9 | *Lactobacillus paraplantarum* | Germany |
| SEQ ID NO 18* | *Streptococcus merionis* | |
| *The dispersin comprising the amino acids sequence shown in SEQ ID NO 18 was expressed and purified as described in example 1 or 2 for the *Lactobacillus.* | | |

**Example 2: Cloning and expression of glycol_hydro_20 hexosaminidases**

The codon optimized synthetic genes encoding the mature peptide sequences of the hexosaminidase with SEQ ID 3 and 6 were inserted into a *Bacillus* expression vector as described in WO12/025577. Briefly, the DNA encoding the mature peptide of the glycol_hydro_20 hexosaminidase gene was cloned in frame to a *Bacillus clausii* secretion signal (BcSP; with the following amino acid sequence: MKKPLGKIVASTALLISVAFSSSIASA (SEQ ID NO: 10). BcSP replaced the native secretion signal in the gene. Downstream of the BcSP sequence, an affinity tag sequence was introduced to ease the purification process (His-tag; with the following amino acid sequence: HHHHHHPR (SEQ ID NO: 11). The gene that was expressed therefore comprised the BcSP sequence followed by the His-tag sequence followed by the mature wild type glycol_hydro_20 sequence. The DNA encoding the mature peptide of the glycol_hydro_20 beta-hexosaminidase gene SEQ ID 9 was amplified from the *Lactobacillus paraplantarum* genomic DNA by standard PCR techniques using specific primers containing an overhang to the cloning vector. The gene was consecutively cloned in frame to a *Bacillus clausii* secretion signal as described above. The final expression plasmid (BcSP-His-tag-glycol_hydro_20) was transformed into a *Bacillus subtilis* expression host. The glycol_hydro_20 BcSP-fusion gene was integrated by homologous recombination into the *Bacillus subtilis* host cell genome upon transformation. The gene construct was expressed under the control of a triple promoter system (as described in WO99/43835). The gene coding for chloramphenicol acetyltransferase was used as maker (as described in (Diderichsen et al., 1993, Plasmid 30: 312-315)). Transformants were selected on LB media agar supplemented with 6 micrograms of chloramphenicol per ml. One recombinant *Bacillus subtilis* clone containing the glycol_hydro_20 expression construct was selected and was cultivated on a rotary shaking table in 500 ml baffled Erlenmeyer flasks each containing 100 ml yeast extract-based

media. After 3-5 days' cultivation time at 30 °C to 37°C, the enzyme containing supernatant was harvested by centrifugation and the enzymes was purified by His-tag purification.

**Example 3: His tag purification method**

The His-tagged glycol_hydro_20 hexosaminidase enzymes were purified by immobilized metal chromatography (IMAC) using $Ni^{2+}$ as the metal ion on 5 mL HisTrap Excel columns (GE Healthcare Life Sciences). The purification took place at pH 7 and the bound protein was eluted with imidazole. The purity of the purified enzymes was checked by SDS-PAGE and the concentration of the enzyme determined by Absorbance 280 nm after a buffer exchange in 50mM HEPES, 100mM NaCl pH7.0

SEQ ID NO 10: MKKPLGKIVASTALLISVAFSSSIASA
SEQ ID NO 11: HHHHHHPR

**Example 4: Biofilm growth and detachment assay**

*Staphylococcus aureus* 15981 was kindly provided by Iñigo Lasa (Valle et al., Mol Microbiol.2003 May; 48 (4):1075-87). The strain was grown on trypticase soy agar (TSA) at 37°C overnight. Next day, a single colony was transferred to 15 ml tripticase soy broth (TSB) and incubated 5 hours at 37°C under shaking. The culture was diluted 1:100 in TSB+1% glucose and 100 μL of the bacterial suspension was transferred to each well of a 96-well microtiter plates (Thermo Scientific, Nunclon Delta Surface, cat # 167008) and incubated 24 hours at 37°C without shaking. and 100 μL of the bacterial suspension was transferred to each well of a 96-well microtiter plates (Thermo Scientific, Nunclon Delta Surface, cat # 167008) and incubated 24 hours at 37°C without shaking. Supernatant was aspirated and wells were washed with 100 μL of 0.9% sodium chloride and filled with 100 μL of either hard water or 3.3 gr/L non-ionic detergent or 3.3 gr/L model A detergent (composition hard water and non-ionic and model A) containing 0 (control) or 20, 10, 5, 2.5, 1.25, 0.62, 0.31, 0.16, 0.08, 0.04, 0.02 and 0.01 μg/mL of enzyme SEQ ID 3, 6 and 9. After incubation at 37°C for 1 hour, wells were washed with water and stained for 15 min with 100 μL of 0.095% crystal violet solution (SIGMA V5265). Wells were then rinsed twice with 100 μL water, dried and the plates were scanned.

The lowest concentration of each enzyme that could remove the visible formation of biofilm of S. *aureus* 15981 after 1 hour incubation, in the presence and absence of detergent was determined (see Table 3). All enzymes were assayed per duplicate in three independent assays. The average of the minimal concentration of enzyme that removed the visible formation of S. *aureus* 15981 from the three assays is listed in Table 3.

Table 3. Minimal concentration of enzyme that can remove the visible formation of S. *aureus* 15981 after 1 hour incubation in either hard water or model A detergent

Table 3

| SEQ ID | Minimal concentration for biofilm removal in hard water μg/mL | Minimal concentration for biofilm removal in non-ionic detergent μg/mL | Minimal concentration for biofilm removal in model A detergent μg/mL |
|---|---|---|---|
| 3 | 0,04 | 0,16 | 0,16 |
| 6 | 0,62 | 2,08 | 3,75 |
| 9 | 13,33 | 13,33 | >20 |

**Example 5: Cleaning properties of powder model detergents**

A crude EPS (extracellular polymeric substances) extract was prepared from *Pseudomonas fluorescens* (Isolate from Iceland) as follows: *P.fluorescens* was restreaked on TSA and incubated for 1 day at 20°C. The strain was inoculated in TSB and incubated O/N at 20°C. After propagation, the culture was diluted (1:100) in M63 supplemented medium (15mM $(NH_4)2SO_4$, 100mM $KH_2PO_4$, 1.8μM $FeSO_4$, 1mM $MgSO_4$.7H2O, 0.4% (w/v) glycerol, 0.2% (w/v) Casamino acids and 0.0001% (w/v) Thiamine), added to a Corning® CellBIND® 225$cm^2$ Angled Neck Cell Culture Flasks with Vent Cap (400ml per flask) and incubated statically for 3 days at 20°C. The biofilm culture was subsequently pelleted by centrifugation (10min, 8000g, 25°C), and the cells were resuspended in 3M NaCl (4ml per flask) and incubated for 30min at 30°C to extract the surface-associated EPS. The EPS-containing supernatant obtained after centrifugation (10min, 5000g, 25°C) was stored at -20°C until further use.

For wash performance testing, 50ul aliquots of the crude EPS extract was spotted on sterile textile swatches

(WFK20A) and incubated for 15 min at ambient temperature. The swatches (sterile or with EPS) were placed in 50 mL test tubes and 10 mL of wash liquor (15°dH water with 0.2 g/L iron(III) oxide nano-powder (544884; Sigma-Aldrich) with 5.3 g/L model T powder detergent) and enzyme was added to each tube. Washes without enzyme were included as controls. The test tubes were placed in a Stuart rotator and incubated for 1 hour at 37°C at 20rpm. The wash liquor was then removed, and the swatches were rinsed twice with 15°dH water and left to dry on filter paper overnight. The remission ($REM^{460nm}$) values were measured using a Macbeth Color-Eye 7000 (CE7000), and are displayed in table 4. Delta values ($REM^{460nm}_{(washed\ with\ enzyme)}$ - $REM^{460nm}_{(washed\ without\ enzyme)}$) are also indicated.

Table 4. Cleaning effects of hexosaminidase (SEQ ID NO 3) in powder model detergent T

| Swatch | Enzyme | Enzyme concentration (μg/ml) | Average REM(460nm) | ΔREM(460nm) |
|---|---|---|---|---|
| wfk20A | No enzyme | 0 | 61,4 | |
| wfk20, EPS | No enzyme | 0 | 52,8 | |
| wfk20, EPS | SEQ ID NO 3 | 0.2 | 60,5 | 7,6 |
| wfk20, EPS | SEQ ID NO 3 | 2 | 67,5 | 14,6 |

**Example 6. Cleaning properties of hexosaminidases in liquid model detergents**

A crude extract of biofilm extracellular polymeric substances (EPS) was prepared from *Staphylococcus aureus* 15981 (kind gift from Iñigo Lasa (Valle, J., A. Toledo-Arana, C. Berasain, J. M. Ghigo, B. Amorena, J. R. Penades, and I. Lasa. 2003, Mol. Microbiol. 48:1075-1087) as follows: 500 mL of TSB + 1% glucose (24563; Roquette Freres) was inoculated, aliquoted into 50ml conical centrifuge tubes (339652; Thermo Scientific Nunc) and incubated for 24 hours at 37°C under shaking conditions (200 rpm). Following incubation, the cells were pelleted by centrifugation (10min, 6000g, 25°C), pooled and resuspended in 4ml 3M NaCl. The suspension was vortexed vigorously and incubated for 15min at ambient temperature to extract the surface-associated EPS. The cells were then re-pelleted (10min, 5000g, 25°C) and the EPS-containing supernatant was retrieved. Milli-Q water was added (6ml) and the solution was sterile-filtered twice (0.45 μm followed by 0.2 μm). The crude extract was stored at -20°C until further use. For wash performance testing, 50ul aliquots of the crude EPS extract was spotted on sterile textile swatches (WFK20A) and incubated for 15 min at ambient temperature. The swatches (sterile or with EPS) were placed in 50 mL test tubes and 10 mL of wash liquor (15°dH water with 0.2 g/L iron(III) oxide nano-powder (544884; Sigma-Aldrich) with 3.33g/L liquid model A detergent or 3.33 g/L nonionic model detergent) and enzyme was added to each tube. Washes without enzyme were included as controls. The test tubes were placed in a Stuart rotator and incubated for 1 hour at 37°C at 20rpm. The wash liquor was then removed, and the swatches were rinsed twice with 15°dH water and left to dry on filter paper overnight. The remisssion ($REM^{460nm}$) values were measured using a Macbeth Color-Eye 7000 (CE7000), and are displayed in table 5 and 6. Delta values ($REM^{460nm}_{(washed\ with\ enzyme)}$ - $REM^{460nm}$($_{washed\ without\ enzyme)}$) are also indicated.

Table 5. Cleaning effects in liquid model detergent A

| Swatch | Enzyme | Enzyme concentration (μg/ml) | REM(460nm) | ΔREM |
|---|---|---|---|---|
| wfk20A | No enzyme | 0,0 | 58,5 | |
| EPS | No enzyme | 0,0 | 30,1 | |
| EPS | SEQ ID NO 3 | 2,0 | 65,5 | 35,4 |
| EPS | SEQ ID NO 3 | 0,2 | 58,2 | 28,1 |
| EPS | SEQ ID NO 9 | 2,0 | 38,6 | 8,5 |
| EPS | SEQ ID NO 9 | 0,2 | 32,1 | 2,0 |

Table 6. Deep-cleaning effects in liquid nonionic model detergent

| Swatch | Enzyme | Enzyme concentration (μg/ml) | REM(460nm) | ΔREM |
|---|---|---|---|---|
| wfk20A | No enzyme | 0,0 | 59,7 | |
| EPS | No enzyme | 0,0 | 30,4 | |

(continued)

| Swatch | Enzyme | Enzyme concentration (μg/ml) | REM(460nm) | ΔREM |
|---|---|---|---|---|
| EPS | SEQ ID NO 3 | 20,0 | 63,2 | 32,8 |
| EPS | SEQ ID NO 3 | 2,0 | 61,3 | 30,8 |
| EPS | SEQ ID NO 3 | 0,2 | 60,9 | 30,4 |
| EPS | SEQ ID NO 6 | 20,0 | 46,1 | 15,6 |
| EPS | SEQ ID NO 6 | 2,0 | 33,6 | 3,1 |
| EPS | SEQ ID NO 6 | 0,2 | 31,3 | 0,9 |
| EPS | SEQ ID NO 9 | 20,0 | 63,9 | 33,4 |
| EPS | SEQ ID NO 9 | 2,0 | 45,4 | 15,0 |
| EPS | SEQ ID NO 9 | 0,2 | 34,4 | 4,0 |

**Example 7: Construction of clades and phylogenetic trees**

The Glyco_hydro_20 domain includes the polypeptides of the invention having hexosaminidase e.g. PNAG activity and comprises the FQS, GADE and/or GAIL clades.

A phylogenetic tree was constructed, of polypeptide sequences containing a Glyco_hydro_20 domain, as defined in PFAM (PF00728, Pfam version 31.0 Finn (2016). Nucleic Acids Research, Database Issue 44:D279-D285). The phylogenetic tree was constructed from a multiple alignment of mature polypeptide sequences containing at least one Glyco_hydro_20 domain. The sequences were aligned using the MUSCLE algorithm version 3.8.31 (Edgar, 2004. Nucleic Acids Research 32(5): 1792-1797), and the trees were constructed using FastTree version 2.1.8 (Price et al., 2010, PloS one 5(3)) and visualized using iTOL (Letunic & Bork, 2007. Bioinformatics 23(1): 127-128). The polypeptide sequences containing a Glyco_hydro_20 domain comprises several motifs; one example is GXDE (SEQ ID NO 12), situated in positions 153 to 156 in *Lactobacillus paraplantarum* (SEQ ID NO 3). Residues D and E are the key catalytic residues of Glyco_hydro_20 enzymes (position 155 to 156 in SEQ ID NO 3).

The polypeptides in Glyco_hydro_20 can be separated into multiple distinct sub-clusters, or clades as listed below. The distinct motifs for each clade are described in details below.

*Generation of FQS clade*

A clade, preferably shared by the polypeptides of the invention, was identified. This clade has not been described previously. The clade is termed FQS and polypeptides of this clade comprises Glyco_hydro_20 domain polypeptides of bacterial origin and are in addition to having PNAG activity, characterized by comprising a certain motif. The polypeptides of the clade comprise the motif example [EQ][NRSHA][YVFL][AGSTC][IVLF][EAQYN][SN] (SEQ ID NO 13), corresponding to EHLCFQS at position 48 to 54 of SEQ ID NO 3.

*Generation of GADE clade*

A clade, preferably shared by the polypeptides of the invention, was identified. This clade has not been described previously. The clade is termed GADE and polypeptides of this clade comprise Glyco_hydro_20 domain polypeptides of bacterial origin and are in addition to having PNAG activity, characterized by comprising certain motifs. The polypeptides of the clade comprise the motif example [VLIM][LIV]G[GAV]DE[VI][PSA] (SEQ ID NO 14), corresponding to position 151 to 158 of SEQ ID NO 3, where G and DE (corresponding to positions 153 and 155-156 of SEQ ID NO 3) are fully conserved in GADE clade and part of the active site. Residues D and E are the key catalytic residues of Glyco_hydro_20 enzymes (position 155 to 156 in SEQ ID NO 3).

*Generation of GAIL clade*

The GAIL clade comprises GADE domain polypeptides of bacterial origin, having hexosaminidase e.g. PNAG activity. This clade has not been described previously, and defines a cluster within the polypeptides of the invention. The clade is termed GAIL and polypeptides of this clade comprise Glyco_hydro_20 domain polypeptides of bacterial origin, and are in addition to having PNAG activity, characterized by comprising certain polypeptide motifs. The polypeptides of the domain comprise the motif example [GK]A[IL][IL][KSR][LQ]L (SEQ ID NO 15), corresponding to position 96 to 102 of SEQ ID NO 3, where A and L (corresponding to positions 97 and 102 of SEQ ID NO 3) are fully conserved in GAIL clade. Examples of polypeptides of the invention included in the clade are SEQ ID NO 3 and SEQ ID NO 9.

An alignment of the polypeptides of the invention is shown in Figure 2. A phylogenetic tree of the polypeptides of the invention is shown in Figure 1.

**Example 8: Cleaning in full-scale washing machine**

For wash performance testing in full scale washing machines, the *P.fluorescens* and *S.aureus* EPS extracts previously mentioned were spotted on 5x5 cm sterile textile swatches (WFK20A, 250μl aliquots) and left to soak for 15 min at ambient temperature. The swatches were then attached to ballast tea towels and washed in Miele Laundry Washing Machines (Miele Softtronic, W2245) with and without enzyme, in liquid or powder detergent. The washes were run in tap water with 0.08 g/L iron(III) oxide nano-powder (544884; Sigma-Aldrich) in 4,6g/L liquid detergent (Persil Universal gel) or with 0.7g/L WFK 09V pigment soil (Wfk-Testgewebe GmbH, #00500) in 5.3 g/L powder model detergent T, respectively. The 30°C Color program was used for these tests (run time 1h, 26min). After washing, the items were line-dried at room temperature overnight. The remission ($REM^{460nm}$) values were measured using a Macbeth Color-Eye 7000 (CE7000), and are displayed in table 7 and 8. Wash performances ($\Delta$REM(460nm) = $REM^{460nm}$($_{\text{washed with enzyme}}$) - $REM^{460nm}$($_{\text{washed without enzyme}}$)) are also indicated.

Table 7. Deep-cleaning of SEQ ID NO 3 in full-scale washing machine in liquid detergent

| EPS spotted on T-shirt | Enzyme | Average Rem460nm values | Wash performance (ΔRem460nm) |
|---|---|---|---|
| *P.fluorescens* EPS, wfk20A | No enzyme | 47.4 | |
| *P.fluorescens* EPS, wfk20A | 0.2 μg/ml SEQ ID NO 3 | 72.2 | 24.8 |
| *S.aureus* EPS, wfk20A | No enzyme | 41.2 | |
| *S.aureus* EPS, wfk20A | 0.2 ug/ml SEQ ID NO 3 | 66.5 | 25.3 |

Table 8. Deep-cleaning of SEQ ID NO 3 in full-scale washing machine in powder model detergent T

| EPS spotted on T-shirt | Enzyme | Average Rem460nm values | Wash performance (ΔRem460nm) |
|---|---|---|---|
| *P.fluorescens* EPS, wfk20A | No enzyme | 58.6 | |
| *P.fluorescens* EPS, wfk20A | 0.2 ug/ml SEQ ID NO 3 | 64.0 | 5.4 |
| *S.aureus* EPS, wfk20A | No enzyme | 60.2 | |
| *S.aureus* EPS, wfk20A | 0.2 ug/ml SEQ ID NO 3 | 63.3 | 3.1 |

**Example 9 Deep-cleaning properties of Hexosaminidase in liquid model detergent on EPS from different microorganisms**

Crude extracts of biofilm extracellular polymeric substances (EPS) were prepared from *S.aureus* and *P.fluorescens* as described above. For wash performance testing, 50ul aliquots of the crude EPS extracts were spotted on sterile textile swatches (WFK20A) and incubated for 15 min at ambient temperature. The swatches (sterile or with EPS) were placed in 50 mL test tubes and 10 mL of wash liquor (15°dH water with 0.2 g/L iron(III) oxide nano-powder (544884; Sigma-Aldrich) with 3.33g/L liquid model A detergent) and enzyme was added to each tube. Washes without enzyme were included as controls. The test tubes were placed in a Stuart rotator and incubated for 1 hour at 37°C at 20rpm. The wash liquor was then removed, and the swatches were rinsed twice with 15°dH water and left to dry on filter paper

overnight. The remission ($REM^{460nm}$) values were measured using a Macbeth Color-Eye 7000 (CE7000), and are displayed in table 9. Wash performances ($\Delta REM(460nm) = REM^{460nm}(_{washed\ with\ enzyme}) - REM^{460nm}(_{washed\ without\ enzyme})$) are also indicated.

Table 9. Deep-cleaning effects of Hexosaminidase with SEQ ID NO 18 in liquid model detergent on EPS from different microorganisms

| | Enzyme | Enzyme concentration (μg/ml) | Average REM (460nm) values | Wash performance (ΔREM(460nm) |
|---|---|---|---|---|
| Clean textile (wfk20A), no EPS | | 0 | 59.3 | |
| *P. fluorescens* EPS (wfk20A) | | 0 | 35.0 | |
| *P. fluorescens* EPS (wfk20A) | SEQ ID NO 18 | 0.002 | 39.3 | 4.3 |
| *P. fluorescens* EPS (wfk20A) | SEQ ID NO 18 | 0.02 | 51.4 | 16.4 |
| *P. fluorescens* EPS (wfk20A) | SEQ ID NO 18 | 0.2 | 56.4 | 21.4 |
| *S.aureus* EPS (wfk20A) | | 0 | 34.7 | |
| *S.aureus* EPS (wfk20A) | SEQ ID NO 18 | 0.002 | 45.5 | 10.5 |
| *S.aureus* EPS (wfk20A) | SEQ ID NO 18 | 0.02 | 54.0 | 19.0 |
| *S.aureus* EPS (wfk20A) | SEQ ID NO 18 | 0.2 | 56.7 | 21.7 |

**Example 10 Biofilm growth and detachment assay**

*Staphylococcus aureus* 15981 was kindly provided by Iñigo Lasa (Valle et al., Mol Microbiol.2003 May; 48 (4):1075-87). The strain was grown on trypticase soy agar (TSA) at 37°C overnight. Next day, a single colony was transferred to 15 ml tripticase soy broth (TSB) and incubated 5 hours at 37°C under shaking. The culture was diluted 1:100 in TSB+1% glucose and 100 μL of the bacterial suspension was transferred to each well of a 96-well microtiter plates (Thermo Scientific, Nunclon Delta Surface, cat # 167008) and incubated 24 hours at 37°C without shaking. and 100 μL of the bacterial suspension was transferred to each well of a 96-well microtiter plates (Thermo Scientific, Nunclon Delta Surface, cat # 167008) and incubated 24 hours at 37°C without shaking. Supernatant was aspirated and wells were washed with 100 μL of 0.9% sodium chloride and filled with 100 μL of either hard water or 3.3 gr/L non-ionic detergent or 3.3 gr/L model A detergent (composition hard water and non-ionic and model A) containing 0 (control) or 20, 10, 5, 2.5, 1.25, 0.62, 0.31, 0.16, 0.08, 0.04, 0.02 and 0.01 μg/mL of dispersin SEQ ID 18. After incubation at 37°C for 1 hour, wells were washed with water and stained for 15 min with 100 μL of 0.095% crystal violet solution (SIGMA V5265). Wells were then rinsed twice with 100 μL water, dried and the plates were scanned.

The lowest concentration of each enzyme that could remove the visible formation of biofilm of the S. *aureus* 15981 organism after 1 hour incubation, in the presence and absence of detergent was determined (see Table 10). All enzymes were assayed per duplicate. The average of the minimal concentration of enzyme that removed the visible formation of S. *aureus* 15981 from the three assays is listed in Table 10.

Table 10. Minimal concentration of enzyme that can remove the visible formation of S. *aureus* 15981 after 1 hour incubation in either hard water or model A detergent

| Enzyme | Minimal concentration for biofilm removal in hard water μg/mL | Minimal concentration for biofilm removal in non-ionic detergent μg/mL | Minimal concentration for biofilm removal in model A detergent μg/mL |
|---|---|---|---|
| SEQ ID NO 18 | 0,04 | 0,04 | 0.08 |

**Example 11 characterization of dispersins**

Dispersin activity as a function of pH

*Activity assay:* The activity of the dispersin having SEQ ID NO: 3 was measured with 4-Nitrophenyl N-acetyl-p-D-glucosaminide (4-NAG, CAS Number 3459-18-5, CHE00244) as substrate as a function of pH (4-10 in 1-unit increments). The concentrations of substrate and the dispersin having SEQ ID NO: 3 were 5 mM and 10.0 μM, respectively, in all measurements. The dilution buffers comprise: 50 mM MES (CAS Number: 4432-31-9), 50 mM glycine (CAS Number: 56-40-6), 50 mM acetic acid (CAS Number: 64-19-7) adjusted to pH 4-10.

The substrate solution (10 mM) was prepared by dissolving 34.23 mg 4-NAG in 10.0 mL water. Dissolution required rigorous vortex mixing and gentle heating. The enzyme concentration was determined by UV-Vis ($\varepsilon_{280}$ =57890 $M^{-1}cm^{-1}$).

The enzyme samples were incubated at the different pH-values in volumes of 200 μL in a thermomixer (in MTP) for 10 min and 500 rpm at 30 °C. After 10 min, the MTP was incubated at 95 °C and 500 rpm for 10 min in thermomixer to end the reaction. Then the samples were transferred to ice bath and cooled for 2 min. The samples were added 20 μL 4 M NaOH to deprotonate pNP (induce yellow color). Absorbance at 405 nm was measured for 2 min in 10 sec. intervals. All measurements were produced in triplicates and reference samples were produced for all conditions (buffer instead of enzyme).

*Results:* The following table display the average absorbance (activity) subtracted the average absorbance of the reference samples measured after 10 min incubation at different pH values:

| pH | 4 | 5 | 6 | 7 | 8 | 9 | 10 |
|---|---|---|---|---|---|---|---|
| $A_{405}$ | 0.498055 | 0.431582 | 0.313236 | 0.2911 | 0.297542 | 0.321879 | 0.3744 |

Dispersin stability as a function of pH and NaCl

*Stability assay - differential scanning fluorimetry:* The thermal stability of the dispersin having SEQ ID NO: 3 was measured as a function of pH (4, 6, 7, 8, 10) and NaCl concentration (100, 200, and 300 mM). The thermal unfolding was monitored using intrinsic fluorescence utilizing a Prometheus NT.48. The concentrations the dispersin having SEQ ID NO: 3 was 0.2 mg/mL in all measurements. The enzyme concentration was determined by UV-Vis ($\varepsilon_{280}$ =57890 $M^{-1}cm^{-1}$). The dilution buffers comprise: 50 mM MES (CAS Number: 4432-31-9), 50 mM glycine (CAS Number: 56-40-6), 50 mM acetic acid (CAS Number: 64-19-7) adjusted to pH 4, 6, 7, 8, or 10.

The enzyme samples were prepared by mixing a 5 M NaCl stock, buffer, water (MQ), and enzyme to obtain the desired concentrations. The total volume of each mixture was 100 μL. The samples were loaded in the instrument in duplicates and measured from 20 to 95 °C with temperature ramping of 2.0 °C/min.

*Results:* Melting temperatures ($T_m$-values) were derived from the thermograms using the PR.ThermControl v.2.0.4 software. The following table display the average $T_m$-values obtained at the different conditions:

| | | pH | | | | |
|---|---|---|---|---|---|---|
| | | 4 | 6 | 7 | 8 | 10 |
| [NaCl] mM | 0 | 52.1 | 52.9 | 51.1 | 50.0 | 29.2 |
| | 100 | 50.0 | 52.3 | 50.1 | 48.5 | N/A |
| | 200 | 49.1 | 51.9 | 49.6 | 47.8 | N/A |
| | 300 | 48.3 | 51.4 | 48.8 | 46.9 | N/A |

Dispersin activity as a function of temperature

*Activity assay:* The activity of the dispersin having SEQ ID NO: 3 was measured with 4-Nitrophenyl N-acetyl-β-D-glucosaminide (4-NAG, CAS Number 3459-18-5, CHE00244) as substrate at pH 7. The concentrations of substrate and the dispersin having SEQ ID NO: 3 were 1 mM and 5 μM, respectively, in all measurements. The dilution buffer comprises: 50 mM MES (CAS Number: 4432-31-9), 50 mM glycine (CAS Number: 56-40-6), 50 mM acetic acid (CAS Number: 64-19-7), pH 7.

The substrate solution (10 mM) was prepared by dissolving 35.9 mg 4-NAG in 10.482 mL water. Dissolution required rigorous vortex mixing and gentle heating. The enzyme concentration was determined by UV-Vis ($\varepsilon_{280}$ =57890 $M^{-1}cm^{-1}$). The reaction mixture comprised 23.7 μL enzyme (42.3 μM), 20 μL substrate, and 156.3 μL buffer.

The enzyme samples were incubated in volumes of 200 μL in a thermomixer for 10 min and 500 rpm at 20, 30, 40, 45, 50, 55, 60, or 70 °C. After 10 min, the samples were transferred to ice bath and cooled for 2 min. The samples were added 10 μL 4 M NaOH to deprotonate pNP (induce yellow color). 180 μL reaction mixture was transferred to a MT plate and absorbance at 405 nm was measured for 1 min in 10 sec. intervals. All measurements were produced in duplicates and reference samples were produced for all conditions (buffer instead of enzyme).

*Results:* The following table display the average absorbance (activity) subtracted the average absorbance of the reference samples measured after 10 min incubation at different temperatures:

| | 20 °C | 30 °C | 40 °C | 45 °C | 50 °C | 55 °C | 60 °C | 70 °C |
|---|---|---|---|---|---|---|---|---|
| **$A_{405}$** | 0.00 | 0.02 | 0.02 | 0.03 | 0.03 | 0.01 | 0.01 | 0.00 |

**Claims**

**1.** A cleaning composition comprising a *Lactobacillus* or *Streptococcus* hexosaminidase, wherein the composition

(a) is a solid, preferably granular, laundry detergent composition and further comprises

(a1) at least one zeolite builder, preferably in an amount of 10 to 50 wt.-%, more preferably 20-30 wt.- %;
(a2) at least one phosphonate builder, preferably in an amount of 0.1 to 5 wt.-%, more preferably 0.4 to 1.5 wt.-%;
(a3) at least one further enzyme, preferably a cellulase, preferably in an amount of active enzyme of 100 to 5000 ppb, more preferably 1000 to 2000 ppb; and
(a4) at least one polymer, preferably a polyvinylpyrrolidon polymer, preferably in an amount of 0.01 to 1 wt.-%, more preferably 0.1 to 0.3 wt.-%; or

(b) is a solid laundry detergent composition and further comprises

(b1) at least one silicate builder, preferably in an amount of 2 to 20 wt.-%, more preferably 5-10 wt.-%;
(b2) optionally carboxymethylcellulose, preferably in an amount of 0.1 to 10 wt.-%, more preferably 0.1 to 4 wt.-%;
(b3) at least one further enzyme, preferably a cellulase, preferably in an amount of active enzyme of 0.1 to 100 ppm, more preferably 0.1 to 10 ppm;
(b4) optionally at least one soil release polymer, preferably a polyvinylpyrrolidon polymer, in an amount of 0.1 to 3 wt.-%, more preferably 0.1 to 1.0 wt.-%; and
(b5) at least one bleaching system, comprising a bleaching agent, a bleach activator and a bleach catalyst, preferably in an amount of 0.1 to 50 wt.-%, more preferably 0.1 to 30 wt.-%; or

(c) is liquid laundry detergent composition and further comprises

(c1) at least one surfactant, preferably nonionic surfactant, preferably in an amount of 1 to 20 wt.-%, preferably 3 to 15 wt.-%;
(c2) optionally at least one phosphonate builder, preferably in an amount of 0.1 to 3 wt.-%, more preferably 0.25 to 1.5 wt.-%
(c3) optionally at least at least one further enzyme, preferably a cellulase, preferably in an amount of enzyme composition of 0.001 to 1 wt.-%, more preferably 0.001 to 0.6 wt.-%; and
(c4) optionally at least one organic solvent, preferably glycerol, preferably in an amount of 0.1 to 10 wt.-%, more preferably 0.1 to 5 wt.-%; or

(d) is a liquid laundry detergent in unit dose form, preferably a pouch comprising a water-soluble film, and further comprises

(d1) water in an amount of up to 20 wt.-%, preferably 5 to 15 wt.-%;
(d2) optionally at least one bittering agent, preferably Benzyldiethyl(2,6-xylylcarbamoyl)-methylammoniumbenzoate, preferably in an amount of 0.00001 to 0.04 wt.-%;

(d3) optionally at least one optical brightener, preferably in an amount of 0.01 to 2 wt.-%, more preferably 0.01 to 1 wt.-%; and
(d4) optionally at least one polymer, preferably in an amount of 0.01 to 7 wt.-%, more preferably 0.1 to 5 wt.-%; or

(e) is a fabric finisher and further comprises

(e1) at least one softening silicone, preferably an amino-functionalized silicone, preferably in an amount of 0.1 to 10 wt.-%, more preferably 0.1 to 2 wt.-%;
(e2) at least one perfume, preferably at least partially encapsulated in microcapsules, more preferably at least partially encapsulated in melamine-formaldehyde microcapsules, preferably in an amount of 0.01 to 3 wt.-%, more preferably 0.1 to 1 wt.-%;
(e3) optionally polyquaternium 10 in an amount of 0.1 to 20 wt.-%, preferably 0.1 to 13 wt.-%;
(e4) optionally polyquaternium 37 in an amount of 0.1 to 20 wt.-%, preferably 0.1 to 13 wt.-%;
(e5) optionally a plant-based esterquat, preferably a canola- or palm-based esterquat, in an amount of 0.1 to 20 wt.-%, preferably 0.1 to 13 wt.-%; and
(e6) optionally adipic acid, in an amount of 0.1 to 20 wt.-%, preferably 0.1 to 13 wt.-%; or

(f) is an acidic cleaning agent, preferably having a pH less than 6, and further comprises

(f1) plant-based or bio-based surfactants, preferably each in an amount of 0.1 to 5, more preferably each in an amount of 0.1 to 2 wt.-%;
(f2) at least one acidic biocide, preferably selected from acids, more preferably HCl and formic acid; and
(f3) at least one soil release, water repellant or water spreading polymer, preferably in an amount of 0.01 to 3 wt.-%, more preferably 0.01 to 0.5 wt.-%; or

(g) is a neutral cleaning agent, preferably having a pH between 6.0 and 7.5, and further comprises

(g1) plant-based or bio-based surfactants, preferably each in an amount of 0.1 to 5, more preferably each in an amount of 0.1 to 2 wt.-%;
(g2) at least one biocide, preferably selected from quaternary ammonium compounds and alcohols; and
(g3) at least one soil release, water repellant or water spreading polymer, preferably in an amount of 0.01 to 3 wt.-%, more preferably 0.01 to 0.5 wt.-%; or

(h) is an alkaline cleaning agent, preferably having a pH of more than 7.5, and further comprises

(h1) plant-based or bio-based surfactants, preferably each in an amount of 0.1 to 5, more preferably each in an amount of 0.1 to 2 wt.-%; or

(i) is a hand dishwashing agent, preferably liquid hand dishwashing agent, and further comprises

(i1) at least one anionic surfactant, preferably in an amount of 0.1 to 40 wt.-%, more preferably 5 to 30 wt.-%;
(i2) at least one amphoteric surfactant, preferably betain, preferably in an amount of 0.1 to 25 wt.-%, more preferably 1 to 15 wt.-%;
(i3) at least one nonionic surfactant, preferably in an amount of 0.1 to 25 wt.-%, more preferably 2 to 10 wt.-%;
(i4) at least one further enzyme, preferably selected from proteases, amylases and combinations thereof, preferably in an amount of enzyme composition of up to 1 wt.-%, more preferably up to 0.6 wt.-%; or

(j) is an automatic dishwashing composition and further comprises

(j1) at least one builder selected from citrate, aminocarboxylates and combinations thereof, preferably in an amount of 5 to 30 wt.-%, more preferably 10 to 20 wt.-%;
(j2) at least one phosphonate builder, preferably in an amount of 0.1 to 5 wt.-%, more preferably 0.4 to 1.5 wt.-%;
(j3) at least one nonionic surfactant, preferably in an amount of 0.1 to 10 wt.-%, more preferably 1 to 5 wt.-%;
(j4) at least one bleaching system, comprising a bleaching agent, a bleach activator and a bleach catalyst, preferably in an amount of 0.1 to 50 wt.-%, more preferably 0.1 to 30 wt.-%; and
(j5) at least one polymer selected from sulfopolymers, cationic polymers and polyacrylates, preferably in

an amount of 0.01 to 15 wt.-%, more preferably 2 to 10 wt.-%; or

(k) further comprises

(k1) at least one sulfopolymer, preferably in an amount of 1 to 15, more preferably 2 to 10 wt.-% and is preferably a dishwashing, more preferably an automatic dishwashing composition; or

(l) further comprises at least one adjunct ingredient selected from probiotics, preferably microbes, spores or combinations thereof; or
(m) is in unit dose form and comprises at least 2, preferably 2, 3, 4 or 5 separate compartments; or is a phosphate-free composition;

wherein the composition optionally further comprises;

(a)

i. one or more polyol(s), preferably selected from glycerol, (mono, di, or tri) propylene glycol, ethylene glycol, polyethylene glycol, sugar alcohols, sorbitol, mannitol, erythritol, dulcitol, inositol, xylitol and adonitol,
ii. optionally one or more enzyme, preferably selected from proteases, amylases or lipases,
iii. optionally one or more surfactant, preferably selected from anionic and nonionic surfactants,
iv. optionally one or more polymer;

or
(b) a granule comprising

iii. a core comprising a *Lactobacillus* or *Streptococcus* hexosaminidase and optionally,
iv. a coating consisting of one or more layer(s) surrounding the core.

**2.** A composition according to claim 1, wherein the hexosaminidase has N-acetylglucosaminidase activity, preferably β-1,6 N-acetylglucosaminidase activity.

**3.** The composition according to any of the preceding claims, wherein the hexosaminidase comprises one or more of the motifs GXDE (SEQ ID NO 12), [EQ][NRSHA][YVFL][AGSTC][IVLF][EAQYN][SN] (SEQ ID NO 13), [VLIM][LIV]G[GAV]DE[VI][PSA] (SEQ ID NO 14), or [GK]A[IL][IL][KSR][LQ]L (SEQ ID NO 15).

**4.** The composition according to any of the preceding claims, wherein the *hexosaminidase* comprises the motifs GXDE (SEQ ID NO 12) and [EQ][NRSHA][YVFL][AGSTC][IVLF][EAQYN][SN] (SEQ ID NO 13).

**5.** The composition according to claim 3, wherein the hexosaminidase comprises the motifs [VLIM][LIV]G[GAV]DE[VI][PSA] (SEQ ID NO 14) and/or [GK]A[IL][IL][KSR][LQ]L (SEQ ID NO 15).

**6.** The composition according to any of the preceding claims, wherein the hexosaminidase is selected from the group consisting of polypeptides having the amino acid sequence of: SEQ ID NO 3, SEQ ID NO 6, SEQ ID NO 9, SEQ ID NO:18 and polypeptides having at least 60%, at least 70%, at least 75%, at least 80%, at least 85%, at least 90%, at least 95%, at least 98% or such as at least 99% sequence identity hereto.

**7.** The composition according to any of the preceding claims, wherein the polypeptide having hexosaminidase activity comprises the amino acid sequence of SEQ ID NO 3 or polypeptides having at least 60 % e.g. 80%, 85%, 90%, 95%, 98% or 99% sequence identity hereto.

**8.** The composition according to any of the preceding claims, wherein the polypeptide having hexosaminidase activity comprises the amino acid sequence of SEQ ID NO 6 or polypeptides having at least 60 % e.g. 80%, 85%, 90%, 95%, 98% or 99% sequence identity hereto.

**9.** The composition according to any of the preceding claims, wherein the polypeptide having hexosaminidase activity comprises the amino acid sequence of SEQ ID NO 9 or polypeptides having at least 60 % e.g. 80%, 85%, 90%, 95%, 98% or 99% sequence identity hereto.

**10.** The composition according to any of the preceding claims, wherein the polypeptide having hexosaminidase activity comprises the amino acid sequence of SEQ ID NO 18 or polypeptides having at least 60 % e.g. 80%, 85%, 90%, 95%, 98% or 99% sequence identity hereto.

**11.** The composition according to any of the preceding claims, wherein the composition further comprises one or more enzymes selected from the group consisting of proteases, lipases, cutinases, amylases, carbohydrases, cellulases, pectinases, mannanases, arabinases, galactanases, xylanases and oxidases.

**12.** Use of a composition according to claim 1 to 11, preferably a detergent composition comprising a hexosaminidase having N-acetylglucosaminidase activity, preferably β-1,6 N-acetylglucosaminidase activity,

a) for preventing, reducing or removing stickiness of the item;
b) for pretreating stains on the item;
c) for preventing, reducing or removing redeposition of soil during a wash cycle;
d) for preventing, reducing or removing adherence of soil to the item;
e) for maintaining or improving whiteness of the item;
f) for preventing, reducing or removing malodor from the item,
wherein the item is a textile.

**13.** Use according to claim 12, wherein the hexosaminidase comprises one or more of the following motifs GXDE (SEQ ID NO 12), [EQ][NRSHA][YVFL][AGSTC][IVLF][EAQYN][SN] (SEQ ID NO 13), [VLIM][LIV]G[GAV]DE[VI][PSA] (SEQ ID NO 14), or [GK]A[IL][IL][KSR][LQ]L (SEQ ID NO 15).

**14.** Use of a composition according to any of claims 12 or 13, wherein the hexosaminidase is selected from the group consisting of polypeptides shown in SEQ ID NO 3, SEQ ID NO 6, SEQ ID NO 9, or polypeptides having at least 60%, at least 70%, at least 75%, at least 80%, at least 85%, at least 90%, at least 95%, at least 98% or such as at least 99% sequence identity hereto.

Figure 1

# Phylogenetic tree

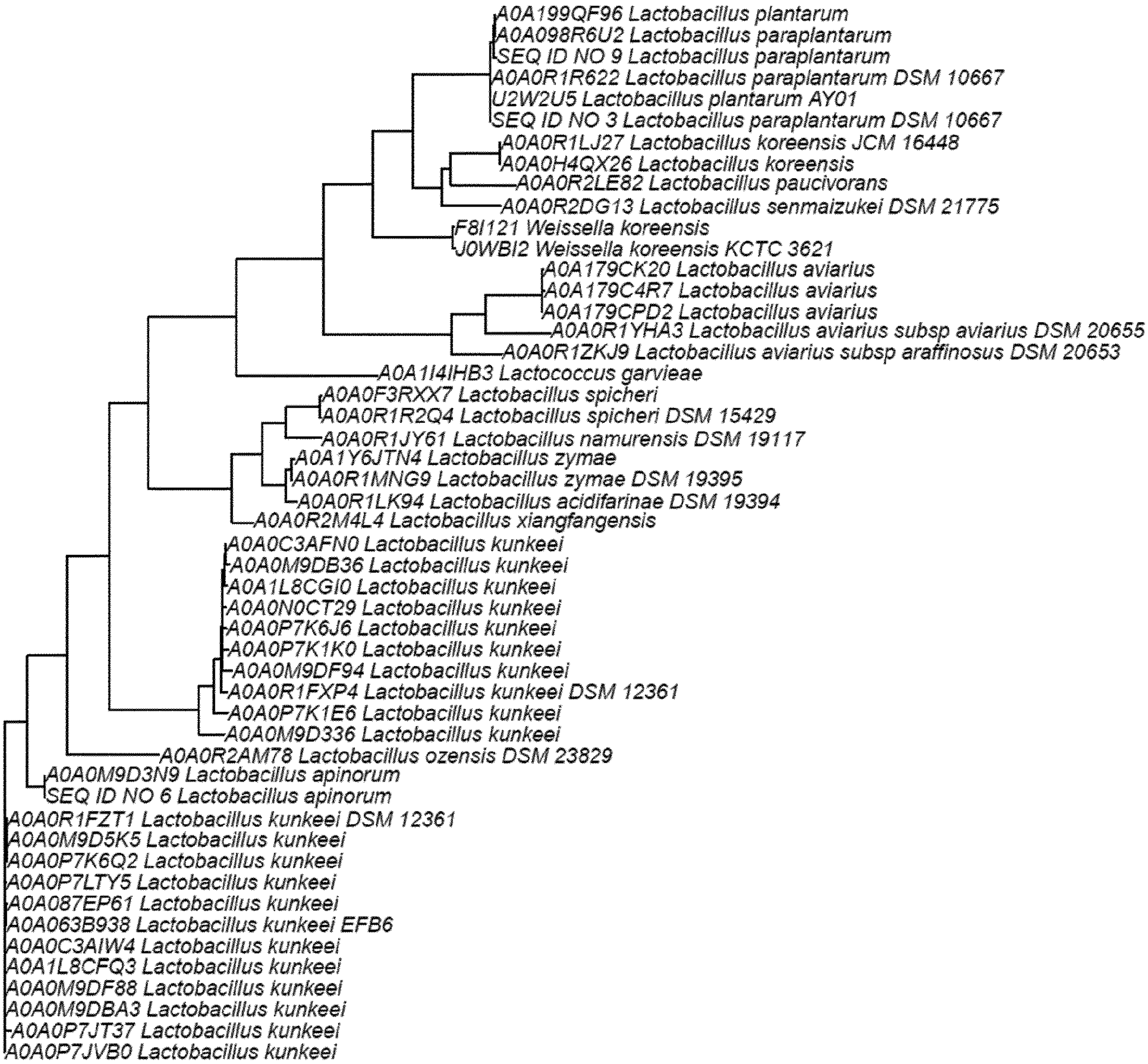

# Figure 2

```
                                                                 10        20        30        40
SEQ ID NO 3 Lactobacillus paraplantarum DSM 10667    ...NSSTLNTSQGVMLDLGRHPLDETAIKAVISAAAEQHMQYVELHLSDN
SEQ ID NO 6 Lactobacillus apinorum                   TLADTSNDTKRIGLSLDCSRTYYSPSTIKKYIDLLKKDHGTYLQLHLNDN
SEQ ID NO 9 Lactobacillus paraplantarum              ...NSSTLNTSQGVMLDLGRHPLDETAIKAVISAAAEQHMQYVELHLSDN

                                                       50        60        70        80
SEQ ID NO 3 Lactobacillus paraplantarum DSM 10667    EHLCFQSAYLG...............NAASATVLSATTLEQLVAYANQLN
SEQ ID NO 6 Lactobacillus apinorum                   ERYGVESSTLGQTTQNATLKDGVYYNNKTHLAFLSKNQLLDVIQYGYTHG
SEQ ID NO 9 Lactobacillus paraplantarum              EHLCFQSAYLG...............NAASATVLSATTLEQLVAYANQLN

                                                            90       100       110       120
SEQ ID NO 3 Lactobacillus paraplantarum DSM 10667    IELVPDVDLPSHAGAILRQLQQTHPDIYNTVKLDDET....IDYTKPAAI
SEQ ID NO 6 Lactobacillus apinorum                   IEVIPEIDLPGHAQSIFKLLSYTSEGKKLVKELENKDGYNEMYYNKQATI
SEQ ID NO 9 Lactobacillus paraplantarum              IELVPDVDLPSHAGAILRQLQQTHPDIYNTVKLDDET....IDYTKPAAV

                                                     130       140       150       160       170
SEQ ID NO 3 Lactobacillus paraplantarum DSM 10667    SLATTLYGELDASFNNQSQHDLMLGADEVPGSASAYIE.LTTFINQVSRF
SEQ ID NO 6 Lactobacillus apinorum                   DFSKKLLSEYVGML..PSGYHIIVGADEITISDKSDQEAVVKYINAIDDY
SEQ ID NO 9 Lactobacillus paraplantarum              SLATTLYGELDASFNNQSQHDLMLGADEVSGSASAYIE.LTTFINQVSRF

                                                       180       190       200       210       220
SEQ ID NO 3 Lactobacillus paraplantarum DSM 10667    QNQHGFNTSIWNDSLLKNELTRLDSNITINYWSQSGNNTDVAIIADRYAN
SEQ ID NO 6 Lactobacillus apinorum                   VNANHLKLEMWNDSFHKAVLSKYHKDILINYWSLTGEVSSSKDRKDNIRM
SEQ ID NO 9 Lactobacillus paraplantarum              QNQNGFNTSIWNDSLLKNELNRLDSNITINYWSQSGNNTDAAIIADRYAN

                                                       230       240       250       260       270
SEQ ID NO 3 Lactobacillus paraplantarum DSM 10667    RVSVPDILASGHPIVNCNSYATYYQIKNIGNVNDDDYFINYLNHTFRPNI
SEQ ID NO 6 Lactobacillus apinorum                   RATLPELNKAGFKTINYNSYYLYMITDPTSFTNESKKIWTSEFKKWKMNM
SEQ ID NO 9 Lactobacillus paraplantarum              RASVPDILASGHPIVNCNSYATYYQFKNIGNVNDDNYFINYLNHTFRPNI

                                                       280       290       300       310       320
SEQ ID NO 3 Lactobacillus paraplantarum DSM 10667    FNEIDTNGHNQDWTIEDGVTTNGILVSLWGADSEHVTPTAIVN....FIK
SEQ ID NO 6 Lactobacillus apinorum                   WNDESTKD......ITKSANNIGAAISIWGEYPNQYTGDQTYNKTYYYVD
SEQ ID NO 9 Lactobacillus paraplantarum              FNEIDTNGHNQDWTIEDGVTTNGILVSLWGADSEHVTPTAIVN....FIK

                                                            330
SEQ ID NO 3 Lactobacillus paraplantarum DSM 10667    RMTIPRSF...
SEQ ID NO 6 Lactobacillus apinorum                   TFLKAQDKFTK
SEQ ID NO 9 Lactobacillus paraplantarum              RMAIPRSF...
```

Europäisches Patentamt
European Patent Office
Office européen des brevets

# EUROPEAN SEARCH REPORT

Application Number
EP 18 20 3731

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/186936 A1 (NOVOZYMES AS [DK]) 2 November 2017 (2017-11-02)<br>* abstract *<br>* page 2, line 24 - page 3, line 10 *<br>----- | 1-7, 11-14 | INV.<br>C11D3/386<br>C12N9/24 |
| X | WO 99/57252 A1 (PROCTER & GAMBLE [US]; SMETS JOHAN [BE] ET AL.) 11 November 1999 (1999-11-11)<br>* abstract *<br>* page 20, last paragraph - page 21, paragraph 1 *<br>* page 22, paragraph 2 *<br>* page 91; claim 2 *<br>----- | 1-7, 11-14 | |
| X | WO 2017/214240 A2 (PROCTER & GAMBLE [US]) 14 December 2017 (2017-12-14)<br>* abstract *<br>* page 32; examples 1-7 *<br>----- | 1-7, 11-14 | |

TECHNICAL FIELDS SEARCHED (IPC)

C11D
C12N

~~The present search report has been drawn up for all claims~~

1

| Place of search | Date of completion of the search | Examiner |
|---|---|---|
| Munich | 2 May 2019 | Grötzinger, Thilo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number
EP 18 20 3731

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

7(completely); 1-6, 11-14(partially)

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 18 20 3731

The Search Division considers that the present European patent application does not comply with the requirements of unity of invention and relates to several inventions or groups of inventions, namely:

1. claims: 7(completely); 1-6, 11-14(partially)

   Use of the hexosaminidase shown in SEQ ID NO:3 in a cleaning composition, and related embodiments.

   ---

2. claims: 8(completely); 1-6, 11-14(partially)

   Use of the hexosaminidase shown in SEQ ID NO:6 in a cleaning composition, and related embodiments.

   ---

3. claims: 9(completely); 1-6, 11-14(partially)

   Use of the hexosaminidase shown in SEQ ID NO:9 in a cleaning composition, and related embodiments.

   ---

4. claims: 10(completely); 1-6, 11-14(partially)

   Use of the hexosaminidase shown in SEQ ID NO:18 in a cleaning composition, and related embodiments.

   ---

# ANNEX TO THE EUROPEAN SEARCH REPORT ON EUROPEAN PATENT APPLICATION NO. EP 18 20 3731

This annex lists the patent family members relating to the patent documents cited in the above-mentioned European search report. The members are as contained in the European Patent Office EDP file on
The European Patent Office is in no way liable for these particulars which are merely given for the purpose of information.

02-05-2019

| Patent document cited in search report | Publication date | Patent family member(s) | Publication date |
|---|---|---|---|
| WO 2017186936 A1 | 02-11-2017 | CN 109415665 A | 01-03-2019 |
| | | EP 3448977 A1 | 06-03-2019 |
| | | WO 2017186936 A1 | 02-11-2017 |
| WO 9957252 A1 | 11-11-1999 | AT 371723 T | 15-09-2007 |
| | | AU 7275498 A | 23-11-1999 |
| | | BR 9910151 A | 09-01-2001 |
| | | CA 2330614 A1 | 11-11-1999 |
| | | CN 1307633 A | 08-08-2001 |
| | | EP 1073724 A1 | 07-02-2001 |
| | | JP 2003522517 A | 29-07-2003 |
| | | WO 9957250 A1 | 11-11-1999 |
| | | WO 9957252 A1 | 11-11-1999 |
| WO 2017214240 A2 | 14-12-2017 | CA 3022860 A1 | 14-12-2017 |
| | | EP 3469056 A2 | 17-04-2019 |
| | | US 2017355931 A1 | 14-12-2017 |
| | | US 2018355287 A1 | 13-12-2018 |
| | | WO 2017214240 A2 | 14-12-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 04061117 A2
- WO 9850512 A
- US 4106991 A
- US 4661452 A
- GB 1483591 A
- WO 2013188331 A
- WO 9307263 A
- WO 9723606 A
- WO 0001793 A
- WO 2006034710 A
- WO 9932595 A
- EP 0164514 A
- WO 09102854 A
- US 5977053 A
- WO 9817767 A
- EP 624154 A
- WO 2007087258 A
- WO 2007087244 A
- WO 2007087259 A
- EP 1867708 A
- WO 2007087242 A
- WO 9426860 A
- WO 9426859 A
- WO 2006130575 A
- WO 200503274 A
- WO 200503275 A
- WO 200503276 A
- EP 1876226 A
- WO 2007087257 A
- WO 2007087243 A
- US 7262042 B
- WO 09021867 A
- WO 9318140 A
- WO 01016285 A
- WO 02016547 A
- WO 9425583 A
- WO 05040372 A
- WO 05052161 A
- WO 05052146 A
- WO 9523221 A
- WO 9221760 A
- EP 1921147 A
- EP 1921148 A
- WO 07044993 A
- WO 8906279 A
- WO 9219729 A
- WO 96034946 A
- WO 9820115 A
- WO 9820116 A
- WO 99011768 A
- WO 0144452 A
- WO 03006602 A
- WO 0403186 A
- WO 04041979 A
- WO 07006305 A
- WO 11036263 A
- WO 11036264 A
- WO 2016001449 A
- WO 2004067737 A
- US 5352604 A
- US 4963655 A
- US 5159060 A
- WO 9512655 A
- WO 9529223 A
- WO 9219707 A
- WO 9404653 A
- WO 9404654 A
- US 5442100 A
- US 5488157 A
- US 5472628 A
- WO 9404651 A
- WO 9525791 A
- WO 9813458 A
- WO 9813459 A
- WO 9813460 A
- WO 9813461 A
- WO 9813462 A
- WO 07141736 A
- WO 07145963 A
- WO 09118375 A
- WO 10055052 A
- WO 11036153 A
- WO 2013004636 A
- WO 9847523 A
- US 6500802 B
- US 5436229 A
- WO 2013004635 A
- WO 2008134343 A
- US 4435307 A
- US 5648263 A
- US 5691178 A
- US 5776757 A
- WO 8909259 A
- EP 0495257 A
- EP 0531372 A
- WO 9611262 A
- WO 9629397 A
- WO 9808940 A

- WO 9407998 A
- EP 0531315 A
- US 5457046 A
- US 5686593 A
- US 5763254 A
- WO 9524471 A
- WO 9812307 A
- WO 99001544 A
- WO 2002099091 A
- WO 2001062903 A
- WO 1999064619 A
- WO 9324618 A
- WO 9510602 A
- WO 9815257 A
- EP 258068 A
- EP 305216 A
- WO 9613580 A
- EP 218272 A
- EP 331376 A
- WO 9506720 A
- WO 9627002 A
- WO 9612012 A
- WO 10065455 A
- WO 10107560 A
- US 5389536 A
- WO 11084412 A
- WO 11084417 A
- WO 11084599 A
- WO 11150157 A
- WO 12137147 A
- EP 407225 A
- WO 9205249 A
- WO 9401541 A
- WO 9425578 A
- WO 9514783 A
- WO 9530744 A
- WO 9535381 A
- WO 9522615 A
- WO 9600292 A
- WO 9704079 A
- WO 9707202 A
- WO 0034450 A
- WO 0060063 A
- WO 0192502 A
- WO 0787508 A
- WO 09109500 A
- WO 10111143 A
- WO 0556782 A
- WO 0967279 A
- WO 10100028 A
- GB 1296839 A
- WO 9510603 A
- WO 9402597 A
- WO 9418314 A
- WO 9743424 A
- WO 99019467 A
- WO 02010355 A
- WO 2006066594 A
- WO 96023873 A
- WO 08153815 A
- WO 0166712 A
- WO 09061380 A
- WO 13184577 A
- WO 10104675 A
- WO 2011098531 A
- WO 2013001078 A
- WO 2013001087 A
- EP 179486 A
- WO 9201046 A
- JP 2238885 A
- WO 9708325 A
- WO 9533836 A
- WO 2009087523 A
- WO 2007138054 A
- WO 2006108856 A
- WO 2006113314 A
- EP 1867808 A
- WO 2003040279 A
- EP 2169040 A
- US 20090011970 A1
- WO 12025577 A
- WO 9943835 A

**Non-patent literature cited in the description**


- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **CUNNINGHAM ; WELLS.** *Science,* 1989, vol. 244, 1081-1085
- **HILTON et al.** *J. Biol. Chem.,* 1996, vol. 271, 4699-4708
- **DE VOS et al.** *Science,* 1992, vol. 255, 306-312
- **SMITH et al.** *J. Mol. Biol.,* 1992, vol. 224, 899-904
- **WLODAVER et al.** *FEBS Lett.,* 1992, vol. 309, 59-64
- **C. E. CAPES.** Handbook of Powder Technology. Elsevier, 1980, vol. 1
- *J. Am. Chem. Soc.,* 1978, vol. 100, 1228
- *Org. Synth., Coll.,* vol. 7, 361
- **DIDERICHSEN et al.** *Plasmid,* 1993, vol. 30, 312-315
- **VALLE et al.** *Mol Microbiol.,* May 2003, vol. 48 (4), 1075-87

- **VALLE, J. ; A. TOLEDO-ARANA ; C. BERASAIN ; J. M. GHIGO ; B. AMORENA ; J. R. PENADES ; I. LASA.** *Mol. Microbiol.,* 2003, vol. 48, 1075-1087
- *Nucleic Acids Research,* vol. 44, D279-D285
- **EDGAR.** *Nucleic Acids Research,* 2004, vol. 32 (5), 1792-1797
- **PRICE et al.** *PloS,* 2010, vol. 5 (3
- **LETUNIC ; BORK.** *Bioinformatics,* 2007, vol. 23 (1), 127-128